(19) Europäisches Patentamt
European Patent Office
Office européen des brevets

(11) **EP 4 545 570 A1**

(12) **EUROPEAN PATENT APPLICATION**
published in accordance with Art. 153(4) EPC

(43) Date of publication:
**30.04.2025 Bulletin 2025/18**

(21) Application number: **23826573.0**

(22) Date of filing: **21.06.2023**

(51) International Patent Classification (IPC):
***C07K 19/00*** *(2006.01)* ***C07K 14/605*** *(2006.01)*
***A61K 45/06*** *(2006.01)* ***C07K 14/00*** *(2006.01)*
***A61K 38/16*** *(2006.01)*

(52) Cooperative Patent Classification (CPC):
**A61K 38/16; A61K 45/06; C07K 14/00; C07K 14/605; C07K 19/00**

(86) International application number:
**PCT/CN2023/101978**

(87) International publication number:
**WO 2023/246928 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.06.2022 CN 202210720621**
**23.06.2022 CN 202210718428**

(71) Applicants:
- **Guangzhou Innogen Pharmaceutical Group Co., Ltd.**
  **Guangzhou, Guangdong 510700 (CN)**
- **Shanghai Innogen Pharmaceutical Technology Co., Ltd.**
  **Pudong New Area, Shanghai 201203 (CN)**
- **Shanghai Innogen Biomedical Engineering Co., Ltd.**
  **Pudong New Area, Shanghai 201413 (CN)**

(72) Inventor: **WANG, Qinghua**
**Shanghai 201203 (CN)**

(74) Representative: **FRKelly**
**Waterways House**
**Grand Canal Quay**
**Dublin D02 PD39 (IE)**

Remarks:
The complete document including Reference Table(s) and the Sequence Listing(s) can be downloaded from the EPO website

# (54) FUSION PROTEIN CONTAINING IMPROVED GLP-1 RECEPTOR AGONIST AND USES

(57) This invention relates to an improved GLP-1 receptor agonist, a fusion protein comprising the improved GLP-1 receptor agonist, a nucleic acid encoding the fusion protein, a vector and cell comprising the nucleic acid, and uses thereof. Specifically, the present invention provides a modified and improved GLP-1-IgG2/Fc fusion protein, a nucleic acid encoding the fusion protein, a vector and cell comprising the nucleic acid, and a composition thereof. The invention further relates to use of the fusion protein, nucleic acid, vector, cell and composition thereof in the manufacture of a medicament for the treatment or prevention of metabolic diseases associated with glucose or lipid metabolism disorders, as well as neurological disorders and other diseases.

FIG. 8

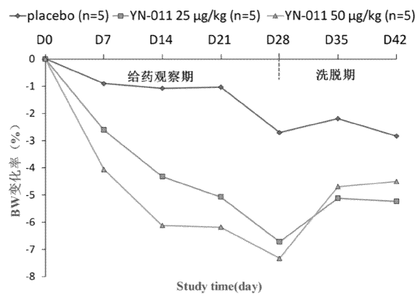


EP 4 545 570 A1

## Description

### FIELD:

The present invention relates to the field of biomedicine. Specifically, the present invention relates to an improved GLP-1 receptor agonist, a fusion protein comprising said improved GLP-1 receptor agonist, a nucleic acid encoding the fusion protein, a vector containing the nucleic acid, cells, and their applications.

### Background

Glucagon-like peptide-1 (GLP-1), also known as incretin, is secreted by L cells in the small intestine. It exerts multi-organ targeted regulatory effects, including promoting insulin secretion, inhibiting glucagon release, slowing gastric emptying, reducing appetite, and playing an important role in the regulation of nutrient intake and absorption. The biological effects of GLP-1 are primarily mediated through the activation of the GLP-1 receptor (GLP-1R). GLP-1R is a G protein-coupled membrane protein primarily expressed in pancreatic beta cells, with varying degrees of expression in other tissues and cells such as the lungs, heart, kidneys, gastrointestinal tract, and brain. Upon binding to the receptor, GLP-1 activates adenylate cyclase (AC), thereby stimulating the generation of the second messenger cyclic adenosine monophosphate (cAMP) and interacting with protein kinase A (PKA) and cAMP-regulated guanine nucleotide exchange factors (camp GEFs) of the Epac family [1].

The natural half-life of GLP-1 in the human body is only 1-2 minutes, primarily due to rapid enzymatic inactivation, including by dipeptidyl peptidase-IV (DPP-IV) [2], and/or renal clearance [3]. Therefore, scientists have developed various long-acting GLP-1 analogs resistant to degradation. For example, human GLP-1 analogs have been modified through amino acid substitutions [4, 5] and/or N-terminal modifications, including fatty acylation [6] and N-acetylation [7], to extend their circulating half-life. Albumin-conjugated GLP-1 (albiglutide) also exhibits an extended half-life [8]. In recent years, various GLP-1 receptor agonists and analogs have been widely used for the treatment of metabolic disorders related to glucose and lipid metabolism, particularly type 2 diabetes mellitus (T2DM) and obesity. GLP-1 receptor agonists play a significant role in diabetes treatment and also have preventive and therapeutic effects on cardiovascular diseases [9] and neurological disorders [10, 11]. Additionally, GLP-1 can bind to GLP-1 receptors in organs such as the kidneys and skin, influencing tissue metabolism and related diseases [12].

The GLP-1 fusion protein disclosed in US Patent US8658174 comprises a GLP-1 peptide fused with an IgG/Fc domain and can be used for the treatment of diabetes.

Studies have shown that GLP1-Fc fusion protein undergoes post-translational modifications. Hou et al. reported in 2019 that the addition of nicotinamide and cysteine during cell culture process helps reduce the hydroxylation level of a GLP analog when fused with IgG4/Fc protein (dulaglutide) [13].

The use of genetic engineering and recombinant protein technology to produce therapeutic fusion proteins aims to express the properties of natural peptides. The process involves cellular engineering steps including transcription, translation, and post-translational modifications. The manufacturing process directly impacts the physicochemical characteristics, conformation, in vivo half-life, biological activity, and production yield of the drug. Therefore, there is still a need for improved GLP-1 receptor agonists and their fusion proteins that are suitable for large-scale production, possess higher yield and activity, and exhibit longer half-life, among other desirable features, for clinical treatment.

### The content of the invention

The purpose of the present invention is to provide an improved GLP-1 receptor agonist and its fusion protein with higher activity and yield. The fusion protein can be obtained through various approaches or methods, such as amino acid substitutions at specific positions in the protein sequence, or modifications like hydroxylation and oxidation. For example, hydroxylation at position K34 of the GLP-1 peptide and/or reduction of oxidation of the GLP-1 peptide can be performed. Furthermore, specific amino acid substitutions or modifications at certain positions of the GLP-1 fusion protein in the present invention significantly extend the half-life of the improved fusion protein, while exhibiting excellent preventive and therapeutic effects in human diseases and animal disease models.

Therefore, one of the advantages of the present invention is to provide an improved GLP-1 fusion protein with increased yield, activity, and/or extended half-life. Additionally, the improved GLP-1 fusion protein provided by the present invention demonstrates remarkable advantages in reducing blood glucose levels, including the reduction of glycated hemoglobin (HbA1c) levels and fasting plasma glucose (FPG) levels. Furthermore, it has a lower incidence of adverse reactions, such as hypoglycemia, nausea, diarrhea, and constipation.

In one aspect, the present invention provides a fusion protein comprising a GLP-1 peptide and an immunoglobulin Fc domain, wherein the GLP-1 peptide is covalently linked to the immunoglobulin Fc domain. The GLP-1 peptide is selected from human GLP-1 (7-37), human GLP-1 (7-36), and DPP-IV resistant human GLP-1. The GLP-1 peptide

contains one or more amino acid substitutions selected from the following group relative to native human GLP-1: A8G, G22E, and R36G. The immunoglobulin Fc domain comprises or is an IgG2-Fc domain, wherein the IgG2-Fc domain contains one or more amino acid substitutions selected from the following group: C222S, A330S, and P331S.

In one embodiment, the GLP-1 peptide has a certain level of hydroxylation at the 34th position lysine (K34) relative to native human GLP-1. In another embodiment, the GLP-1 peptide is substantially unoxidized at the 31st position tryptophan (W31) relative to native human GLP-1.

In one embodiment, the GLP-1 peptide has at least 90% sequence identity compared to the amino acid sequences represented by SEQ ID NO: 1, SEQ ID NO: 2, or SEQ ID NO: 3, and includes one or more amino acid substitutions relative to native human GLP-1 selected from the following group: A8G, G22E, and R36G.

In one embodiment, the IgG2-Fc domain is derived from human IgG2. In another embodiment, the IgG2-Fc domain has a certain level of oxidation at the 253rd methionine (M253) corresponding to SEQ ID NO: 7.

In one embodiment, the IgG2-Fc domain has at least 90% sequence identity compared to the amino acid sequences shown as SEQ ID NO: 5 or SEQ ID NO: 6, and it includes one or more amino acid substitutions selected from the following group: C222S, A330S, and P331S.

In one embodiment, the fusion protein of the present invention includes the GLP-1 peptide shown as SEQ ID NO: 3, and it includes the immunoglobulin Fc domain shown as SEQ ID NO: 6.

In one embodiment, the GLP-1 peptide is covalently linked to the immunoglobulin Fc domain through a linker. In one embodiment, the GLP-1 fusion protein of the present invention includes the GLP-1 peptide shown as SEQ ID NO: 3, the linker shown as SEQ ID NO: 9, and also includes the immunoglobulin Fc domain shown as SEQ ID NO: 6.

In one embodiment, the fusion protein also includes a signal peptide.

On another aspect, the present invention provides a dimer comprising two identical peptide chains connected by disulfide bonds, wherein each peptide chain comprises the fusion protein described herein.

On another aspect, the present invention provides a nucleotide sequence comprising the coding sequence for the fusion protein described herein.

On another aspect, the present invention provides a vector comprising the nucleotide sequence described in this document.

On another aspect, the present invention provides a cell comprising the nucleotide sequence or vector described in this document.

On another aspect, the present invention provides a composition comprising the fusion protein, dimer, nucleotide sequence, vector, or cell described in this document.

On another aspect, the present invention provides a method for constructing the cell described in this document, comprising:

a) Introducing the nucleic acid encoding the fusion protein into a vector to construct an expression vector.
b) Transferring the expression vector into recombinant or naturally expressing arginine hydroxylase cells to obtain recombinant cells.

Preferably, the level or activity of arginine hydroxylase expressed in the recombinant or naturally expressing cells is higher than the level or activity of arginine hydroxylase expressed in COS-7 cells.

More preferably, the cells are CHO cells, particularly CHO-K1 cells.

Furthermore, the preferred vector is the pKN012 vector.

In another aspect, the present invention provides a method for constructing recombinant cells, comprising:

c) Inserting the nucleotide sequence shown in SEQ ID NO: 26 into the NcoI and HindIII sites of the pKN012 vector to generate the pKN012-GLP1-IgG2/Fc expression vector.
d) Transfecting the pKN012-GLP1-IgG2/Fc expression vector into CHO-K1 cells to obtain recombinant cells.

In another aspect, the present invention provides a method for producing the fusion protein, comprising the step of obtaining the fusion protein using the cells described in this document or the cells prepared using the construction method.

In another aspect, the present invention provides a method for assessing the quality of the fusion protein, wherein the fusion protein comprises GLP-1 peptide and immunoglobulin IgG2-Fc domain. The method comprises the following steps: detecting the level of hydroxylation of the fusion protein at position K34 relative to native human GLP-1.

In another aspect, the present invention provides a use of the fusion protein, dimer, nucleotide, vector, cell, or composition as described herein, in the preparation of pharmaceuticals for the treatment or prevention of diseases.

The other features and advantages of the present invention will become apparent from the following detailed description in conjunction with the accompanying embodiments. The detailed description and specific examples provided herein are given for illustrative purposes while embodying the preferred embodiments of the present invention. Various

changes and modifications within the spirit and scope of the present invention will be readily apparent to those skilled in the art.

**BRIEF DESCRIPTION OF THE DRAWINGS:**

Figure 1 is a schematic diagram of the pKN012-GLP1-IgG2/Fc vector.

Figure 2 shows the mass spectrum of the enzymatic cleavage peptide segment 27-34: EFIAWLVK (+16Da) modified with GLP-1 fusion protein YN-011 after Lys-C digestion. A: Primary mass spectrum of EFIAWLVK; B: Primary mass spectrum of EFIAWLVK (+16 Da) modification; C: Secondary mass spectrum of EFIAWLVK; D: Secondary mass spectrum of EFIAWLVK (+16 Da) modification.

Figure 3A shows the UV detection data for the modified peptide 27-34 (aa21-28) with EFIAWLVK (+16 Da) modification, the unmodified peptide 54-79 (aa48-73) without the +16 Da modification, and the unmodified peptide 224-240 (aa218-234) after Lys-C digestion. Figure 3B is an enlarged view of the inset in Figure 3A.

Figure 4 depicts the average plasma concentration-time profiles following single and multiple subcutaneous injections of 1 mg, 2 mg, 3 mg, and 4 mg of YN-011 in T2DM subjects. Figures 4A and 4B represent the average plasma concentration-time profiles for single subcutaneous administration, while Figures 4C and 4D represent the average plasma concentration-time profiles for multiple subcutaneous administration.

Figure 5 presents the schematic diagram of a Phase IIa double-blind, placebo-controlled study conducted in T2DM subjects to assess the efficacy and safety of YN-011 administered subcutaneously at dose levels of 1 mg, 2 mg, 3 mg, and 4 mg. The filled triangles represent the administration of YN-011 at the specified dose levels, filled circles indicate the oral glucose tolerance test (OGTT) measurements, empty triangles represent adaptive dosing, and pentagrams represent safety evaluations.

Figure 6 displays the impact of multiple doses of YN-011 on fasting blood glucose levels in T2DM subjects. (At each time point, YN-011 demonstrated a significant difference compared to the placebo at dose levels of 3 mg and 4 mg, with a p-value of $< 0.05$.)

Figure 7 displays the impact of multiple doses of YN-011 on HbA1c levels in T2DM subjects. (At each time point, YN-011 demonstrated a significant difference compared to the placebo at dose levels of 1 mg, 3 mg, and 4 mg, with a p-value of $< 0.05$.)

Figure 8 represents the change in body weight (BW) of obese rhesus monkeys following repeated subcutaneous injections of YN-011.

Figure 9 illustrates the results after 48 hours of TNF-$\alpha$ stimulation on SH-SY5Y neuronal cells at different concentrations (*P<0.05 TNF-$\alpha$ (20 ng/ml) vs control; **P<0.01 TNF-$\alpha$ (40, 60, 80, 100 ng/ml) vs control).

Figure 10 demonstrates the effects of YN-011 and GABA on inhibiting TNF-$\alpha$-induced reduction in SH-SY5Y cell viability (**P<0.01 TNF-$\alpha$ vs control; #P<0.05 YN-011 (10 nM or 100 nM) or GABA (100 $\mu$M) + TNF-$\alpha$ vs TNF-$\alpha$; ##P<0.01 YN-011 (500 nM) + TNF-$\alpha$ vs TNF-$\alpha$; n = 6).

Figure 11 demonstrates a significant increase in SH-SY5Y cell viability with the combined use of YN-011 and GABA (**P<0.01 TNF-$\alpha$ vs control, #P<0.05 TNF-$\alpha$ + GABA + YN-011 vs TNF-$\alpha$ + YN-011; n = 6).

Figure 12 demonstrates the reduction of TNF-$\alpha$-induced apoptosis in SH-SY5Y neuronal cells by YN-011 (**P<0.01 TNF-$\alpha$ vs control, #P<0.05 10 nM YN-011 + TNF-$\alpha$ vs TNF-$\alpha$, ##P<0.01 100 nM or 500 nM YN-011 + TNF-$\alpha$ vs TNF-$\alpha$; n = 3).

Figure 13 illustrates the reduction of TNF-$\alpha$-induced apoptosis in SH-SY5Y neuronal cells by GABA (**P<0.01 TNF-$\alpha$ vs control, #P<0.05 10 $\mu$M or 100 $\mu$M GABA + TNF-$\alpha$ vs TNF-$\alpha$; n = 3).

Figure 14 demonstrates the reduction of TNF-$\alpha$-induced apoptosis in SH-SY5Y neuronal cells by the combined use of YN-011 and GABA (**P<0.01 TNF-$\alpha$ vs control, #P<0.05 GABA or YN-011 + TNF-$\alpha$ vs GABA + YN-011 + TNF-$\alpha$; n = 3).

Figure 15 is a live cell staining image that demonstrates the damage promoted by TNF-$\alpha$ in SH-SY5Y neuronal cells (**: significant; ***: highly significant).

Figure 16 is a live cell staining image that demonstrates the protective effect of YN-011 against TNF-$\alpha$-induced damage in neuronal cells (** P < 0.01 TNF-$\alpha$ vs control; # P < 0.05 10 nM YN-011 + TNF-$\alpha$ vs TNF-$\alpha$; ## P < 0.01 100 nM or 500 nM YN-011 + TNF-$\alpha$ vs TNF-$\alpha$; n = 3).

Figure 17 is a live cell staining image that demonstrates the protective effect of GABA against TNF-$\alpha$-induced damage in neuronal cells (** P < 0.01 TNF-$\alpha$ vs control; # P < 0.05 10 $\mu$M GABA + TNF-$\alpha$ vs TNF-$\alpha$; ## P < 0.01 100 $\mu$M GABA + TNF-$\alpha$ vs TNF-$\alpha$; n = 3).

Figure 18 is a live cell staining image that demonstrates the protective effect of the combined use of YN-011 and GABA against TNF-$\alpha$-induced damage in neuronal cells (** P < 0.01 TNF-$\alpha$ vs control; # P < 0.05 GABA or YN-011 + TNF-$\alpha$ vs GABA + YN-011 + TNF-$\alpha$; n = 3).

Figure 19 demonstrates the reduction of apoptosis in TNF-α-induced neuronal cells by YN-011 (** $P < 0.01$ TNF-α vs control; # $P < 0.05$ 100 nM or 500 nM YN-011 + TNF-α vs TNF-α; n = 3).

Figure 20 illustrates the reduction of apoptosis in TNF-α-induced neuronal cells by GABA (** $P < 0.01$ TNF-α vs control; # $P < 0.05$ 100 μM GABA + TNF-α vs TNF-α; n = 3).

Figure 21 demonstrates the reduction of apoptosis in TNF-α-induced neuronal cells by the combined use of YN-011 and GABA (** $P < 0.01$ TNF-α vs control; # $P < 0.05$ 100 nM YN-011 + TNF-α vs 100 μM GABA + 100 nM YN-011 + TNF-α; ## $P < 0.01$ 100 μM GABA + TNF-α vs 100 μM GABA + 100 nM YN-011 + TNF-α; n = 3).

Figure 22 illustrates the reduction of apoptosis in TNF-α-induced neuronal cells by YN-011 (** $P < 0.01$ TNF-α vs control; # $P < 0.05$ 10 nM, 100 nM, or 500 nM YN-011 + TNF-α vs TNF-α; n = 3).

Figure 23 demonstrates the reduction of apoptosis in TNF-α-induced neuronal cells by GABA (** $P < 0.01$ TNF-α vs control; # $P < 0.05$ 100 μM GABA + TNF-α vs TNF-α; n = 3).

Figure 24 illustrates the reduction of apoptosis in TNF-α-induced neuronal cells by the combined use of YN-011 and GABA (** $P < 0.01$ TNF-α vs control; # $P < 0.05$ TNF-α + 100 μM GABA + 100 nM YN-011 vs TNF-α + 100 μM GABA; ## $P < 0.05$ TNF-α + 100 μM GABA + 100 nM YN-011 vs TNF-α + 100 nM YN-011; n = 3).

Figure 25 demonstrates the reduction of inflammatory cytokine mRNA expression induced by Aβ1-42 oligomers in HMC3 microglial cells by GABA (* $P < 0.05$, ** $P < 0.01$ Aβ1-42 oligomers vs control; # $P < 0.05$ GABA + Aβ1-42 oligomers vs Aβ1-42 oligomers; n = 3).

Figure 26 demonstrates the reduction of inflammatory cytokine mRNA expression induced by Aβ1-42 oligomers in HMC3 microglial cells by YN-011 (* $P < 0.05$, ** $P < 0.01$ Aβ1-42 oligomers vs control; # $P < 0.05$, ## $P < 0.01$ YN-011 + Aβ1-42 oligomers vs Aβ1-42 oligomers; n = 3).

Figure 27 demonstrates the reduction of inflammatory cytokine mRNA expression induced by Aβ1-42 oligomers in HMC3 microglial cells by the combined use of YN-011 and GABA (* $P < 0.05$, ** $P < 0.01$ Aβ1-42 oligomers vs control; # $P < 0.05$, GABA + YN-011 + Aβ1-42 oligomers vs GABA + Aβ1-42 oligomers or YN-011 + Aβ1-42 oligomers; n = 3).

Figure 28 illustrates the reduction of inflammatory cytokine expression induced by Aβ1-42 oligomers in HMC3 microglial cells by GABA (** $P < 0.01$ Aβ1-42 oligomers vs control; # $P < 0.05$ GABA + Aβ1-42 oligomers vs Aβ1-42 oligomers; n = 3).

Figure 29 demonstrates the reduction of inflammatory cytokine expression induced by Aβ1-42 oligomers in HMC3 microglial cells by YN-011 (** $P < 0.01$ Aβ1-42 oligomers vs control; # $P < 0.05$ YN-011 + Aβ1-42 oligomers vs Aβ1-42 oligomers; n = 3).

Figure 30 demonstrates the reduction of inflammatory cytokine expression induced by Aβ1-42 oligomers in HMC3 microglial cells by the combination of YN-011 and GABA (** $P < 0.01$ Aβ1-42 oligomers vs control; # $P < 0.05$ GABA + YN-011 + Aβ1-42 oligomers vs GABA + Aβ1-42 oligomers or YN-011 + Aβ1-42 oligomers; n = 3).

## DETAILED DESCRIPTION OF THE DISCLOSURE:

The following is a detailed description to assist those skilled in the art in practicing the present invention. Unless otherwise defined, all technical and scientific terms used herein have the same meaning as commonly understood by a person skilled in the art to which the invention belongs. The terminology used in the present invention is for the purpose of describing specific embodiments only and is not intended to limit the invention. All publications, patent applications, patents, figures, and other references mentioned herein are incorporated by reference in their entirety into the present invention.

### I. Definitions

Unless otherwise indicated, the terms defined in this document and the terminology used should be understood as dictionary definitions, definitions in incorporated documents, and/or the generally known meanings of the defined terms.

All references, patents, and patent applications cited in this document are incorporated by reference in their entirety for the respective subject matter they are cited for, and in certain cases, may encompass the entire content of the referenced document.

All features disclosed in this specification may be combined in any manner. Each feature disclosed in this specification may be replaced by alternative features serving the same, equivalent, or similar purpose. Therefore, unless otherwise indicated, each feature disclosed is merely an example of a series of equivalent or similar features.

The terms "peptide," "polypeptide," and "protein" as used herein refer to amino acid chains comprising two or more naturally or non-naturally occurring amino acid residues, whether or not post-translationally modified (e.g., glycosylated or phosphorylated). The polypeptides disclosed in the present invention may include, for example, 3 to 3500 naturally or non-naturally occurring amino acid residues. The protein referred to can be a single peptide chain or a multi-subunit protein (e.g., composed of two or more polypeptides). The terms "peptide," "polypeptide," and "protein" as

described herein can be used interchangeably and may contain naturally occurring amino acids as well as non-naturally occurring amino acids or analogs or mimetics of amino acids. The peptides, polypeptides, or proteins described in this application can be obtained by any methods known in the art, including but not limited to natural isolation, recombinant expression, chemical synthesis, etc.

The term "amino acid" as used herein refers to organic compounds that contain an amino group (-NH2), a carboxyl group (-COOH), and a specific side chain characteristic of each amino acid. The names of amino acids in this application are also represented by standard single-letter or three-letter codes, summarized as follows:

| Name | Three-letter code | Single-letter code |
|---|---|---|
| Alanine | Ala | A |
| Arginine | Arg | R |
| Asparagine | Asn | N |
| Aspartic acid | Asp | D |
| Cysteine | Cys | C |
| Glutamate | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The term "GLP-1 peptide" as used in this document refers to GLP-1 receptor agonist peptides in which the amino acid residue at position 34 or corresponding to position 34 is lysine. Examples of such peptides include SEQ ID NO: 1 or SEQ ID NO: 2. Specifically, this includes but is not limited to GLP-1 (7-37), GLP-1 (7-36-NH2) (also interchangeably referred to as GLP-1 (7-36)), DPP-IV resistant GLP-1, and other GLP-1 analogs with lysine at position 34 or corresponding to position 34. For instance, GLP-1 peptides can include those derived from liraglutide (VICTOZA® from Novo Nordisk), semaglutide (OZEMPIC® from Novo Nordisk), albiglutide (SYNCRIA® from GlaxoSmithKline), taspoglutide (Roche), dulaglutide (TRULICITY® from Eli Lilly), or LY2428757 (Eli Lilly), as well as GLP-1 peptides disclosed in WO2021163972A1, CN111217915A, WO2011056713A2, and WO2000034332A1, which are incorporated by reference. For example, the mentioned peptides can be GLP-1 analogs containing the "KG" amino acid motif sequence.

The terms "polynucleotide" or "oligonucleotide" as used in this document refer to two or more covalently linked nucleotides. Unless otherwise specified in context, this term generally includes, but is not limited to, deoxyribonucleic acid (DNA) and ribonucleic acid (RNA), which can be single-stranded (ss) or double-stranded (ds). For example, the polynucleotide molecules or oligonucleotides of the present invention can be composed of single-stranded and double-stranded DNA, DNA with a mixture of single-stranded and double-stranded regions, single-stranded and double-stranded RNA, and RNA. The mixture of single-stranded and double-stranded regions may contain hybrid molecules comprising both DNA and RNA, which can be either single-stranded or, more typically, a mixture of single-stranded and double-stranded regions. Furthermore, the polynucleotide molecules can be composed of a three-stranded region containing RNA or DNA or both RNA and DNA. The term "oligonucleotide" as used in this document generally refers to polynucleotides with a length of no more than 200 base pairs and can be single-stranded or double-stranded. The

sequences provided in this document can be DNA sequences or RNA sequences. However, it should be understood that the provided sequences include both DNA and RNA, as well as complementary RNA and DNA sequences, unless otherwise specified in context. For example, the sequence 5'-GAATCC-3' should be understood to include 5'-GAAUCC-3', 5'-GGATTC-3', and 5'-GGAUUC-3'.

The term "sequence identity" or "sequence similarity" as used in this document refers to the percentage of sequence similarity between two peptide sequences or two nucleotide sequences. To determine the percentage of sequence identity between two amino acid sequences or two nucleotide sequences, the sequences are aligned for optimal comparison purposes (e.g., introducing gaps in the sequence of the first amino acid or nucleotide sequence to align it optimally with the second amino acid or nucleotide sequence), and then the amino acid residues or nucleotides at corresponding positions are compared. In other words, the percentage (%) sequence identity of an amino acid sequence (or nucleic acid sequence) can be calculated by dividing the number of identical amino acid residues (or bases) that are the same as the reference sequence being compared by the total number of amino acid residues (or bases) in the candidate or reference sequence (taking the shorter one as a reference). When a position in the first sequence is occupied by an amino acid residue or nucleotide that is the same as the corresponding position in the second sequence, the residue is considered identical at that position. The percentage of similarity between two sequences is a function of the number of shared identical positions in the sequence (i.e., similarity percentage = number of identical overlapping positions / total number of positions $\times$ 100%). In one embodiment, the lengths of the two sequences are the same. Mathematical algorithms can also be used to determine the percentage of sequence similarity between two sequences. One preferred, non-limiting example of a mathematical algorithm used for comparing two sequences is the Karlin-Altschul algorithm [14], which was later modified as the Karlin-Altschul algorithm [15]. This algorithm is incorporated into the NBLAST and XBLAST programs [16], which can be used with the NBLAST nucleotide program parameter set to perform BLAST nucleotide searches, e.g., score = 100, word length = 12, to obtain nucleotide sequences homologous to a specific polynucleotide molecule. BLAST protein searches can be performed using the XBLAST program parameter set, e.g., score = 50, word length = 3, to obtain amino acid sequences homologous to the protein molecules described in this document. Gapped BLAST can be used to obtain gapped alignments for comparison purposes [17]. Alternatively, PSI-BLAST can be used to perform iterative searches to detect distant relationships between molecules (Id.). When using BLAST, Gapped BLAST, and PSI-Blast programs, the default parameters of each program can be used (e.g., XBLAST and NBLAST) (see, e.g., the NCBI website). Another preferred, non-limiting example of a mathematical algorithm used for sequence comparison is the algorithm proposed by Myers and Miller [18], which is incorporated into the ALIGN program (version 2.0), a part of the GCG sequence alignment software package. When comparing amino acid sequences using the ALIGN program, the PAM120 weight residue table, a gap length penalty of 12, and a gap penalty of 4 can be used. The percentage of sequence identity between two sequences can be determined using techniques similar to those described above, with or without allowing gaps. When calculating the percentage of identity, typically only exact matches are considered.

In the present invention, "conservative amino acid substitution" refers to the substitution of one amino acid residue with another amino acid residue without eliminating the essential properties of the protein. Appropriate conservative amino acid substitutions can be performed by replacing amino acids with similar hydrophobicity, polarity, and R-chain length. Examples of conservative substitutions include substituting one non-polar (hydrophobic) residue with another non-polar residue (e.g., alanine, isoleucine, valine, leucine, or methionine), substituting one polar (hydrophilic) residue with another polar residue (e.g., between arginine and lysine), between glutamine and asparagine, between glycine and serine, substituting one basic residue with another basic residue (e.g., lysine, arginine, or histidine), or substituting one acidic residue with another acidic residue (e.g., aspartic acid or glutamic acid). The phrase "conservative substitution" also includes the use of chemically derived residues or non-natural amino acids in place of non-derived residues, provided that the peptide exhibits the necessary activity.

In the present invention, the term "fusion protein" refers to a protein that contains two or more peptides that form different functional domains. For example, the GLP-1 fusion protein described in this article includes the GLP-1 peptide and the immunoglobulin Fc domain.

In the present invention, the term "linker" refers to any chemical moiety that is capable of covalently connecting one portion to another portion. For example, the linker can be a sequence of 1, 2, 3, 4, or 5 amino acid residues, or an artificial amino acid sequence with a length ranging from 5 to 15, 20, 30, 50, or more amino acid residues, connected by peptide bonds and used to link one or more peptides. The linker may or may not have a secondary structure. Linker sequences are known in the art, for example, see Holliger et al., Proc. Natl. Acad. Sci. USA 90:6444-6448 (1993); Poljak et al., Structure 2:1121-1123 (1994).

In the present invention, the term "CH2" refers to the constant domain 2 of the immunoglobulin heavy chain. Similarly, the term "CH3" refers to the constant domain 3, which is another structural domain of the immunoglobulin heavy chain.

In the present invention, the term "hinge region" refers to the flexible region between the antigen-binding fragment (Fab) and the crystallizable fragment (Fc) in the context of immunoglobulins, such as IgG.

The term "vector" as used in this document refers to a vehicle capable of operatively inserting genetic elements,

allowing the genetic elements to be expressed, producing proteins, RNA, or DNA encoded by the genetic elements, or replicating the genetic elements. Vectors can be used to transform, transduce, or transfect host cells, enabling the carried genetic elements to be expressed within the host cells. Examples of vectors include plasmids, phages, cosmids, artificial chromosomes such as yeast artificial chromosomes (YACs), bacterial artificial chromosomes (BACs), or P1-derived artificial chromosomes (PACs), bacteriophages such as lambda phage or M13 phage, and animal viruses, among others. Vectors may contain various regulatory elements for expression control, including promoter sequences, transcription initiation sequences, enhancer sequences, selection elements, and reporter genes. Additionally, vectors may contain replication origin sites. Vectors may also include components that facilitate their entry into cells, including but not limited to viral particles, liposomes, or protein coats. Vectors can be expression vectors or cloning vectors.

The terms "DPPIV" and "DPP-IV" refer to dipeptidyl peptidase-IV, which is an enzyme that can inactivate native GLP-1.

The term "hydroxylation level" refers to the percentage of residues at amino acid positions in a peptide sample that are modified by hydroxylation. For example, a hydroxylation level of 20% means that 20% (by mole fraction) of peptide molecules are hydroxylated at specific amino acid positions. Modulators can be used to increase or decrease the hydroxylation level of expressed proteins. For instance, when present in the expression system, minoxidil and Zn2+ (e.g., from ZnSO4) can inhibit hydroxylation and reduce the hydroxylation level. The hydroxylation level can be measured using methods described in this embodiment or by mass spectrometry, as described by Hou et al. [13], or as further described in this document.

The term "oxidation level" refers to the percentage of residues at amino acid positions in a peptide sample that are modified by oxidation. For example, an oxidation level of 2% means that 2% (by mole fraction) of peptide molecules are oxidized at specific amino acid positions. The oxidation level can be measured using methods described in this embodiment or by mass spectrometry methods as described in WO2002046227A2, or according to the protein oxidation assay method by Bettinger et al. [19], or as described further in this document.

In the present invention, the term "pharmaceutical grade" refers to the chemical purity or proportion of a drug, biologic molecule, or reagent that meets the requirements for pharmaceutical production.

In the present invention, the term "treatment" refers to the administration of an effective amount of a compound, composition, or formulation to a subject, which can consist of a single administration or optionally include a series of procedures. As known in the art, "treatment" refers to a method employed to achieve beneficial or desired results, including clinical outcomes. Beneficial or desired clinical outcomes may include, but are not limited to, alleviation or improvement of one or more symptoms or conditions, reduction in the severity of a disease, stabilization (i.e., no worsening) of a disease state, prevention of disease progression, reversal of a disease, improvement or mitigation of a disease, and relief of symptoms associated with a disease (whether partial, complete, or temporary). Beneficial or desired clinical outcomes may include improved fasting blood glucose levels and/or HbA1c levels, weight loss, improved liver lipid content, as well as improved cognitive function, motor coordination, and so on.

In the present invention, the term "subject," also referred to as a "patient," as used in this document, includes all animals, including mammals, and preferably refers to humans. The term "subject" may also include livestock animals such as cattle, pigs, sheep, poultry, and horses; or rodents such as rats and mice; or primates such as apes, monkeys, chimpanzees, gorillas, orangutans, and baboons; or domesticated animals such as dogs and cats.

In the present invention, the term "pharmaceutically acceptable carrier" refers to any carrier, excipient, or formulation that is acceptable in biological or other aspects of pharmaceuticals. Unless the carrier, excipient, or formulation is incompatible with the active ingredient, its use in a therapeutic formulation is considered acceptable. The use of such pharmaceutically acceptable carriers is well-known in the art, and various components that can be included in pharmaceutical formulations are described in reference [20].

In the present invention, the term "therapeutically effective dose" refers to any dose that produces the desired effects in a subject, such as relieving symptoms, slowing disease progression, preventing disease onset, etc. This dose can be administered multiple times to achieve the desired effect over a period of time. In the context of this document, the term can refer to a dose that lowers blood glucose levels in the subject.

In the present invention, the term "co-administration" refers to the administration of two or more substances (such as compounds, compositions, etc.) to a subject, where these substances have biological activity. The specific dosing regimen will depend on the pharmacokinetics of the two or more substances in the presence of each other.

In understanding the scope of the present invention, the terms "contain" and its derivatives, as used in this document, are open-ended terms that specify the presence of the stated features, elements, components, moieties, integers, and/or steps, but do not exclude the presence of other unstated features, elements, components, groups, integers, and/or steps. The same applies to similar terms with similar meanings, such as the terms "include," "have," and their derivatives.

The terms "comprised of," "consisting of," and their derivatives, as used in this document, are closed-ended terms that specify the presence of the stated features, elements, components, groups, integers, and/or steps, and further exclude the presence of other unstated features, elements, components, groups, integers, and/or steps.

The numerical ranges enumerated in this document, through endpoint listing, include all numbers and fractions contained within that range (e.g., 1 to 5 includes 1, 1.5, 2, 2.75, 3, 3.90, 4, and 5). It should also be understood that all numbers and fractions are assumed to be modified by the term "about."

In addition, degree terms such as "substantially," "approximately," and "approximately" used in this document indicate a reasonable amount of deviation from the modifying term that would not significantly alter the final result. If such deviation does not negate the meaning of the modifier, these degree terms should be interpreted as including a deviation of at least ± 5% from the modifier. More specifically, the term "about" refers to a deviation of approximately ± 0.1-25%, ± 1-20%, ± 1-15%, ± 1-10%, such as up to 10% or up to 5% from the reference value.

As used in this specification and the appended claims, the singular forms "a," "an," and "one" include references to the plural, unless otherwise specified. Therefore, for example, a composition containing "a compound" includes a mixture of two or more compounds. It should also be noted that, unless otherwise specified, the term "or" is generally used in its inclusive sense, meaning "and/or."

Furthermore, the definitions and embodiments described in specific sections are intended to be applicable to other embodiments described in this document, as understood by those skilled in the art. For example, in the following paragraphs, different aspects of the invention are defined in more detail. Each aspect defined in this way can be combined with any other aspect or multiple aspects, unless explicitly stated otherwise. Specifically, any feature indicated as preferred or advantageous can be combined with any other feature indicated as preferred or advantageous.

While methods and materials similar or equivalent to those described in this document can be used in the practice or testing of the present invention, specific methods and materials are also described in this embodiment.

## II. Proteins and Fusion Proteins

The present invention provides stable GLP-1 peptides and fusion proteins comprising the GLP-1 peptide fused with, for example, the IgG/Fc domain.

One aspect of the present invention provides a GLP-1 fusion protein comprising a GLP-1 peptide and an immunoglobulin Fc domain. The fusion protein is covalently linked between the GLP-1 peptide and the immunoglobulin Fc domain. The GLP-1 peptide is selected from human GLP-1 (7-37), human GLP-1 (7-36), and DPP-IV resistant human GLP-1. The GLP-1 peptide contains one or more amino acid substitutions selected from the following group relative to native human GLP-1: A8G, G22E, and R36G. The immunoglobulin Fc domain is an IgG2-Fc domain, which contains one or more amino acid substitutions selected from the following group: C222S, A330S, and P331S.

### *GLP-1 peptide*

Surprisingly, the yield and activity of the GLP-1 fusion protein of the present invention can be enhanced by increasing the hydroxylation level of the GLP-1 peptide at the 34th position relative to native human GLP-1 (K34), reducing oxidation at the 31st tryptophan (W31) position relative to native human GLP-1, and/or introducing one or more site mutations in the GLP-1 peptide (such as A8G, G22E, R36G) or site mutations in the IgG2-Fc domain (such as C222S, A330S, P331S).

The unprocessed form of native human GLP-1 peptide consists of 37 amino acids, and its amino acid sequence is shown as SEQ ID NO: 43, commonly referred to as "GLP-1 (1-37)". The unprocessed form of native human GLP-1 peptide undergoes processing in the pancreas and small intestine to form GLP-1(7-37) or GLP-1(7-36). Unless otherwise specified, the amino acid positions of the GLP-1 peptides mentioned in this application correspond to the amino acid positions of SEQ ID NO: 43. For example, the K34 position of the GLP-1 peptides mentioned in this application corresponds to the 34th position of SEQ ID NO: 43. For instance, GLP-1(7-36) refers to a GLP-1 peptide fragment formed by amino acids between the 7th and 36th positions of SEQ ID NO: 43, and GLP-1(7-37) refers to a GLP-1 peptide fragment formed by amino acids between the 7th and 37th positions of SEQ ID NO: 43. In certain embodiments, the amino acid sequence of GLP-1(7-36) is shown as SEQ ID NO: 1. In certain embodiments, the amino acid sequence of GLP-1(7-37) is shown as SEQ ID NO: 2.

Unless otherwise specified, the naming convention for amino acid mutations mentioned in this application is as follows: the amino acid name before the mutation - the amino acid position where the mutation occurs - the amino acid name after the mutation. For example, the A8G mutation in the GLP-1 peptide refers to the substitution of alanine (A) at the 8th position corresponding to SEQ ID NO: 43 with glycine (G).

The hydroxylation level of the GLP-1 fusion protein of the present invention can be determined by various methods known in the art. For example, mass spectrometry as described by Hou et al. can be used for measurement [13]. Unless otherwise specified, the hydroxylation level referred to in this application is calculated based on the proportion of molecules. For instance, if out of 100g of the GLP-1 fusion protein described in the present invention, 10g of the GLP-1 fusion protein is hydroxylated at the K34 position of the GLP-1 peptide, while the remaining 90g of the GLP-1 fusion protein is not hydroxylated at the K34 position, the hydroxylation level of the GLP-1 fusion protein is considered to be 10%.

The GLP-1 fusion protein of the present invention has improved in vivo half-life and/or yield when produced through recombinant methods in certain embodiments. Therefore, the GLP-1 fusion protein, as well as reagents used for the preparation of the GLP-1 fusion protein, can be used for the formulation of pharmaceuticals.

In one embodiment, the GLP-1 peptide has a certain level of hydroxylation at the 34th position, which corresponds to the lysine residue (K34) in relation to natural human GLP-1. K34 refers to the lysine residue at the 34th position of the natural human GLP-1 peptide. Those skilled in the art are aware that the same lysine can correspond to different positions in other GLP-1 peptide sequences.

The term "GLP-1 fusion protein" can refer to multiple molecules, where the molecules can be hydroxylated or non-hydroxylated at K34, and the level of hydroxylation can vary among these molecules.

The present invention also provides compositions comprising GLP-1 fusion proteins. In one embodiment, the level of hydroxylation at K34 of the GLP-1 fusion protein is about 10% to 100%, for example, at least about 20%, at least about 26%, at least about 30%, at least about 40%, or at least about 50%. Any percentage or range between 10% and 100% is contemplated.

In one embodiment, the level of hydroxylation is between 10% and 100%, for example, equal to or greater than 10%, or equal to or greater than 15%, or equal to or greater than 20%, or equal to or greater than 26%, or equal to or greater than 30%, or equal to or greater than 35%, or equal to or greater than 40%, or equal to or greater than 45%, or equal to or greater than 50%, or equal to or greater than 55%, or equal to or greater than 60%, or equal to or greater than 65%, or equal to or greater than 70%, or equal to or greater than 75%, or equal to or greater than 80%, or equal to or greater than 85%, or equal to or greater than 90%, or equal to or greater than 95%.

As shown in the embodiments of this application, the yield of hydroxylated GLP-1 fusion protein is approximately 100 to 500 times higher compared to GLP-1 fusion proteins that are not detected to be hydroxylated at the K34 position of GLP-1.

The present invention further discovered that the GLP-1 peptide has a lower level of oxidation at the tryptophan residue at position 31 (W31) relative to native human GLP-1. The lower level of oxidation at W31 may be advantageous for the stability and/or activity of the GLP-1 fusion protein. In certain embodiments, the GLP-1 peptide is substantially unoxidized at the tryptophan residue at position 31 (W31) relative to native human GLP-1. In certain embodiments, the level of oxidation at W31 of the GLP-1 peptide, relative to native human GLP-1, is less than 0.5% (e.g., less than 0.4%, less than 0.3%, less than 0.2%, or less than 0.1%), or undetectable. W31 refers to the tryptophan residue at position 31 of the native human GLP-1 peptide. Those skilled in the art are aware that the same tryptophan residue can correspond to different positions in other GLP-1 peptides.

The detection of oxidation can be performed using methods known in the embodiments of this application or in the prior art, such as the mass spectrometry method described in WO2002046227A2 or the protein oxidation assay method by Bettinger et al. [19]. Unless otherwise specified, the oxidation level mentioned in this application is expressed as a percentage based on the number of molecules. For example, in 100g of the GLP-1 fusion protein disclosed in the present invention, if 10g of the GLP-1 fusion protein is oxidized at the tryptophan residue at position 31 (W31) of GLP-1, and the remaining 90g of the GLP-1 fusion protein is unoxidized at W31, the oxidation level of the GLP-1 fusion protein is considered to be 10%.

The GLP-1 peptide of the fusion protein disclosed in this article can be of human origin. In certain embodiments, the GLP-1 peptide is selected from human GLP-1 (7-37), human GLP-1 (7-36), and DPP-IV resistant human GLP-1, and it contains amino acid substitutions A8G and G22E relative to natural human GLP-1.

In certain embodiments, the GLP-1 peptide is selected from human GLP-1 (7-37), human GLP-1 (7-36), and DPP-IV resistant human GLP-1, and it contains amino acid substitutions A8G, G22E, and R36G relative to natural human GLP-1.

In some embodiments, the GLP-1 peptide is GLP-1 (7-37). In some embodiments, the GLP-1 peptide is GLP-1 (7-36). In some embodiments, the GLP-1 peptide is DPP-IV resistant GLP-1. In some embodiments, the GLP-1 peptide can contain amino acid substitutions, such as one, two, or three mutations in A8G, G22E, and R36G.

In some embodiments, the GLP-1 peptide disclosed in this application has a sequence identity of at least about 70%, at least about 75%, at least about 80%, at least about 85%, at least about 90%, or at least about 95% compared to the amino acid sequences shown in SEQ ID NO: 1, SEQ ID NO: 2, or SEQ ID NO: 3. It includes one or more amino acid substitutions selected from the following group relative to natural human GLP-1: A8G, G22E, and R36G. In some embodiments, the GLP-1 peptide disclosed in this application has a sequence identity of at least about 70%, at least about 75%, at least about 80%, at least about 85%, at least about 90%, or at least about 95% compared to the amino acid sequences shown in SEQ ID NO: 1, SEQ ID NO: 2, or SEQ ID NO: 3, and it stimulates insulin secretion in a glucose-dependent manner. In some embodiments, the GLP-1 peptide disclosed in this application has a sequence identity of at least about 70%, at least about 75%, at least about 80%, at least about 85%, at least about 90%, or at least about 95% compared to the amino acid sequences shown in SEQ ID NO: 1, SEQ ID NO: 2, or SEQ ID NO: 3. It includes one or more amino acid substitutions selected from the following group relative to natural human GLP-1: A8G, G22E, and R36G, and it stimulates insulin secretion in a glucose-dependent manner.

| NAME | Amino Acid Sequence | SEQ ID NO: |
|---|---|---|
| GLP-1 (1-37) | HDEFERHAEGTFTSDVSSYLEGQAAKEFIAW LVKGRG | 43 |
| GLP-1 (7-36) | HAEGTFTSDVSSYLEGQAAKEFIAWLVKGR | 1 |
| GLP-1 (7-37) | HAEGTFTSDVSSYLEGQAAKEFIAWLVKGRG | 2 |
| GLP-1 mutations (A8G, G22E, R36G) | HGEGTFTSDVSSYLEEQAAKEFIAWLVKGGG | 3 |

In certain embodiments, the GLP-1 peptide is human GLP-1 (7-37) and includes substitutions of A8G, G22E, and R36G relative to native human GLP-1.

In one embodiment, the amino acid sequence of the GLP-1 peptide includes the amino acid sequence shown in SEQ ID NO: 3. In certain embodiments, the amino acid sequence of the GLP-1 peptide is as shown in SEQ ID NO: 3.

In one embodiment, the GLP-1 peptide is DPP-IV resistant human GLP-1.

Native GLP-1 has a short circulating half-life (t1/2 < 2 minutes), primarily due to rapid enzymatic inactivation, including dipeptidyl peptidase-IV (DPP-IV), and/or renal clearance. Therefore, when using native GLP-1, continuous subcutaneous infusion via a pump is required to maintain its effects in the body [21]. Consequently, long-acting GLP-1 analogs resistant to degradation have been developed for therapeutic purposes. For instance, Dulaglutide is a DPP-IV protected GLP-1 analog fused with an IgG4/Fc fragment, with a half-life of 4.7-5.5 days [22].

*Fc domain*

In certain embodiments, the IgG2-Fc domain described in the present application is the Fc domain derived from human IgG2.

In this application, 'IgG2-Fc' and 'IgG2/Fc' can be used interchangeably and both refer to the Fc domain of immunoglobulin IgG2.

In some embodiments, the IgG2-Fc domain described in this application has a certain level of oxidation at the 253rd methionine (M253) corresponding to SEQ ID NO: 7. In certain embodiments, the IgG2-Fc domain described in this application exhibits reduced oxidation at the 253rd methionine (M253) corresponding to SEQ ID NO: 7. In some embodiments, the IgG2-Fc domain described in this application shows no oxidation at the 253rd methionine (M253) corresponding to SEQ ID NO: 7. In this application, M253 refers to the methionine residue at the 253rd position of IgG2 corresponding to SEQ ID NO: 7. Those skilled in the art will understand that the same methionine can correspond to different positions in other IgG peptides.

In some embodiments, the oxidation level of the IgG2-Fc domain at the M253 position corresponding to SEQ ID NO: 7 is less than or equal to about 15%, less than or equal to about 10%, less than or equal to about 9%, less than or equal to about 8%, less than or equal to about 7%, less than or equal to about 6%, less than or equal to about 5%, less than or equal to about 4%, less than or equal to about 3%, less than or equal to about 2%, less than or equal to about 1%, less than or equal to about 0.5%, or undetectable.

As mentioned before, the GLP-1 fusion protein can refer to multiple molecules, where the molecules can be oxidized or unoxidized at one or two positions, either at the M253 of the IgG2-Fc domain and/or the W31 of the GLP-1 peptide. The oxidation level of the molecules can vary. The oxidation occurs at the amino acid structures indicated by rectangular boxes in the following formulas:

| The M253 position of the IgG2-Fc domain | The W31 position of the GLP-1 peptide |
| --- | --- |
| 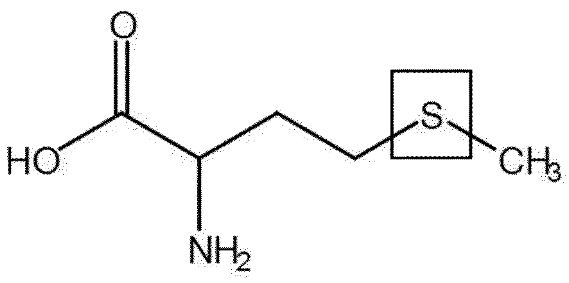 | 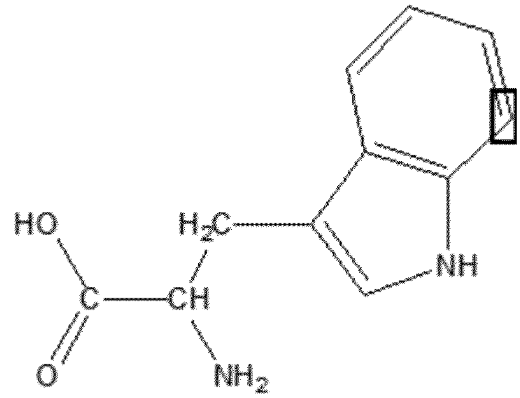 |

In certain embodiments, the GLP-1 fusion protein of the present invention shows no detectable oxidation at the W31 position of the GLP-1 peptide relative to native human GLP-1, and approximately 2% oxidation level at the M253 position of the IgG2-Fc domain. Other GLP-1 analog fusion proteins exhibit oxidation levels of 2-8% at the W31 (for the GLP-1 peptide) and/or M253 (for the IgG2-Fc domain).

The GLP-1 peptide in the fusion protein is covalently linked (directly or through a linker peptide) to the Fc portion of an immunoglobulin (Ig). In one embodiment, the immunoglobulin is IgG. The disclosed fusion protein in this article can have an IgG-Fc that is IgG2-Fc.

In one embodiment, the IgG2-Fc domain includes the C222S substitution. The C222S substitution in IgG2-Fc can increase the flexibility of the N-terminal hinge region by eliminating the disulfide bond between the two monomers of the dimer. The increased flexibility of the N-terminal hinge region can decrease the binding affinity to Fcγ receptors, thereby reducing antibody-dependent cell-mediated cytotoxicity (ADCC) and complement-dependent cytotoxicity (CDC).

In another embodiment, the IgG2-Fc domain includes the A330S and P331S substitutions. The A330S and P331S substitutions reduce the affinity for Fcγ receptors and C1q complement protein.

In one embodiment, the IgG2-Fc domain includes the C222S, A330S, and P331S substitutions.

Except for the amino acid position M253, which corresponds to the amino acid position of SEQ ID NO: 7, the other amino acid residue positions in the IgG2-Fc domain described in this application, including A330, P331, and C222, correspond to the positions of human IgG2 as shown in GenBank accession number QRG33935.1. The IgG2-Fc portion can consist of 228 amino acids, as shown in SEQ ID NO: 5, corresponding to amino acids 219 to 445 of human IgG2 as shown in, for example, GenBank accession number QRG33935.1. Those skilled in the art would easily recognize the residue positions of amino acids such as A330, P331, and C222 in the reference sequences SEQ ID NO: 5 or SEQ ID NO: 6 or in shorter or longer Fc fragments.

In one embodiment, the IgG2-Fc domain described in this application has a sequence identity of at least about 70%, at least about 75%, at least about 80%, at least about 85%, at least about 90%, or at least about 95% compared to the amino acid sequences shown in SEQ ID NO: 5 or SEQ ID NO: 6. It includes one or more amino acid substitutions selected from the following group: C222S, A330S, and P331S. The fusion protein, compared to GLP-1 peptides fused with no IgG/Fc domain or with IgG4/Fc domain, exhibits an improved half-life.

In one embodiment, the IgG2-Fc domain has a sequence identity of at least about 70%, at least about 75%, at least about 80%, at least about 85%, at least about 90%, or at least about 95% compared to the amino acid sequences shown in SEQ ID NO: 5 or SEQ ID NO: 6. It includes one or more amino acid substitutions selected from the following group: C222S, A330S, and P331S.

In one embodiment, the IgG2-Fc domain has a sequence identity of at least 90% compared to the amino acid sequences shown in SEQ ID NO: 5 or SEQ ID NO: 6, and it includes A330S and P331S substitutions. Preferably, the IgG2-Fc domain also includes a C222S substitution. More preferably, the amino acid sequence of the IgG2-Fc domain is as shown in SEQ ID NO: 6.

In one embodiment, the amino acid sequence of the GLP-1 peptide in the disclosed GLP-1 fusion protein is as shown in SEQ ID NO: 3, and the amino acid sequence of the immunoglobulin Fc domain is as shown in SEQ ID NO: 6.

| NAME | Amino Acid Sequence | SEQ ID NO: |
|---|---|---|
| IgG2/Fc domain (human IgG2 heavy chain Fc region residues 219-445) | ERKCCVECPPCPAPPVAGPSVFLFPPKPKDTLMI SRTPEVTCVVVDVSHEDPEVQFNWYVDGVEV HNAKTKPREEQFNSTFRVVSVLTVVHQDWLN GKEYKCKVSNKGLPAPIEKTISKTKGQPREPQV YTLPPSREEMTKNQVSLTCLVKGFYPSDIAVE WESNGQPENNYKTTPPMLDSDGSFFLYSKLTV DKSRWQQGNVFSCSVMHEALHNHYTQKSLSL SPG | 5 |
| IgG2/Fc domain mutation (human IgG2 heavy chain Fc region residues 219-445, including C222S, A330S, and P331S site mutations) | ERKSCVECPPCPAPPVAGPSVFLFPPKPKDTLMI SRTPEVTCVVVDVSHEDPEVQFNWYVDGVEV HNAKTKPREEQFNSTFRVVSVLTVVHQDWLN GKEYKCKVSNKGLPSSIEKTISKTKGQPREPQV YTLPPSREEMTKNQVSLTCLVKGFYPSDIAVE WESNGQPENNYKTTPPMLDSDGSFFLYSKLTV DKSRWQQGNVFSCSVMHEALHNHYTQKSLSL SPG | 6 |

The corresponding positions of the A8G, G22E, R36G substitutions, and K34 hydroxylation in the GLP-1 peptide mentioned in this application, relative to SEQ ID NO: 3 and SEQ ID NO: 7, are shown in the following table. Additionally, the corresponding positions of the C222S, A330S, P331S substitutions, and M253 oxidation on the IgG2-Fc domain mentioned in this application, relative to QRG33935.1 and SEQ ID NO: 7, are also shown in the following table.

| Modified Sites | Position in SEQ ID NO: 6 of IgG2 (219-445) Relative to the sequence shown in QRG33935.1 | Position in SEQ ID NO: 6 of IgG2 (219-445) Relative to the sequence shown in QRG33935.1 | Position in SEQ ID NO: 7 |
|---|---|---|---|
| A8G | AA2 | | AA2 |
| G22E | AA16 | | AA16 |
| R36G | AA30 | | AA30 |
| Hydroxylation of K34 | AA28 | | AA28 |
| Oxidation at W31 relative to native human GLP-1 | AA25 | | AA25 |
| Oxidation at M253 | | AA427 | AA253 |
| C222S | | AA222 | AA48 |
| A330S | | AA330 | AA155 |

(continued)

| Modified Sites | Position in SEQ ID NO: 6 of IgG2 (219-445) Relative to the sequence shown in QRG33935.1 | Position in SEQ ID NO: 6 of IgG2 (219-445) Relative to the sequence shown in QRG33935.1 | Position in SEQ ID NO: 7 |
|---|---|---|---|
| P331S | | AA331 | AA156 |

In one embodiment, the GLP-1 peptide is located at the N-terminus of the immunoglobulin Fc domain. In another embodiment, the GLP-1 peptide is located at the C-terminus of the immunoglobulin Fc domain.

*Linker*

In one embodiment, the GLP-1 peptide is directly covalently linked to the immunoglobulin Fc domain.
In one embodiment, the GLP-1 peptide is covalently linked to the immunoglobulin Fc domain through a linker.
In one embodiment, the linker is selected from the following group: cleavable linker, non-cleavable linker, flexible linker, rigid linker, helical linker, and non-helical linker.
In one embodiment, the linker includes a connecting peptide.
The GLP-1 peptide and IgG-Fc domain of the fusion protein disclosed in this article, such as the IgG2-Fc domain, can be linked together through a connecting peptide. The term "connecting peptide," as used in this article, refers to any portion that joins different functional domains of a peptide. The connecting peptide can have any suitable length and structure.
In the present invention, the term "connecting peptide" refers to a preferred segment of amino acids that links different functional domains of a peptide together. Various connecting peptides have been considered, and the connecting peptide can have any suitable length and structure.
In the present invention, the term "cleavable linker" refers to a linker that is sensitive to intracellular proteases, pH, or chemical factors, and is easily cleaved in the presence of such factors.
In the present invention, the term "non-cleavable linker" refers to a linker that is stable and resistant to intracellular proteases, pH, or chemical factors, and is not easily cleaved.
In the present invention, the term "flexible linker" refers to a linker that increases spatial flexibility when connecting different protein components, allowing the protein components to fold and adopt their conformations without significant interference from each other.
In the present invention, the term "rigid linker" refers to a linker that maintains a fixed distance between different protein components when connecting them.
In the present invention, the term "helical linker" refers to a linker where the rigid units within it can form a helical structure (such as an $\alpha$-helix) either internally or between identical adjacent sequences, thus providing a linker that imparts a relatively stable conformation to the resulting fusion protein.
In the present invention, the term "non-helical linker" refers to a linker that is unable to form a helical structure.
In one embodiment, the linker comprises a sequence containing glycine and serine residues. Preferably, the linker containing glycine and serine residues includes one, two, three, four, or more repetitions as shown in SEQ ID NO: 39 (GGGS), SEQ ID NO: 40 (GGGGS), SEQ ID NO: 41 (GGGGGS), or SEQ ID NO: 42 (GGGGGGGS).
In one embodiment, the linker comprises an amino acid sequence selected from the following group: SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, and SEQ ID NO: 19.

| Name | Amino acid sequence | SEQ ID NO: |
|---|---|---|
| Linker peptide | SSGAPPPSGGGGS | 9 |
| Linker peptide | GGGGSGGGGSGGGGS | 10 |
| Linker peptide | GGGGSGGGGSGGGGSGGGGS | 11 |
| Linker peptide | GSTGGGGSGKPGSGEGGGGS | 12 |
| Linker peptide | ESGRSGGGGSGGGGS | 13 |
| Linker peptide | EGKSSGSGSESKST | 14 |

(continued)

| Name | Amino acid sequence | SEQ ID NO: |
|---|---|---|
| Linker peptide | EGKSSGSGSESKSTQ | 15 |
| Linker peptide | EGKSSGSGSESKVD | 16 |
| Linker peptide | GSTSGSGKSSEGKG | 17 |
| Linker peptide | KESGSVSSEQLAQFRSLD | 18 |
| Linker peptide | ESGSVSSEELAFRSLD | 19 |
| Linker peptide | GGGS | 39 |
| Linker peptide | GGGGS | 40 |
| Linker peptide | GGGGGS | 41 |
| Linker peptide | GGGGGGGS | 42 |

In one embodiment, the linker peptide that connects the GLP-1 peptide and IgG2/Fc domain can have a sequence identity of at least about 70%, at least about 75%, at least about 80%, at least about 85%, at least about 90%, or at least about 95% with the amino acid sequence shown in SEQ ID NO: 9. In one embodiment, the linker peptide includes the amino acid sequence shown in SEQ ID NO: 9. In one embodiment, the amino acid sequence of the linker peptide is the same as the sequence shown in SEQ ID NO: 9.

In one embodiment, in the GLP-1 fusion protein described in this application, the amino acid sequence of the GLP-1 peptide is as shown in SEQ ID NO: 3, the amino acid sequence of the immunoglobulin Fc domain is as shown in SEQ ID NO: 6, and the amino acid sequence of the linker peptide is as shown in SEQ ID NO: 9.

*Fusion protein*

In one embodiment, the fusion protein disclosed in the present application shares at least about 70%, at least about 75%, at least about 80%, at least about 85%, at least about 90%, or at least about 95% sequence identity with the amino acid sequence represented by SEQ ID NO: 7. In another embodiment, the fusion protein disclosed in the present application shares at least about 70%, at least about 75%, at least about 80%, at least about 85%, at least about 90%, or at least about 95% sequence identity with the amino acid sequence represented by SEQ ID NO: 7, wherein the GLP-1 peptide contains A8G, G22E, and R36G substitutions, and the IgG2-Fc domain contains C222S, A330S, and P331S substitutions. In yet another embodiment, the fusion protein disclosed in the present application shares at least about 70%, at least about 75%, at least about 80%, at least about 85%, at least about 90%, or at least about 95% sequence identity with the amino acid sequence represented by SEQ ID NO: 7, wherein the GLP-1 peptide contains A8G, G22E, R36G substitutions, the IgG2-Fc domain contains C222S, A330S, P331S substitutions, and it stimulates insulin secretion from β-cells in a glucose-dependent manner. In a further embodiment, the fusion protein disclosed in the present application shares at least about 70%, at least about 75%, at least about 80%, at least about 85%, at least about 90%, or at least about 95% sequence identity with the amino acid sequence represented by SEQ ID NO: 7, wherein the GLP-1 peptide contains A8G, G22E, R36G substitutions, the IgG2-Fc domain contains C222S, A330S, P331S substitutions, and it stimulates insulin secretion from β-cells in a glucose-dependent manner.

In one embodiment, the fusion protein disclosed in the present application has the amino acid sequence represented by SEQ ID NO: 7, or an amino acid sequence that shares at least 80% (e.g., at least about 85%, at least about 90%, or at least about 95%) sequence identity with SEQ ID NO: 7.

In one embodiment, the fusion protein disclosed in the present application has the amino acid sequence represented by SEQ ID NO: 7, or an amino acid sequence that shares at least about 90% sequence identity with SEQ ID NO: 7.

In one embodiment, the fusion protein disclosed in the present application has the amino acid sequence represented by SEQ ID NO: 7. In another embodiment, the fusion protein also has the amino acid sequence represented by SEQ ID NO: 7.

| Name | Amino acid sequence | SEQ ID NO: |
|---|---|---|
| GLP-1-IgG2/Fc fusion protein (comprising GLP-1, a linker peptide, and IgG2/Fc domain) | HGEGTFTSDVSSYLEEQAAKEFIAWLVKGGGSSGAP PPSGGGGSERKSCVECPPCPAPPVAGPSVFLFPPKPK DTLMISRTPEVTCVVVDVSHEDPEVQFNWYVDGVE VHNAKTKPREEQFNSTFRVVSVLTVVHQDWLNGKE YKCKVSNKGLPSSIEKTISKTKGQPREPQVYTLPPSRE EMTKNQVSLTCLVKGFYPSDIAVEWESNGQPENNY KTTPPMLDSDGSFFLYSKLTVDKSRWQQGNVFSCSV MHEALHNHYTQKSLSLSPG | 7 |

As demonstrated in the exemplary embodiments, the GLP-1 fusion protein disclosed in this article, through sequence modifications, has an extended half-life. For instance, the GLP-1 fusion protein YN-011 exhibits a half-life of approximately 8.6 days (207 hours).

In one embodiment of the present invention, the GLP-1 fusion protein also includes a signal peptide.

The term "signal peptide," as referred to in this document, refers to a peptide that directs the secretion of the fusion protein into the extracellular medium. This peptide can also be referred to as a "leader peptide," "peptide precursor," "prepeptide," and the like. The use of signal peptides to guide protein secretion is known in the art (e.g., U.S. Patent US8658174, the contents of which are incorporated herein by reference in their entirety). Examples of signal peptides include, but are not limited to, human CD33 signal peptide, human growth hormone-releasing hormone (GHRH) signal peptide, human $\alpha$-1-microglobulin/bikunin precursor (AMBP) signal peptide, green fluorescent protein signal peptide, mouse immunoglobulin heavy chain signal peptide, and mouse immunoglobulin $\kappa$ light chain signal peptide. Signal peptides are cleaved during the secretion process. In some embodiments, the cleavage of the signal peptide results in an active histidine residue at the N-terminus of the GLP-1 peptide.

In one embodiment, the signal peptide is the human CD33 signal peptide.

In one embodiment, the signal peptide has at least approximately 70%, at least approximately 75%, at least approximately 80%, at least approximately 85%, at least approximately 90%, or at least approximately 95% sequence identity with the amino acid sequence as shown in SEQ ID NO: 4 (MPLLLLLPLLWAGALA), and allows for the secretion of the fusion protein. In one embodiment, the signal peptide has the amino acid sequence as shown in SEQ ID NO: 4 or has at least 90% sequence identity with SEQ ID NO: 4. In one embodiment, the signal peptide has the amino acid sequence as shown in SEQ ID NO: 4.

In one embodiment, the fusion protein described in the present application has at least approximately 70%, at least approximately 75%, at least approximately 80%, at least approximately 85%, at least approximately 90%, or at least approximately 95% sequence identity with the amino acid sequence as shown in SEQ ID NO: 8. In one embodiment, the fusion protein described in the present application has at least approximately 70%, at least approximately 75%, at least approximately 80%, at least approximately 85%, at least approximately 90%, or at least approximately 95% sequence identity with the amino acid sequence as shown in SEQ ID NO: 8, and the GLP-1 peptide contains substitutions such as A8G, G22E, R36G, and the IgG2-Fc domain contains substitutions such as C222S, A330S, and P331S. In one embodiment, the fusion protein described in the present application has at least approximately 70%, at least approximately 75%, at least approximately 80%, at least approximately 85%, at least approximately 90%, or at least approximately 95% sequence identity with the amino acid sequence as shown in SEQ ID NO: 8, and stimulates insulin secretion in a glucose-dependent manner. In one embodiment, the fusion protein described in the present application has at least approximately 70%, at least approximately 75%, at least approximately 80%, at least approximately 85%, at least approximately 90%, or at least approximately 95% sequence identity with the amino acid sequence as shown in SEQ ID NO: 8, and the GLP-1 peptide contains substitutions such as A8G, G22E, R36G, and the IgG2-Fc domain contains substitutions such as C222S, A330S, and P331S, and stimulates insulin secretion in a glucose-dependent manner.

In one embodiment, the fusion protein described in the present application has the amino acid sequence as shown in SEQ ID NO: 8 or has at least 80% sequence identity (for example, at least approximately 85%, at least approximately 90%, or at least approximately 95%) with the amino acid sequence as shown in SEQ ID NO: 8.

In one embodiment, the fusion protein described in the present application has the amino acid sequence as shown in SEQ ID NO: 8 or has at least approximately 90% sequence identity with the amino acid sequence as shown in SEQ ID NO: 8.

In one embodiment, the fusion protein described in the present application has the amino acid sequence as shown in SEQ ID NO: 8. In another embodiment, the fusion protein also has the amino acid sequence as shown in SEQ ID NO: 8.

| Name | Amino acid sequence | SEQ ID NO: |
|---|---|---|
| GLP-1-IgG2/Fc fusion protein (including signal peptide, GLP-1, linker peptide, and IgG2/Fc domain) | MPLLLLLPLLWAGALAHGEGTFTSDVSSYLEEQAAK EFIAWLVKGGGSSGAPPPSGGGGSERKSCVECPPCPA PPVAGPSVFLFPPKPKDTLMISRTPEVTCVVVDVSHE DPEVQFNWYVDGVEVHNAKTKPREEQFNSTFRVVS VLTVVHQDWLNGKEYKCKVSNKGLPSSIEKTISKTK GQPREPQVYTLPPSREEMTKNQVSLTCLVKGFYPSDI AVEWESNGQPENNYKTTPPMLDSDGSFFLYSKLTVD KSRWQQGNVFSCSVMHEALHNHYTQKSLSLSPG | 8 |

In one embodiment, the fusion protein described in the present application has a half-life of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, or at least 14 days in the subject's body.

In another preferred embodiment, the GLP-1 fusion protein is pharmaceutical grade.

Another aspect of the present invention provides a dimer comprising two identical peptide chains connected by disulfide bonds, wherein each peptide chain comprises the fusion protein as described in this disclosure.

**III. Nucleic Acid**

In another aspect, the present invention also provides a polynucleotide comprising nucleotides that encode the peptides or polypeptides described in the present invention, such as GLP-1 peptide, Fc fragment, or fusion protein.

In another aspect, the present invention also provides a nucleic acid molecule comprising nucleotides that encode the peptides or polypeptides described in this document, such as GLP-1 peptide, Fc fragment, or fusion protein.

The term "nucleic acid" or "nucleotide" as used in this application refers to single-stranded or double-stranded forms of deoxyribonucleic acid (DNA) or ribonucleic acid (RNA) and their polymers. Unless otherwise specified, specific nucleotide sequences also implicitly encompass conservative variants thereof, such as degenerate codon substitutions, alleles, orthologs, SNPs, complementary sequences, and explicitly disclosed sequences. Specifically, degenerate codon substitutions can be achieved by generating sequences wherein one or more selected (or all) codons at the third position are replaced with mixed bases and/or deoxyinosine residues (see Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

In one embodiment, the nucleic acids described in this application are codon-optimized, for example, optimized for human use. The nucleic acid molecules described herein can be used in the methods disclosed in this document.

Peptides and fusion proteins can be synthesized using standard protein chemistry techniques [23]. Additionally, automated peptide synthesizers can be purchased, such as the Advanced ChemTech Mode1396 or Milligen/Biosearch 9600, for the synthesis of peptides and fusion proteins. Furthermore, the peptides, polypeptides, or their fragments or variants described in this article can also be produced through recombinant expression using various known expression systems in the field.

In one embodiment, the disclosed nucleic acid comprises a nucleotide sequence as shown in SEQ ID NO: 26 or SEQ ID NO: 27, or a nucleotide sequence that shares at least 70% sequence identity with SEQ ID NO: 26 or SEQ ID NO: 27.

In one embodiment, the disclosed nucleic acid has at least approximately 70%, at least approximately 75%, at

least approximately 80%, at least approximately 85%, at least approximately 90%, at least approximately 95%, at least approximately 96%, at least approximately 97%, at least approximately 98%, or at least approximately 99% sequence identity with SEQ ID NO: 26. In another embodiment, the disclosed nucleic acid is a nucleotide sequence as shown in SEQ ID NO: 26.

In one embodiment, the disclosed nucleic acid has at least approximately 70%, at least approximately 75%, at least approximately 80%, at least approximately 85%, at least approximately 90%, at least approximately 95%, at least approximately 96%, at least approximately 97%, at least approximately 98%, or at least approximately 99% sequence identity with SEQ ID NO: 27. In another embodiment, the disclosed nucleic acid is a nucleotide sequence as shown in SEQ ID NO: 27.

In some embodiments, the disclosed multiple nucleic acid comprises any one of the multiple nucleic acid sequences shown as SEQ ID NO: 20, SEQ ID NO: 21, or SEQ ID NO: 22, or a multiple nucleic acid sequence that has at least approximately 70%, at least approximately 75%, at least approximately 80%, at least approximately 85%, at least approximately 90%, at least approximately 95%, at least approximately 96%, at least approximately 97%, at least approximately 98%, or at least approximately 99% sequence identity with SEQ ID NO: 20, SEQ ID NO: 21, or SEQ ID NO: 22.

In some embodiments, the disclosed multiple nucleic acid comprises the multiple nucleic acid sequence shown as SEQ ID NO: 24 or the multiple nucleic acid sequence shown as SEQ ID NO: 25, or a multiple nucleic acid sequence that has at least approximately 70% (e.g., at least about 70%, at least about 75%, at least about 80%, at least about 85%, at least about 90%, at least about 95%, at least about 96%, at least about 97%, at least about 98%, or at least about 99%) sequence identity with SEQ ID NO: 24 or SEQ ID NO: 25.

In certain embodiments, the disclosed multiple nucleic acid comprises the multiple nucleic acid sequence shown as SEQ ID NO: 23 or a multiple nucleic acid sequence that has at least approximately 70% (e.g., at least about 70%, at least about 75%, at least about 80%, at least about 85%, at least about 90%, at least about 95%, at least about 96%, at least about 97%, at least about 98%, or at least about 99%) sequence identity with SEQ ID NO: 23.

In certain embodiments, the disclosed multiple nucleic acid comprises any one of the multiple nucleic acid sequences shown as SEQ ID NO: 28 to SEQ ID NO: 38, or a multiple nucleic acid sequence that has at least approximately 70% (e.g., at least about 70%, at least about 75%, at least about 80%, at least about 85%, at least about 90%, at least about 95%, at least about 96%, at least about 97%, at least about 98%, or at least about 99%) sequence identity with any one of the sequences represented by SEQ ID NO: 28 to SEQ ID NO: 38.

The sequences of SEQ ID NO: 20~38 are shown in the following table.

| Name | Nucleotide sequences | SEQ ID NO |
|---|---|---|
| GLP-1(7-36) | CACGCCGAGGGCACCTTCACCAGCGACGTGAGCAGCT<br>ACCTGGAGGGCCAGGCCGCCAAG<br>GAGTTCATCGCCTGGCTGGTGAAGGGCAGG | 20 |
| GLP-1(7-37) | CACGCCGAGGGCACCTTCACCAGCGACGTGAGCAGCT<br>ACCTGGAGGGCCAGGCCGCCAAG<br>GAGTTCATCGCCTGGCTGGTGAAGGGCAGGGGC | 21 |
| GLP-1 mutations (A8G, G22E, R36G) | CACGGCGAGGGCACCTTCACCAGCGACGTGAGCAGCT<br>ACCTGGAGGAGCAGGCCGCCAAG<br>GAGTTCATCGCCTGGCTGGTGAAGGGCGGCGGC | 22 |
| CD33 signal peptide | ATGCCCCTGCTGCTGCTGCTGCCCCTGCTGTGGGCCGG<br>CGCCCTGGCC | 23 |

(continued)

| Name | Nucleotide sequences | SEQ ID NO |
|---|---|---|
| IgG2/Fc domain (human IgG2 heavy chain Fc region residues 219-445) | GAGAGGAAGTGCTGCGTGGAGTGCCCCCCCTGCCCCG<br>CCCCCCCCGTGGCCGGCCCCAGC<br>GTGTTCCTGTTCCCCCCCAAGCCCAAGGACACCCTGAT<br>GATCAGCAGGACCCCCGAGGTG<br>ACCTGCGTGGTGGTGGACGTGAGCCACGAGGACCCCG<br>AGGTGCAGTTCAACTGGTACGTG<br>GACGGCGTGGAGGTGCACAACGCCAAGACCAAGCCCA<br>GGGAGGAGCAGTTCAACAGCACC<br>TTCAGGGTGGTGAGCGTGCTGACCGTGGTGCACCAGG<br>ACTGGCTGAACGGCAAGGAGTAC<br>AAGTGCAAGGTGAGCAACAAGGGCCTGCCCGCCCCCA<br>TCGAGAAGACCATCAGCAAGACC<br>AAGGGCCAGCCCAGGGAGCCCCAGGTGTACACCCTGC<br>CCCCCAGCAGGGAGGAGATGACC<br>AAGAACCAGGTGAGCCTGACCTGCCTGGTGAAGGGCT<br>TCTACCCCAGCGACATCGCCGTG<br>GAGTGGGAGAGCAACGGCCAGCCCGAGAACAACTACA<br>AGACCACCCCCCCCATGCTGGAC<br>AGCGACGGCAGCTTCTTCCTGTACAGCAAGCTGACCGT<br>GGACAAGAGCAGGTGGCAGCAG<br>GGCAACGTGTTCAGCTGCAGCGTGATGCACGAGGCCC<br>TGCACAACCACTACACCCAGAAG<br>AGCCTGAGCCTGAGCCCCGGC | 24 |

(continued)

| Name | Nucleotide sequences | SEQ ID NO |
|---|---|---|
| IgG2/Fc domain mutation (human IgG2 heavy chain Fc region residues 219-445, including mutations at positions C222S, A330S, and P331S) | GAGAGGAAGAGCTGCGTGGAGTGCCCCCCCTGCCCCG CCCCCCCCGTGGCCGGCCCCAGC GTGTTCCTGTTCCCCCCCAAGCCCAAGGACACCCTGAT GATCAGCAGGACCCCCGAGGTG ACCTGCGTGGTGGTGGACGTGAGCCACGAGGACCCCG AGGTGCAGTTCAACTGGTACGTG GACGGCGTGGAGGTGCACAACGCCAAGACCAAGCCCA GGGAGGAGCAGTTCAACAGCACC TTCAGGGTGGTGAGCGTGCTGACCGTGGTGCACCAGG ACTGGCTGAACGGCAAGGAGTAC AAGTGCAAGGTGAGCAACAAGGGCCTGCCCAGCAGCA TCGAGAAGACCATCAGCAAGACC AAGGGCCAGCCCAGGGAGCCCCAGGTGTACACCCTGC CCCCCAGCAGGGAGGAGATGACC AAGAACCAGGTGAGCCTGACCTGCCTGGTGAAGGGCT TCTACCCCAGCGACATCGCCGTG GAGTGGGAGAGCAACGGCCAGCCCGAGAACAACTACA AGACCACCCCCCCCATGCTGGAC AGCGACGGCAGCTTCTTCCTGTACAGCAAGCTGACCGT GGACAAGAGCAGGTGGCAGCAG GGCAACGTGTTCAGCTGCAGCGTGATGCACGAGGCCC TGCACAACCACTACACCCAGAAG AGCCTGAGCCTGAGCCCCGGC | 25 |
| GLP-1-IgG2/Fc fusion protein (containing GLP-1, linker peptide, | CACGGCGAGGGCACCTTCACCAGCGACGTGAGCAGCT ACCTGGAGGAGCAGGCCGCCAAG GAGTTCATCGCCTGGCTGGTGAAGGGCGGCGGCAGCA GCGGCGCCCCCCCCCCAGCGGC | 26 |

(continued)

| Name | Nucleotide sequences | SEQ ID NO |
|---|---|---|
| and IgG2/Fc domain) | GGCGGCGGCAGCGAGAGGAAGAGCTGCGTGGAGTGCC CCCCCTGCCCCGCCCCCCCCGTG GCCGGCCCCAGCGTGTTCCTGTTCCCCCCCAAGCCCAA GGACACCCTGATGATCAGCAGG ACCCCCGAGGTGACCTGCGTGGTGGTGGACGTGAGCC ACGAGGACCCCGAGGTGCAGTTC AACTGGTACGTGGACGGCGTGGAGGTGCACAACGCCA AGACCAAGCCCAGGGAGGAGCAG TTCAACAGCACCTTCAGGGTGGTGAGCGTGCTGACCGT GGTGCACCAGGACTGGCTGAAC GGCAAGGAGTACAAGTGCAAGGTGAGCAACAAGGGC CTGCCCAGCAGCATCGAGAAGACC ATCAGCAAGACCAAGGGCCAGCCCAGGGAGCCCCAGG TGTACACCCTGCCCCCCAGCAGG GAGGAGATGACCAAGAACCAGGTGAGCCTGACCTGCC TGGTGAAGGGCTTCTACCCCAGC GACATCGCCGTGGAGTGGGAGAGCAACGGCCAGCCCG AGAACAACTACAAGACCACCCCC CCCATGCTGGACAGCGACGGCAGCTTCTTCCTGTACAG CAAGCTGACCGTGGACAAGAGC AGGTGGCAGCAGGGCAACGTGTTCAGCTGCAGCGTGA TGCACGAGGCCCTGCACAACCAC TACACCCAGAAGAGCCTGAGCCTGAGCCCCGGC | |

(continued)

| Name | Nucleotide sequences | SEQ ID NO |
|---|---|---|
| GLP-1-IgG2/Fc fusion protein (containing signal peptide, GLP-1, linker peptide, and IgG2/Fc domain) | ATGCCCCTGCTGCTGCTGCTGCCCCTGCTGTGGGCCGG CGCCCTGGCCCACGGCGAGGGC ACCTTCACCAGCGACGTGAGCAGCTACCTGGAGGAGC AGGCCGCCAAGGAGTTCATCGCC TGGCTGGTGAAGGGCGGCGGCAGCAGCGGCGCCCCCC CCCCCAGCGGCGGCGGCGGCAGC GAGAGGAAGAGCTGCGTGGAGTGCCCCCCCTGCCCCG CCCCCCCCGTGGCCGGCCCCAGC GTGTTCCTGTTCCCCCCCAAGCCCAAGGACACCCTGAT GATCAGCAGGACCCCCGAGGTG ACCTGCGTGGTGGTGGACGTGAGCCACGAGGACCCCG AGGTGCAGTTCAACTGGTACGTG GACGGCGTGGAGGTGCACAACGCCAAGACCAAGCCCA GGGAGGAGCAGTTCAACAGCACC TTCAGGGTGGTGAGCGTGCTGACCGTGGTGCACCAGG ACTGGCTGAACGGCAAGGAGTAC AAGTGCAAGGTGAGCAACAAGGGCCTGCCCAGCAGCA TCGAGAAGACCATCAGCAAGACC AAGGGCCAGCCCAGGGAGCCCCAGGTGTACACCCTGC CCCCCAGCAGGGAGGAGATGACC AAGAACCAGGTGAGCCTGACCTGCCTGGTGAAGGGCT TCTACCCCAGCGACATCGCCGTG GAGTGGGAGAGCAACGGCCAGCCCGAGAACAACTACA AGACCACCCCCCCCATGCTGGAC AGCGACGGCAGCTTCTTCCTGTACAGCAAGCTGACCGT GGACAAGAGCAGGTGGCAGCAG GGCAACGTGTTCAGCTGCAGCGTGATGCACGAGGCCC TGCACAACCACTACACCCAGAAG AGCCTGAGCCTGAGCCCCGGC | 27 |

(continued)

| Name | Nucleotide sequences | SEQ ID NO |
|---|---|---|
| Linker peptide | AGCAGCGGCGCCCCCCCCCCAGCGGCGGCGGCAGC | 28 |
| Linker peptide | (GGCGGCGGCGGCAGC)3 | 29 |
| Linker peptide | (GGCGGCGGCGGCAGC)4 | 30 |
| Linker peptide | GGCAGCACCGGCGGCGGCGGCAGCGGCAAGCCCGGCAGCGGCGAGGGCGGCGGCGGCAGC | 31 |
| Linker peptide | GAGAGCGGCAGGAGCGGCGGCGGCGGCAGCGGCGGCGGCGGCAGC | 32 |
| Linker peptide | GAGGGCAAGAGCAGCGGCAGCGGCAGCGAGAGCAAGAGCACC | 33 |
| Linker peptide | GAGGGCAAGAGCAGCGGCAGCGGCAGCGAGAGCAAGAGCACCCAG | 34 |
| Linker peptide | GAGGGCAAGAGCAGCGGCAGCGGCAGCGAGAGCAAGGTGGAC | 35 |
| Linker peptide | GGCAGCACCAGCGGCAGCGGCAAGAGCAGCGAGGGCAAGGGC | 36 |
| Linker peptide | AAGGAGAGCGGCAGCGTGAGCAGCGAGCAGCTGGCCCAGTTCAGGAGCCTGGAC | 37 |
| Linker peptide | GAGAGCGGCAGCGTGAGCAGCGAGGAGCTGGCCTTCAGGAGCCTGGAC | 38 |

### IV. Vector and Cells

In another aspect of the present invention, a vector containing the nucleic acids described in this application is provided.

Any suitable vector can be used for the intended purpose. For example, various vectors can be introduced into expression systems such as mammalian, insect, or bacterial expression systems for the expression and purification of the expressed proteins. Some vectors can be used for virus production. These different vectors are well-known in the art.

In some embodiments, the vector is used in conjunction with an in vitro expression system to produce the fusion protein. The expression and purification of the fusion protein can be carried out using any suitable methods known in the art.

Possible expression vectors include, but are not limited to, plasmids or modified viruses (e.g., replication-defective retroviruses, including lentiviral vectors, adenoviruses, and adeno-associated viruses). In one embodiment, an expression vector that can be used in conjunction with the fusion protein of the present invention is pKN012, which can be obtained through commercial sources (Beijing Kohnoor Science & Technology Co., Ltd.).

The vector can contain appropriate regulatory sequences and components. Suitable regulatory sequences can be selected from various sources, including bacterial, fungal, viral, mammalian, or insect genes. Examples of such regulatory sequences include transcriptional promoters and enhancers, RNA polymerase binding sites, ribosome binding

sites, and translation initiation signals. Additionally, depending on the cells to be transfected/infected/transduced and the vector used, other sequences such as replication origins, additional DNA restriction sites, enhancers, and transcription-inducing sequences can be incorporated into the expression vector. In one embodiment, the regulatory sequences guide or enhance expression in neural tissues and/or cells. In one embodiment, the vector is a viral vector. The recombinant expression vector can also contain a selectable marker gene that facilitates the selection of host cells transformed, infected, or transfected with the vector for expression of the antibodies described herein. The recombinant expression vector may further include other expression cassettes encoding, for example, fusion portions (e.g., "fusion proteins") that can aid in detection, including labels and tags as described herein.

In one embodiment, the vector contains one or more optional components as shown in Figure 1. For example, in one embodiment, the vector containing the nucleic acid encoding the GLP-1 fusion protein is pKN012-GLP1-IgG2.

Various methods can be used for the transduction of cells, including viral vectors, "naked" DNA, DNA in lipids or other nanoparticles, DNA with adjuvants, gene guns, etc. For example, retroviral vectors such as lentiviral vectors can also be used for transducing cells in vivo. Other vector systems that can be used for implementing the present invention include adenoviral and adeno-associated viral vectors.

Another aspect of the present invention provides a recombinant cell that contains the nucleic acid encoding the GLP-1 fusion protein or the vector described in the present application.

Stably expressing recombinant cells can be prepared by transforming, transfecting, or transducing the recombinant cells with a vector containing the nucleic acid, preferably the nucleic acid described in this application.

In one embodiment, the cells of the present invention further express recombinant or endogenous lysyl hydroxylase.

In one embodiment, the cells of the present invention express lysyl hydroxylase at levels or activity higher than that of COS-7 cells. In other words, the lysyl hydroxylase activity level is increased in comparison to COS-7 cells. For example, the cells can be modified to increase the expression of lysyl hydroxylase (e.g., by preparing cells that recombinantly express lysyl hydroxylase), or they can be cells that naturally have an elevated level of lysyl hydroxylase compared to COS-7 cells. For instance, the lysyl hydroxylase level or activity expressed by the cells of the present application can be at least 5%, at least 10%, at least 15%, at least 20%, at least 25%, at least 30%, at least 35%, at least 40%, at least 45%, or at least 50% higher than the lysyl hydroxylase level or activity expressed by COS-7 cells.

In one embodiment, the cells of the present application are eukaryotic cells. In some embodiments, the eukaryotic cells are mammalian cells. In one embodiment, the mammalian cells are human cells. For example, the mammalian cells can be human embryonic kidney cells 293 (HEK293 cells), such as HEK293T cells, HEK293 S cells, or HEK293F cells. In one embodiment, the mammalian cells are Chinese hamster ovary (CHO) cells, such as CHO-K1 cells, CHO-S cells, or CHO-DG44 cells.

In one embodiment, the recombinant cells of the present invention are obtained by suspension adaptation of Chinese hamster ovary cells (CHO), with CHOK1 cells being provided as an example.

## V. Methods for Cell Construction, Production of Fusion Protein, and Detection

Another aspect of the present invention provides a method for constructing recombinant cells, comprising: introducing nucleic acids encoding the GLP-1 fusion protein of the present invention into a vector to construct an expression vector; and transferring the expression vector into cells that recombinantly or naturally express lysine hydroxylase to obtain recombinant cells.

In one embodiment, the expression of lysine hydroxylase in the cells that recombinantly or naturally express it is increased compared to the levels or activity of lysine hydroxylase expressed in other cells (e.g., COS-7 cells).

In one embodiment, the cells are CHO cells, such as CHO-K1 cells, CHO-S cells, or CHO-DG44 cells.

In one embodiment, the vector can be the pKN012 vector.

In one embodiment, the method for constructing the recombinant cells includes the following steps:

a) Insert the nucleotide sequence shown in SEQ ID NO: 26 into the NcoI and HindIII sites of the pKN012 vector, generating the pKN012-GLP1-IgG2/Fc expression vector.
b) Transfect CHO-K1 cells with the pKN012-GLP1-IgG2/Fc expression vector to obtain recombinant cells.

In another embodiment, the method for constructing recombinant cells comprises the following steps:

a) Insert the nucleotide sequence shown in SEQ ID NO: 27 into the NcoI and HindIII sites of the pKN012 vector, generating the pKN012-GLP-1-IgG2/Fc expression vector.
b) Transfect the CHO-K1 cells with the pKN012-GLP-1-IgG2/Fc expression vector to obtain recombinant cells.

Another aspect of the present invention provides a method for producing the GLP-1 fusion protein, comprising

the steps of obtaining the fusion protein from the recombinant cells described in the present specification or the cells prepared using the construction method described in the present specification.

As described in the present specification, the GLP-1 fusion protein can be synthesized. Additionally, as described in the present specification, the fusion protein can also be prepared using recombinant cells, including recombinant Chinese hamster ovary (CHO) cells expressing the GLP-1 fusion protein. Therefore, the present invention further provides a method for preparing the GLP-1 fusion protein, comprising the steps of culturing recombinant cells expressing the GLP-1 fusion protein, wherein the culture includes one or more process steps or materials described in the examples. For example, the method may include one or more process steps as described in Table 2 and/or one or more materials as described in Table 3 or 4.

In some embodiments, the recombinant cells expressing the GLP-1 fusion protein can be prepared using HEK293T, HEK293S, HEK293F, and/or CHO cells (such as CHO-K1 cells). For example, under conditions suitable for in vivo applications, the recombinant cells can be used to produce recombinant peptides and/or fusion proteins.

In one embodiment, the method further includes the following step: detecting the hydroxylation level of the fusion protein on residue K34 relative to native human GLP-1. Without being bound by any theory, it is believed that the prepared fusion protein is considered qualified (e.g., meeting pharmaceutical manufacturing standards) when the hydroxylation level of the GLP-1 fusion protein on residue K34 relative to native human GLP-1 is between 10% and 100% (e.g., equal to or greater than 10%, 15%, 20%, 26%, 27%, 28%, 29%, 30%, 35%, 40%, 45%, 50%, 55%, 60%, 65%, 70%, 75%, 80%, 85%, 90%, or 95%).

Another aspect of the present invention provides a method for detecting the quality of the fusion protein, wherein the fusion protein comprises GLP-1 peptide and the immunoglobulin IgG2-Fc domain. The method includes the following steps: detecting the hydroxylation level of the fusion protein on residue K34 relative to native human GLP-1.

In one embodiment, the GLP-1 peptide is selected from human GLP-1(7-37), human GLP-1(7-36), and DPP-IV-resistant human GLP-1. The GLP-1 peptide includes one or more amino acid substitutions relative to native human GLP-1 selected from the following group: A8G, G22E, and R36G. The IgG2-Fc domain includes one or more amino acid substitutions selected from the following group: C222S, A330S, and P331S. In one embodiment, the GLP-1 peptide includes the amino acid sequence shown in SEQ ID NO: 3, and the IgG2-Fc domain includes the amino acid sequence shown in SEQ ID NO: 6. In another embodiment, the amino acid sequence of the GLP-1 peptide is as shown in SEQ ID NO: 3, and the amino acid sequence of the IgG2-Fc domain is as shown in SEQ ID NO: 6.

In one embodiment, the quality of the fusion protein is confirmed as "qualified" when the hydroxylation level of the fusion protein on K34 relative to native human GLP-1 is between 10% and 100%. For example, it can be equal to or greater than 10%, 15%, 20%, 26%, 27%, 28%, 29%, 30%, 35%, 40%, 45%, 50%, 55%, 60%, 65%, 70%, 75%, 80%, 85%, 90%, or 95%. "Qualified" refers to meeting the drug standards established by the national drug regulatory authorities or similar organizations, such as drug standards, drug registration standards, enterprise drug standards, pharmacopoeias, etc., ensuring that the produced drug meets the standards for drug manufacturing.

## VI. Composition

Another aspect of the present invention provides a composition comprising the GLP-1 fusion protein, dimer, nucleotide, vector, or recombinant cell described in this application.

In one embodiment, the composition may further include a suitable diluent or carrier. In another preferred embodiment, the carrier is a pharmaceutically acceptable carrier.

In one embodiment, the composition is a pharmaceutical composition.

In one embodiment, the composition is a pharmaceutical composition that comprises the GLP-1 fusion protein and a pharmaceutically acceptable carrier.

In one embodiment, the composition comprises the GLP-1 fusion protein, wherein the GLP-1 peptide of the fusion protein has a certain level of hydroxylation at the 34th lysine (K34) residue relative to native human GLP-1. In certain embodiments, the fusion protein exhibits a hydroxylation level of equal to or greater than 10%, 15%, 20%, 26%, 27%, 28%, 29%, 30%, 35%, 40%, 45%, 50%, 55%, 60%, 65%, 70%, 75%, 80%, 85%, 90%, or 95% on the GLP-1 peptide at the K34 residue relative to native human GLP-1.

In one embodiment, the composition comprises the GLP-1 fusion protein, wherein the GLP-1 peptide of the fusion protein is substantially unoxidized at the 31st tryptophan (W31) residue relative to native human GLP-1. In certain embodiments, the oxidation level of the GLP-1 peptide at the W31 residue relative to native human GLP-1 is less than 5% (e.g., less than 4%, less than 3%, less than 2%, less than 1%, less than 0.5%, less than 0.4%, less than 0.3%, less than 0.2%, less than 0.1%) or undetectable.

In one embodiment, the composition comprises the GLP-1 fusion protein, wherein the IgG2-Fc domain of the fusion protein has a certain level of oxidation at the 253rd methionine (M253) residue corresponding to SEQ ID NO: 7. In certain embodiments, the oxidation level of the IgG2-Fc domain at the 253rd methionine (M253) residue corresponding to SEQ ID NO: 7 is less than or equal to 15%, such as less than or equal to 10%, less than or equal to 9%, less than or equal to

8%, less than or equal to 7%, less than or equal to 6%, less than or equal to 5%, less than or equal to 4%, less than or equal to 3%, less than or equal to 2%, less than or equal to 1%, less than or equal to 0.5%, or undetectable.

In one embodiment, the composition further comprises a buffered saline solution.

In one embodiment, the composition further comprises carbohydrates.

In one embodiment, the composition further comprises surfactants. The pharmaceutical composition of the present invention can be prepared, packaged, and/or sold as a unit dose and/or multiple unit doses. The composition can be prepared in various forms.

In one embodiment, the composition comprises GLP-1 fusion protein formulated as a dosage form with a dosage range of about 0.25 mg to about 20 mg. For example, the dosage can be about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 7 mg, about 7.5 mg, about 8 mg, about 8.5 mg, about 9 mg, about 9.5 mg, about 10 mg, about 11 mg, about 12 mg, about 13 mg, about 14 mg, about 15 mg, about 16 mg, about 17 mg, about 18 mg, about 19 mg, or about 20 mg of GLP-1 fusion protein.

The GLP-1 fusion protein, nucleotides, vectors, recombinant cells, or compositions described can be used for the preparation of pharmaceuticals and/or for administration via various routes, such as parenteral (e.g., intravenous, subcutaneous, or intramuscular), enteral (e.g., oral), or other routes of administration.

In one embodiment, the GLP-1 fusion protein or its combination can be administered via the parenteral route or formulated into preparations for parenteral administration.

In one embodiment, the GLP-1 fusion protein or its combination can be administered via subcutaneous route or formulated into preparations for subcutaneous administration.

In one embodiment, the GLP-1 fusion protein or its combination can be administered intravenously or formulated into preparations for intravenous administration.

In one embodiment, the GLP-1 fusion protein or its combination can be administered intramuscularly or formulated into preparations for intramuscular administration.

The dilution solutions suitable for GLP-1 fusion protein and/or cells include, but are not limited to, saline solution, pH-buffered solutions, the dilution solutions described in this document, glycerol solution, or other solutions suitable for freezing peptides and/or cells.

The dilution solutions suitable for nucleic acids and/or vectors include, but are not limited to, saline solution, pH-buffered solutions, the dilution solutions described in this document, water, and so on.

In another preferred embodiment, the dilution solution is sterile.

## VII. Methods and Uses for the Treatment and Prevention of Diseases

As shown in this article, the substitution of GLP-1 peptide with A8G, G22E, and/or R36G, and/or the increase in hydroxylation level at K34, and the decrease in oxidation level at W31, and/or the substitution of C222S, A330S, and P331S in the IgG2/Fc portion of the GLP-1 fusion protein, enhance the yield, activity, and/or half-life of the GLP-1 fusion protein. The modified GLP-1 fusion protein, nucleic acids, vectors, and recombinant cells described in this article are suitable for the preparation of pharmaceuticals and their combinations, and are used for corresponding therapeutic purposes.

Another aspect of the present invention is that the fusion proteins, dimers, nucleic acids, vectors, cells, or compositions disclosed in this article can be used for treating or preventing the occurrence or progression of diseases or conditions in subjects.

Another aspect of the present invention provides a use of the disclosed fusion proteins, dimers, nucleic acids, vectors, cells, or compositions as GLP-1 receptor agonists.

Another aspect of the present invention provides a use of the disclosed fusion proteins, dimers, nucleic acids, vectors, cells, or compositions for the treatment, prevention, or attenuation of diseases or progression of symptoms.

Another aspect of the present invention provides a method of preparing pharmaceutical compositions comprising the fusion proteins, dimers, nucleic acids, vectors, cells, or compositions for the treatment, prevention, or attenuation of diseases or progression of symptoms.

Another aspect of the present invention provides a method for treating, preventing, or attenuating diseases or progression of symptoms by administering a therapeutically effective amount of the fusion proteins, dimers, nucleic acids, vectors, cells, or compositions to a subject in need thereof.

Another aspect of the present invention provides the use of the fusion proteins, dimers, nucleic acids, vectors, cells, or compositions described herein in the preparation of pharmaceuticals for the treatment or prevention of diseases.

Another aspect of the present invention provides the use of the fusion proteins, dimers, nucleic acids, vectors, cells, or compositions described herein for the treatment or prevention of diseases.

Another aspect of the present invention provides a method for treating or preventing diseases, comprising

administering a therapeutically effective amount of the fusion proteins, dimers, nucleic acids, vectors, cells, or compositions described herein to a subject.

In one embodiment, the pharmaceutical composition comprising the fusion protein is administered at a dose ranging from about 0.2 mg to about 20 mg per person per administration. In another embodiment, the amount of fusion protein is about 1 mg to about 10 mg. In yet another embodiment, the amount of fusion protein is about 1 mg to about 5 mg. In a further embodiment, the amount of fusion protein is specifically selected from the range of about 0.25 mg, about 0.3 mg, about 0.35 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 11 mg, about 12 mg, about 13 mg, about 14 mg, about 15 mg, about 16 mg, about 17 mg, about 18 mg, about 19 mg, or about 20 mg.

Different formulations can be used, including but not limited to solutions, suspensions, capsules, and tablets.

In one embodiment, the treatment regimen may involve multiple administrations.

In one embodiment, the administration frequency of the GLP-1 fusion protein or its combination can be once every 3 days, once a week, or once every two weeks.

In one embodiment, the administration regimen of the GLP-1 fusion protein, dimer, nucleotide, vector, cell, or its combination can be weekly administration followed by a break, and then subsequent weekly administrations. In another preferred example, the break period is 2 weeks.

In one embodiment, the GLP-1 fusion protein, dimer, nucleotide, vector, cell, or its combination can be administered once followed by a 2-week break, and then four consecutive weekly administrations. In another preferred embodiment, the treatment further includes an initial dose of 1 mg given one week before the start of the administration regimen.

In one embodiment, the GLP-1 fusion protein, dimer, nucleotide, vector, cell, or its combination can be administered via routes such as gastrointestinal, intravenous, subcutaneous, or intramuscular.

In one embodiment, the diseases include metabolic diseases related to glucose or lipid metabolism, complications of metabolic diseases, or neurological disorders and diseases.

In one embodiment, the disease or condition is a metabolic disease related to glucose or lipid metabolism.

In one embodiment, the metabolic diseases related to glucose or lipid metabolism are selected from the following group: diabetes, non-alcoholic steatohepatitis (NASH), non-alcoholic fatty liver disease (NAFLD), obesity, and metabolic syndrome.

In one embodiment, the metabolic disease related to glucose or lipid metabolism is diabetes, including type 2 diabetes. In another embodiment, the metabolic disease related to glucose or lipid metabolism is obesity. In another embodiment, the metabolic disease related to glucose or lipid metabolism is NAFLD. In another embodiment, the metabolic disease related to glucose or lipid metabolism is non-alcoholic liver fibrosis (NASH).

In one embodiment, the subjects are newly diagnosed or previously diagnosed with diabetes (e.g., type 2 diabetes). Diabetes can be diagnosed through various methods, such as fasting plasma glucose (FPG). According to the American Diabetes Association guidelines, a diagnosis of diabetes is made when the fasting plasma glucose level is greater than or equal to 126 mg/dL.

In one embodiment, the likelihood of the subjects having diabetes (e.g., type 2 diabetes) is increased. For example, the subjects may be predisposed to diabetes due to obesity or genetic susceptibility, such as having a family history of diabetes.

In one embodiment, the subjects are obese individuals. Obesity can be defined by referencing the body mass index (BMI). For example, the World Health Organization (WHO) defines obesity as having a BMI equal to or greater than 30. In another embodiment, the subjects have a BMI of at least approximately 20 kg/m2. In another embodiment, the subjects may have blood glucose levels higher than the average level for their age-matched peers but not sufficient for a diagnosis of diabetes. In another embodiment, the subjects may also be individuals with a family history of diabetes.

In one embodiment, the subjects are newly diagnosed or previously diagnosed with NAFLD or NASH. In another embodiment, the likelihood of the subjects developing NAFLD or NASH is increased. For example, the subjects may have a genetic predisposition to NAFLD or NASH.

In one embodiment, the complications of metabolic diseases include cardiovascular complications (e.g., coronary heart disease, sudden cardiac death, heart failure) caused by metabolic diseases, kidney complications (e.g., acute kidney injury, diabetic nephropathy), or liver complications.

In one embodiment, the diseases or conditions are neurological disorders. In one embodiment, the neurological disorder is a neurodegenerative disease. In one embodiment, the neurodegenerative diseases include Alzheimer's disease (AD), motor neuron diseases, Huntington's disease, and Parkinson's disease (PD).

In one embodiment, the neurodegenerative disease is Alzheimer's disease. In another embodiment, the neurodegenerative disease is Parkinson's disease. In another embodiment, the neurodegenerative disease is motor neuron disease. In another embodiment, the neurodegenerative disease is Huntington's disease.

In one embodiment, the subjects are newly diagnosed or previously diagnosed with Alzheimer's disease. In another embodiment, the likelihood of the subjects having Alzheimer's disease is increased. For example, the subjects may be predisposed to Alzheimer's disease due to their genetic profile, such as having a family history of Alzheimer's disease or mutations related to Tau or APP associated with Alzheimer's disease.

In one embodiment, the subjects are newly diagnosed or previously diagnosed with Parkinson's disease. In another embodiment, the likelihood of the subjects developing Parkinson's disease is increased. For example, the subjects may be predisposed to Parkinson's disease due to their genetic profile, such as having a family history of Parkinson's disease.

The fusion protein, dimer, polynucleotide, vector, cell, or composition of the present invention can be combined with any other known drugs or therapies for the treatment of diseases.

In one embodiment, the disclosed fusion protein, dimer, polynucleotide, vector, cell, or composition can be used in combination with drugs for the treatment of diabetes, including currently marketed drugs such as insulin, metformin, sulfonylureas (including glimepiride, glipizide, glyburide), α-glucosidase inhibitors such as acarbose, as well as other marketed and under development drugs for the treatment of diabetes. In one embodiment, the diabetes drug is metformin or insulin. In another embodiment, the disclosed fusion protein, dimer, polynucleotide, vector, cell, or composition can be used in combination with Alzheimer's disease drugs and/or non-pharmacological interventions such as cognitive therapy. In one embodiment, the disclosed fusion protein, dimer, polynucleotide, vector, cell, or composition is used in combination with γ-aminobutyric acid (GABA) for the treatment of neurodegenerative diseases. In one embodiment, the disclosed fusion protein, dimer, polynucleotide, vector, cell, or composition is used in combination with γ-aminobutyric acid (GABA) for the treatment of Alzheimer's disease.

In some embodiments, the disclosed GLP-1 fusion protein in combination with γ-aminobutyric acid (GABA) can inhibit the TNF-α-induced decrease in SH-SY5Y cell viability.

In some embodiments, the disclosed GLP-1 fusion protein in combination with γ-aminobutyric acid (GABA) can reduce TNF-α-induced apoptosis in neuronal cells SH-SY5Y.

In some embodiments, the disclosed GLP-1 fusion protein in combination with γ-aminobutyric acid (GABA) can provide protection against TNF-α-induced damage in neuronal cells.

In some embodiments, the disclosed GLP-1 fusion protein in combination with γ-aminobutyric acid (GABA) can reduce TNF-α-induced apoptosis in neuronal cells.

In some embodiments, the disclosed GLP-1 fusion protein in combination with γ-aminobutyric acid (GABA) can decrease the expression of inflammatory factors (e.g., TNF-α, IL-6) mRNA induced by Aβ1-42 oligomers in HMC3 microglial cells.

In one embodiment of the application of the present invention, the drug is a GLP-1 receptor agonist.

The above disclosure summarizes the present application. A more complete understanding can be obtained by referring to the specific examples below. These examples are provided for illustrative purposes and are not intended to limit the scope of the present application. Changes in form and substitution of equivalents may be considered when appropriate or convenient. Although specific terms are used herein, they are used descriptively and not for limiting purposes.

The following non-limiting examples are provided to describe the contents of the present invention.

## Examples

Unless otherwise specified, the experimental methods used in the following examples are conventional methods.

Unless otherwise specified, materials and reagents used in the following examples can be obtained from commercial sources.

### Example 1: Fusion Protein Expression and Activity Assay

1.1 Plasmid Construction

A vector encoding the GLP-1 fusion protein was constructed. The fusion protein consists of human GLP-1 (7-37) and human IgG2/Fc (including the hinge region, CH2, and CH3 of human IgG2 heavy chain). The signal peptide sequence of human CD33 (hCD33) was fused with the GLP-1 sequence to guide the secretion of the peptide into the culture medium. The cDNA fragment encoding the fusion protein hCD33-GLP-1-IgG2/Fc (hinge-ch2-ch3) was chemically synthesized (as shown in SEQ ID NO: 27) and inserted into the NcoI and HindIII sites of the pKN012 vector, generating pKN012-GLP-1-IgG2/Fc.

The pKN012-GLP-1-IgG2/Fc stable expression vector was transformed into Escherichia coli DH5α competent cells. Plasmid DNA was extracted from the bacterial strain containing pKN012-GLP-1-IgG2/Fc and digested with PvuI. After electrophoresis, a target band with the correct molecular weight was observed. The linearized plasmid was quantified

after ethanol precipitation and used for stable transfection.

To establish CHO-K1 cells (Lot#58995535/ATCC) stably expressing GLP-1-IgG2/Fc, linearized pKN012-GLP-1-IgG2/Fc (2 μg) was transfected into CHO-K1 cells grown in a 6-well plate (2.5 × 105 cells/well) using electroporation (electroporation-mediated transfection). After 24 hours of transfection, the cells were dispersed and cultured in CD-CHO medium containing MSX (Methionine Sulfoximine, 100 μM/L), selecting cells that had stably integrated the recombinant plasmid into the genome. The culture medium was changed every 3 days until colonies formed. Individual colonies were isolated and expanded into stable cell lines. The GLP-1 fusion protein in the culture supernatant of the cell lines grown in a 24-well plate was tested using a Rat GLP-1 RIA kit (YN-011). Cells capable of secreting the fusion protein were selected for further identification. The amino acid sequence of the prepared GLP-1 fusion protein is shown in SEQ ID NO: 7 (referred to as "YN-011" in this application).

1.2 Assessment of YN-011 Activity by Inducing cAMP Levels

Natural GLP-1 stimulates insulin secretion from beta cells in a glucose-dependent manner. To evaluate whether purified YN-011 possesses the functionality of natural GLP-1, its effect on insulin secretion from clonal insulin-secreting INS-1 cells was measured. INS-1 cells were serum-starved and glucose-starved, then treated with different amounts of purified YN-011 in the presence of 0, 5, or 20 mM glucose as indicated. In the absence of glucose, YN-011 did not stimulate insulin secretion from beta cells. However, in the presence of 5 mM or 20 mM glucose, YN-011 dose-dependently stimulated insulin secretion from beta cells. The data indicate that the GLP-1-IgG2/Fc fusion protein YN-011 possesses biological activity and can stimulate insulin secretion in an glucose-dependent manner in INS-1 cells.

In the absence of glucose, INS-1 cells treated with YN-011 (120 nM) maintained basal levels of cAMP. However, in the presence of 5 mM glucose, the cAMP levels in YN-011-treated cells significantly increased, reaching levels comparable to those induced by exendin-4.

## Example 2: Increased GLP-1 K34 Hydroxylation Level Enhances Protein Yield and Activity

2.1 Determination of Modification Level by LC-MS/MS Peptide Mapping

Mass spectrometry analysis was performed using Lys-C digestion for identification. The YN-011 sample was denatured, reduced, and alkylated, followed by Lys-C enzyme hydrolysis to generate peptides. Online LC-MS/MS analysis was conducted using a Thermo LTQ Velos Orbitrap instrument, and the data were processed and analyzed using Mascot software. It was observed that the peptide segment 21-28: EFIAWLVK exhibited a molecular weight increase of 16 Daltons (Da) due to modification. The primary and secondary mass spectra of peptides without the +16 Da modification and peptides with the +16 Da modification in the YN-011 sample are shown in Figure 2. The mass measurement error of the primary spectra was within 10 ppm. By analyzing the mass-to-charge ratio of the secondary fragment ions, the site of the +16 Da modification was determined to be K28. K28 is also referred to as K34 since the active GLP-1 (e.g., amino acids 7-37 of GLP-1) lacks the first six residues. The peptide 21-28, also known as 27-34 peptide, can be found in natural human GLP-1 as well.

The modification level at the K34 site in YN-011 was determined using UV280 and EIC (Extracted Ion Chromatography) methods, as shown in Table 1 and Figures 3A and 3B. The calculation method for UV280 is as follows:

$$k = \frac{\frac{k\varepsilon_w}{k\varepsilon_w + \varepsilon_Y} A_1}{\frac{k\varepsilon_w}{k\varepsilon_w + \varepsilon_Y} A_1 + A_o} \qquad \begin{matrix} \varepsilon_w = 5500 \\ \varepsilon_w = 1490 \end{matrix}$$

Where:

K: The ratio of modified peptides with +16 Da to the total amount of peptides in the amino acids 21-28 segment (sum of unmodified peptides and +16 Da modified peptides).
A1: Absorbance value at 280 nm for the amino acids 21-28 peptide segment without the +16 Da modification.
A0: Absorbance value at 280 nm for the amino acids 21-28 peptide segment with the +16 Da modification.

**Table 1: Detection of K34 modification levels in YN-011 using UV280 and EIC methods.**

| Sample Name | Sequence Position | Amino Acid Sequence | Translated Modification (PTMs) | UV280 Ratio (%) | EIC* Ratio (%) |
|---|---|---|---|---|---|
| YN-011 Sample 1 | 21-28 | EFIAWLVK | / | / | / |
| | | EFIAWLV**K** | +16Da | 29.6% | 31.8% |
| YN-011 Sample 2 | 21-28 | EFIAWLVK | / | / | / |
| | | EFIAWLV**K** | +16Da | 37.0% | 31.4% |
| YN-011 Sample 3 | 21-28 | EFIAWLVK | / | / | / |
| | | EFIAWLV**K** | +16Da | 30.0% | 31.1% |
| Dulaglutide | 21-28 | EFIAWLVK | / | / | / |
| | | EFIAWLV**K** | +16Da | 8.2% | 4.4% |
| *Note: UV280 and EIC methods were used to detect the K34 modification levels in YN-011.* | | | | | |

2.2 K34 Hydroxylation Level in YN-011

After conducting mass spectrometry analysis and confirming that the +16Da modification occurs at the 34th position of lysine (Lys), considering that +16Da corresponds to the molecular weight of an oxygen atom, it suggests the possibility of an oxidation reaction. In fact, there are two enzymes in cells that catalyze the modification of lysine side chains. One is called lysyl oxidase (Lysyl oxidase, see reference https://en.wikipedia.org/wiki/Lysyl_oxidase), and the other is lysyl hydroxylase (Lysyl hydroxylase, see reference https://en.wikipedia.org/wiki/Lysyl_hydroxylase). Lysyl oxidase can oxidize the amino group of the lysine side chain at the 6th carbon, forming an aldehyde group. However, this modification only results in a 1Da difference compared to the unmodified form, which does not match the observed +16Da modification. On the other hand, lysyl hydroxylase adds a hydroxyl group to the carbon ($\gamma$ position) of the lysine side chain, forming a stable hydroxylated lysine (hydroxylysine). Hydroxylation of lysine increases the molecular weight by 16Da, as it adds a hydroxyl group and reduces a hydrogen atom on the side chain. Therefore, the observed +16Da modification is consistent with lysine hydroxylation. The hydroxylation site at position K34 is shown below:

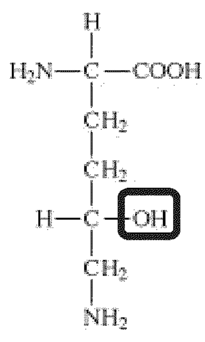

hydroxylysine (Hylys or Lys−OH)

In conclusion, the observed +16Da modification at K34 in YN-011 is highly likely to be due to lysine hydroxylation. Therefore, adding hydroxylation reaction inhibitors during cell culture allows for the verification of the +16Da modification as lysine hydroxylation. It also enables the collection of proteins with different proportions of the +16Da modification to study the impact of the degree of +16Da modification on protein biological activity.

The cell culture process of stable CHOK1 cells capable of secreting the fusion protein YN-011, as described in Implementation Example 1, is shown in Table 2. The relevant materials and cell culture media required for cell culture are listed in Tables 3 and 4, respectively. Mass spectrometry analysis, as described earlier, is used to detect the modification levels of the fusion protein. The inventors found that the fusion protein is indeed hydroxylated at K34, with a hydroxylation level ranging from 15% to 40%.

To investigate the biological effects of K34 hydroxylation, 8 shake flasks (SF1-SF8) were set up for culturing stable CHOK1 cells expressing the fusion protein YN-011. SF1-SF8 were used to evaluate various conditions. Hydroxylation reaction inhibitors were added during the cell culture process to collect proteins with different hydroxylation modification proportions and study the impact of the degree of hydroxylation on protein biological activity. Minoxidil is an

inhibitor of lysyl hydroxylase that suppresses lysine hydroxylation, while Zn2+ ions competitively inhibit hydroxylation enzyme activity by competing with Fe2+ ions during the reaction. SF1 was used as a control without the addition of any inhibitors. Minoxidil used in this experiment was dissolved in a 0.1N HCl solution. HCl was added to SF2 as the HCl control. Different concentrations of minoxidil were added to SF3-SF5 at concentrations of 0.1 mM, 0.5 mM, and 1.0 mM, respectively. SF6 and SF7 were experimental groups where Zn2+ was added. Zn2+ reagent used was ZnSO4, and the concentrations of ZnSO4 used were 200 μM and 400 μM, respectively. SF8 included both minoxidil and Zn2+ (provided by ZnSO4) as inhibitors, with total concentrations of minoxidil and Zn2+ being 0.5 mM and 200 μM, respectively.

**Table 2. Cell Culture Process**

| Basal Medium | Cell Density | Additive | Time | Percentage | Temperature |
|---|---|---|---|---|---|
| Dynamis (0.5% ACA) | 0.5($\times 10^6$ cell/ml) | Feed C(15g/L FM012) , FAA02 | Day 3, 6, 8, 10 | 7.7%, 11%, 11%, 8.8% | 36.5°C→30°C (Day 4) |

**Table 3. Materials Used in the Cell Culture Process**

| Name | Abbreviations | Company | Item number |
|---|---|---|---|
| DynamisTM AGT medium | Dynamis | Gibco | A26175 |
| CD Efficient Feed C AGT | Feed C | Gibco | A1327501 |
| Sheff-CHO PLUS PF ACF | FM012 | Sheffield | 5X00483 |
| L-Cysteine | Cys | Applichem | A3671 |
| L-Tyrosine | Tyr | Applichem | A3401 |
| L-Glutamine | Gln | J.T.Baker | 2078-06 |
| Anhydrous glucose | Glucose | J.T.Baker | 1919-09 |
| HCl, 6.0N solution | HCl | J.T.Baker | 0327-02 |
| NaOH, 10N solution | NaOH | J.T.Baker | 5000-02 |
| Minoxidil | Minoxidil | Sigma | M4145 |
| $ZnSO_4$ | $Zn^{2+}$ | Sigma | Z0251 |
| Anticoagulant | ACA | Gibco | 0010057 |

**Table 4 cell culture medium and its composition**

| Cell Culture Medium | Composition |
|---|---|
| Dynamis (0.5% ACA) | IL Dynamis (adding 5ml ACA) |
| Feed C(including 15g/L FM012) | 1L Feed C dissolving 15g FM012 |
| FAA02 | IL FAA02(including 7.95g L-Cysteine and 4.75g L-Tyrosine) |

2.3 Increasing the hydroxylation level of K34 in GLP-1 can enhance protein yield and activity.

The results of protein yield and activity are shown in Table 5. The hydroxylation levels of the blank control SF1 and SF2, which only received HCl addition, were in the range of 15.7% to 16.0%. From SF3 to SF5, the hydroxylation proportion gradually decreased with increasing concentrations of minoxidil (one of the hydroxylation inhibitors). When the dosage of minoxidil reached 0.5 mM, the hydroxylation proportion was 8.7%. When the minoxidil concentration was increased to 1 mM, the proportion was 9.4%. Cell growth and protein expression were significantly inhibited, with a decrease of approximately 17% in peak cell density and a decrease in protein yield of 57.0% to 67.9%. Additionally, when 400 μM of Zn2+ (another hydroxylation inhibitor) was added to the cell culture, the hydroxylation level also decreased to 10.7%. In the experiment where a combination of inhibitors was added (minoxidil at 0.5 mM and Zn2+ at 200 μM) in SF8, the hydroxylation level decreased to 9.6%.

In the presence of hydroxylation inhibitors such as minoxidil, the hydroxylation proportion gradually decreases with increasing concentration. Cell growth and protein expression are significantly inhibited, with a decrease of approximately 17% in peak cell density and a gradual decrease in yield ranging from 57.0% to 67.9%. Similarly, in the presence of the hydroxylation inhibitor Zn2+, the hydroxylation level also decreases with increasing Zn2+ concentration, resulting in a decrease in protein yield of 22.9% to 34.2%. When the hydroxylation level reaches 15.7% to 16.0%, the yield increases, doubling compared to the low hydroxylation level. Therefore, higher hydroxylation levels result in higher yields.

SUPA-1 is a GLP-1-IgG2/Fc fusion protein disclosed in the US patent US8658174. No linking peptide is used between the GLP-1 peptide and IgG2/Fc. Apart from the A8G substitution in the GLP-1 peptide, no other site mutations are present on the GLP-1 peptide and IgG2/Fc. SUPA-1 was not detected to be hydroxylated or undergo any other modifications, and under the same conditions, the protein yield is 22 mg/L. In contrast, when the hydroxylation level of K34 in YN-011 is 8.7% to 9.6%, the yield can reach 0.77 g/L to 1.03 g/L, which is over 100 times higher than that of SUPA-1. When the hydroxylation degree of K34 exceeds 15%, the yield rate of YN-011 can be over 500 times that of SUPA-1.

Furthermore, the blank control (SF1) and the two samples with the lowest hydroxylation levels (SF4 and SF5) were tested for the biological activity of YN-011 using the method described in Example 1. The results showed that SF1 had an activity of 88%, while SF4 and SF5 had activities of 83% and 68%, respectively. Thus, higher hydroxylation levels tend to increase the biological activity.

In summary, it is confirmed that K34 in the fusion protein is indeed hydroxylated, and this modification greatly enhances the yield of YN-011 and shows an increasing trend in biological activity.

Furthermore, by scaling up the cell culture to a 15 L and three 200 L reactors, YN-011 obtained from these batches exhibited 20% to 30% hydroxylation at position 34, with a yield of approximately 2.7 g/L.

**Table 5. Summary of Yield and Protein Activity Results**

| No. | Test conditions | Hydroxylation level by UV280 detection | Initial cell inoculation density (×106 cells/mL) | Cumulative live cell inoculation density (×106 cells/day/mL) | Time (Days) | Protein Yield (g/L) | Single cell yield (pg/cell/day) |
|---|---|---|---|---|---|---|---|
| SF1 | Control group 1 | 15.7 | 0.5 | 221.9 | 14 | 2.4 | 10.81 |
| SF2 | Control group 2, 0.1N HCl | 16.3 | 0.5 | 213.3 | 14 | 2.28 | 10.69 |
| SF3 | Minoxidil, 0.1 mM | 12.7 | 0.5 | 215.4 | 14 | 2.1 | 9.75 |
| SF4 | Minoxidil, 0.5 mM | 8.7 | 0.5 | 179.8 | 14 | 1.03 | 5.73 |
| SF5 | Minoxidil, 1.0 mM | 9.4 | 0.5 | 170.5 | 14 | 0.77 | 4.52 |
| SF6 | Zn2+, 200uM | 14.6 | 0.5 | 203.6 | 14 | 1.85 | 9.09 |
| SF7 | Zn2+, 400uM | 10.7 | 0.5 | 206.5 | 14 | 1.58 | 7.65 |
| SF8 | Minoxidil, 0.5 mM; $Zn^{2+}$, 200uM | 9.6 | 0.5 | 174.9 | 14 | 0.92 | 5.26 |

**Example 3 Determination of Oxidation Levels by LC-MS/MS**

Following the protein oxidation measurement method described in Example 2 or by referring to Bettinger et al. [19], the oxidation levels were determined. It was observed that the GLP-1 fusion protein YN-011 of the present invention was not oxidized at position W31, whereas the oxidation level of Degludec at the same position exceeded 5%. The oxidation occurred at the location indicated by the rectangular box in the structure of W31.

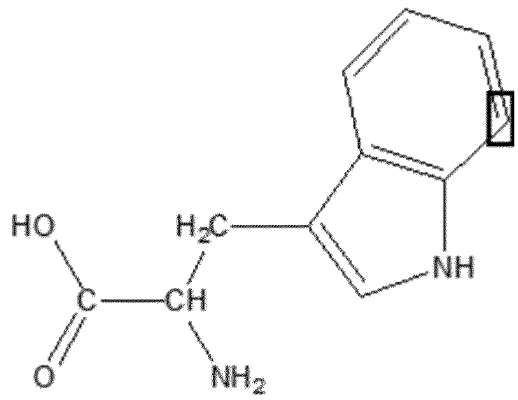

Additionally, the GLP-1 fusion protein YN-011 of the present invention exhibited an oxidation level of approximately 2%-4% at position M253, whereas Degludec showed an oxidation level exceeding 5% at the same position.

**Example 4: Prevention and Therapeutic Effects of YN-011 in db/db Mice (Type 2 Diabetes Model)**

The impact of multiple subcutaneous injections of YN-011 on reducing blood glucose levels was evaluated in db/db mice (Jackson Laboratories, 000642). The mice were kept under normal lighting conditions (12 hours light/12 hours dark) and at room temperature, with free access to food (normal rodent chow) and water. Db/db mice lack leptin receptors and spontaneously develop obesity, hyperglycemia, and pancreatic β-cell atrophy by 4 weeks of age.

Sixty affected db/db mice (33-45g) were divided into six groups of 10 mice each (5 males and 5 females) and administered 0 (PBS buffer control), 0.15, 0.3, 0.6, or 1.2 mg/kg of YN-011, or 0.3 mg/kg of Degludec via subcutaneous injection. Another group of wild-type mice (17-22g) with the same genetic background served as the normal control. The administration frequency was once every 3 days (Q3D), with a total of 9 doses administered over a period of 26 days. As shown in Figure 6, throughout the entire experiment, the random plasma glucose (RPG) levels of the db/db mice in the model control group remained consistently high and significantly higher than those of the normal control mice ($P < 0.001$). Following the first administration, YN-011 significantly reduced the RPG concentration in db/db mice, with the therapeutic effect observed as early as 3 hours after administration at a dose as low as 0.15 mg/kg. The duration of the hypoglycemic effect ranged from 34 to 72 hours within the dose range of 0.15-1.2 mg/kg. YN-011 also significantly promoted insulin secretion, with an increase in serum insulin levels observed 3 hours after administration.

After the 9th administration of YN-011, the RPG levels in YN-011-treated mice were significantly lower than those in the control group at all dose groups and measurement time points, except for the 0.15 mg/kg group after the 4th administration (day 10), where the difference was not significant ($P = 0.07$). To gain a more intuitive understanding of the blood glucose reduction in each group of mice, the average rate of decline in random blood glucose levels was calculated at each measurement time point throughout the experiment. The results showed that the average rates of decline in random blood glucose levels at 72 hours after the 7th administration were 31.8%, 46.2%, and 50.8% in the 0.15, 0.3, and 0.6 mg/kg YN-011 groups, respectively, reaching as high as 54.3% in the 1.2 mg/kg YN-011 treatment group. The hypoglycemic effect of YN-011 was sustained for 72 hours after repeated administration.

Long-term administration of YN-011 also significantly reduced fasting blood glucose levels in db/db mice. Throughout the entire experiment, the fasting blood glucose levels of db/db mice remained consistently high and significantly higher than those of the normal control mice ($P < 0.001$). After subcutaneous injections of YN-011 at different doses every 3 days, the fasting blood glucose levels of mice in all YN-011 groups showed a significant decrease compared to the model control group ($P < 0.001$). The rates of fasting blood glucose reduction at 54 hours (day 21) after the 7th administration were 46.8%, 55.6%, 59.5%, and 64.7% in the 0.15, 0.3, 0.6, and 1.2 mg/kg YN-011 groups, respectively. This suggests that the hypoglycemic effect of YN-011 at doses of 0.15, 0.3, 0.6, and 1.2 mg/kg, administered every 3 days, can be maintained for at least 54 hours (3 days after administration). The positive control group treated with 0.3 mg/kg Degludec also showed a significant decrease in fasting blood glucose levels at all measurement time points.

To provide a more intuitive understanding of the blood glucose reduction in each group of mice, the average rate of decline in fasting blood glucose levels at each measurement time point throughout the experiment was calculated. The average rates of fasting blood glucose reduction in the 0.15, 0.3, 0.6, and 1.2 mg/kg YN-011 groups were 55.0%, 61.8%, 63.7%, and 66.1%, respectively. The average rate of fasting blood glucose reduction in the positive control group treated with 0.3 mg/kg Degludec was 63.6%.

Therefore, multiple subcutaneous injections of YN-011 every 3 days significantly reduced fasting blood glucose levels in type 2 diabetic db/db mice. The effect was evident at a dose of 0.15 mg/kg, and the hypoglycemic effect of YN-011 at doses of 0.15, 0.3, 0.6, and 1.2 mg/kg could be maintained for at least 54 hours (3 days after administration).

Subcutaneous injections of YN-011 at doses of 0.15-0.6 mg/kg in db/db mice showed a decreasing trend in serum fructosamine levels, while the 1.2 mg/kg YN-011 treatment group exhibited a significant reduction in serum fructosamine levels.

In the model control group, the random and fasting body weights of db/db mice increased continuously throughout the experiment, while the YN-011 treatment groups at doses of 0.3, 0.6, and 1.2 mg/kg showed significant decreases in random and fasting body weights ($P < 0.05$, $P < 0.01$, $P < 0.001$). In contrast, the SUPA-1 fusion protein disclosed in U.S. Patent US8658174 did not have a significant effect on the body weight of db/db mice. This indicates that the GLP-1 fusion protein of the present application performs better.

Compared to the solvent control group, the YN-011 treatment groups showed a significant decrease in epididymal fat content and its ratio to body weight. The scapular fat content was significantly reduced in the 0.15, 0.3, and 0.6 mg/kg YN-011 groups. The subcutaneous fat content, inguinal fat content, and their ratios to body weight were significantly reduced in the 0.3, 0.6, and 1.2 mg/kg YN-011 groups. The perirenal fat content was significantly reduced in the 1.2 mg/kg YN-011 group.

After the last administration (9th dose, day 26), YN-011 dose-dependently increased fasting serum insulin levels in db/db mice, accompanied by a significant increase in pancreatic β-cell mass.

Compared to the solvent control group, multiple subcutaneous injections of YN-011 significantly decreased serum triglyceride levels, and 0.6 mg/kg YN-011 significantly reduced serum free fatty acid levels in db/db mice.

In summary, multiple subcutaneous injections of YN-011 demonstrated significant therapeutic effects in db/db mice. It not only improved glucose metabolism but also had significant therapeutic effects on abnormal lipid metabolism.

**Example 5: Phase IIa Clinical Trial of YN-011**

5.1 Study Design

This phase IIa clinical trial is a double-blind, placebo-controlled study conducted in subjects with type 2 diabetes mellitus (T2DM) to evaluate the efficacy and safety of subcutaneous administration of YN-011 at doses of 1 mg, 2 mg, 3 mg, and 4 mg. The design of this study is shown in Figure 5.

Key inclusion criteria for the phase IIa study:

- Patients with T2DM (WHO 1999) who have not taken metformin for at least 1 week and have not taken any other oral antidiabetic drugs for at least 2 weeks.
- HbA1c levels at screening: 7.0% ≤ HbA1c ≤ 10.0%.
- Age between 18 and 65 years at screening.
- BMI ≥ 20 kg/m2 and ≤ 40 kg/m2.

Key exclusion criteria for the phase IIa study:

- Type 1 diabetes.
- Fasting C-peptide < 0.81 ng/mL.
- Laboratory parameters meeting one of the following criteria: alanine aminotransferase (ALT) levels ≥ 2.5 × ULN, and/or aspartate aminotransferase (AST) levels ≥ 2.5 × ULN, fasting triglycerides > 5.6 mmol/L; estimated glomerular filtration rate (eGFR) calculated by the CKD-EPI (EPI-(Scr)) equation < 45 mL/min/1.73 $m^2$,
- Known susceptibility to familial (first-degree relatives) or personal history of type 2 multiple endocrine neoplasia or medullary thyroid carcinoma.
- Uncontrolled hypertension.
- Known history of pancreatitis, pancreatic cancer, or serum amylase > 1.2 × ULN, or high-risk factors for pancreatitis at screening.
- Patients with uncontrolled hypothyroidism.
- Suspicion of active infection.
- Positive hepatitis B surface antigen (HBsAg), hepatitis C antibody (HCV-Ab), human immunodeficiency virus antibody (HIV-Ab), or Treponema pallidum antibody (TPAb).
- Treatment with GLP-1 receptor agonists, DPP-4 inhibitors, or insulin in the 3 months prior to randomization.
- History of grade 3-4 allergy to any protein drug as per CTCAE.

- Blood donation or blood loss exceeding 450 mL in the 3 months prior to screening.
- Any significant endocrine, immune, coagulation, urinary reproductive tract abnormality, or hematological disease.
- Clinically significant gastric emptying disorder (e.g., gastric outlet obstruction), severe chronic gastrointestinal disease (e.g., active ulcer within 6 months), long-term use of drugs directly affecting gastrointestinal motility, or previous gastrointestinal surgery.
- Any other condition that the investigator or primary physician deems unsuitable for participation in the study.

Subjects were randomized in a 4:1 ratio to receive YN-011 or placebo. YN-011 was administered at doses of 1 mg, 2 mg, and 3 mg, with a dosing regimen of a single dose followed by a 2-week rest period, then once-weekly dosing for 4 consecutive weeks. Subjects in the 4 mg group had the same dosing regimen, except they received an initial dose of 1 mg one week before dosing. All subjects received a total of 5 randomized doses.

The primary endpoint was the safety and tolerability of YN-011 in subjects with T2DM. Secondary endpoints included changes in fasting blood glucose from baseline on a weekly basis, changes in HbA1c from baseline at week 4 and week 7, changes in glycated albumin from baseline at week 4 and week 7, changes in glucose tolerance, and changes in pancreatic beta-cell function assessed by oral glucose tolerance test. Blood samples were collected from all subjects for pharmacokinetic testing. Safety assessment included adverse events, laboratory tests, vital signs, 12-lead electrocardiograms, physical examinations, and evaluation of anti-drug antibodies (ADAs).

5.2 YN-011 Single or Multiple Dose Pharmacokinetics

Within the dose range of 1.0 mg to 4.0 mg, YN-011 exhibited an increasing trend. After a single dose, the half-life (T1/2) of YN-011 was approximately 207 hours (8.6 days), with a median Tmax of 60-84 hours (Figure 4). Following the first dose of YN-011 after randomization, dosing occurred once every week, with the fourth dose being administered consecutively. The plasma concentration of YN-011 reached a steady state after the fourth dose (Figure 4). The fusion protein of this application showed a significantly extended half-life compared to SUPA-1 after amino acid substitution. Additionally, compared to the marketed drugs Dulaglutide with an elimination half-life of 4.7-5.5 days [22] and Semaglutide with an elimination half-life of 5.7-6.7 days [24], YN-011 also exhibited a significantly longer half-life than Dulaglutide and Semaglutide.

5.3 Efficacy of YN-011 in Type 2 Diabetes Mellitus

YN-011 was administered subcutaneously at formal doses of 1 mg, 2 mg, 3 mg, and 4 mg (as per the dosing scheme shown in Figure 5) for the treatment of type 2 diabetes mellitus.

A total of 40 subjects received at least one dose of YN-011 or placebo and were included in the safety analysis. The mean age of the subjects was 51.7 ± 10.32 years, with a mean BMI of 25.80 ± 2.875 kg/m2 and a mean weight of 71.91 ± 12.360 kg. 40% of the subjects were female. Among the 40 subjects, 38 (95%) had comorbidities, including 32 (80%) with confirmed fatty liver by ultrasound, 25 (62.5%) with hyperlipidemia, 19 (47.5%) with hypertension, 10 (22.5%) with atherosclerosis, and 10 (22.5%) with lung lesions. Throughout the entire trial, none of the 40 subjects received concomitant medication. The baseline characteristics of the subjects are summarized in the following table (Table 6).

**Table 6. Baseline Characteristics of T2DM Subjects**

| | 1 mg n = 8 | 2 mg n = 8 | 3 mg n = 8 | 4 mg n = 8 | Placebo n = 8 |
|---|---|---|---|---|---|
| Overweight N (%) | 2 (25%) | 7 (87.5%) | 6 (75%) | 4 (50%) | 4 (50%) |
| Obesity N (%) | 0 (0%) | 1 (12.5%) | 0 (0%) | 0 (0%) | 1 (12.5%) |
| Presence of fatty liver on ultrasound, N (%) | 4 (50.0%) | 6 (75.0%) | 6 (75.0) | 8 (100%) | 8 (100%) |
| AST/ALT/Total Bilirubin > ULN Baseline N (%) | 1 (12.5%) | 0 (0%) | 0 (0%) | 0 (0%) | 1 (12.5%) |
| Dyslipidemia N (%) | 3 (37.5%) | 5 (62.5%) | 7 (87.5%) | 5 (62.5%) | 5 (62.5%) |
| High blood pressure N (%) | 2 (25.0%) | 3 (37.5%) | 5 (62.5%) | 5 (62.5%) | 4 (50.0%) |
| Atherosclerosis N (%) | 0 (0%) | 3 (37.5%) | 4 (50.0%) | 2 (25.0%) | 0 (0%) |
| Lung swelling N (%) | 2 (25.0%) | 3 (37.5%) | 1 (12.5%) | 3 (37.5%) | 0 (0%) |

YN-011's effects on fasting blood glucose at different doses are shown in Figure 6. Multiple subcutaneous administrations of YN-011 at 3 mg or 4 mg resulted in a sustained and significantly improved clinical and statistically

significant improvement in fasting blood glucose compared to placebo.

The impact of different doses of YN-011 on HbA1c is illustrated in Figure 7. Multiple subcutaneous administrations of YN-011 at doses of 1 mg, 3 mg, or 4 mg demonstrated a persistent improvement in HbA1c levels, which was clinically and statistically significant compared to placebo. No significant side effects or safety risks were observed.

**Example 6: Preventive and Therapeutic Effects of YN-011 in Obesity**

6.1 Effects of YN-011 in High-Fat Diet-Induced Obese Mice

The effects of multiple administrations of YN-011 on glucose tolerance, insulin sensitivity, metabolism, and weight reduction were evaluated in high-fat diet (HFD)-induced obese mice. Male C57BL/6 mice, 5 months old (obtained from commercial sources such as Shanghai Slake Experimental Animals Co., Ltd., under standard housing conditions), were fed with an HFD (60% of total calories from fat, 20% from carbohydrates) for 6 months to establish the diet-induced obesity (DIO) mouse model. The DIO mice (weighing >50 g) were then divided into two groups (5 animals per group), with the experimental group receiving subcutaneous injections of 0.3 mg/kg of YN-011 twice a week (BIW) and the control group receiving phosphate-buffered saline (PBS) injections. The treatment was administered for 4 weeks.

The experimental results showed that BIW injections of YN-011 for 4 weeks significantly reduced body weight in DIO mice. Compared to the PBS control group, a significant decrease in visceral fat, particularly epididymal fat content, was observed in the YN-011-treated mice. YN-011 had no impact on the weight of other weight-related tissues, including brown adipose tissue (BAT), white adipose tissue (WAT) in the inguinal region, pancreas, or calf muscle.

In comparison to the control group, BIW injections of YN-011 for 4 weeks significantly reduced ectopic lipid accumulation and hepatic triglycerides, as well as serum ALT levels (YN-011 vs. Ctrl = 34.2 $\pm$ 7.7 vs. 153.4 $\pm$ 18.7, $P < 0.01$) and AST levels (YN-011 vs. Ctrl = 72.20 $\pm$ 19.29 vs. 145.6 $\pm$ 16.8, $P < 0.05$). After 4 weeks of BIW treatment, YN-011 also significantly improved the lipid profile, with a 30% reduction in total cholesterol (TC) ($P < 0.001$), a 68% reduction in triglycerides (TG) ($P < 0.001$), and a 57% reduction in non-esterified fatty acids (NEFA) ($P < 0.001$).

In comparison to the control group, DIO mice receiving multiple administrations of YN-011 showed a significant decrease in food consumption. YN-011 treatment showed an increasing trend in metabolic rate (VO2 and VCO2) and energy expenditure (EE), but without statistical significance. When normalized to body weight, the mice treated with YN-011 exhibited significantly increased VO2, VCO2, and EE during the night. YN-011 had no effect on UcP1 expression in BAT and epididymal WAT but significantly upregulated Ucp1 expression in inguinal WAT, indicating that YN-011 did not enhance BAT thermogenesis but promoted browning of inguinal white adipose tissue. Therefore, YN-011-treated DIO mice exhibited higher core body temperature at room temperature and maintained higher rectal temperature when exposed to a cold environment, indicating increased thermogenesis compared to the control group. These results suggest that YN-011 enhances the adaptation of obese mice to cold environments by generating more calories.

Through glucose fluctuation, intraperitoneal glucose tolerance testing, and insulin tolerance experiments, YN-011 significantly reduced blood glucose ($P < 0.01$) and improved insulin sensitivity ($P < 0.01$) in DIO mice after 4 weeks of treatment.

In conclusion, YN-011 effectively reduced body weight in obese mice and improved obesity-related metabolic disorders, including hyperglycemia, hyperlipidemia, and hepatic steatosis. The beneficial effects of YN-011 on metabolism were associated with the inhibition of food consumption and browning and remodeling of WAT.

6.2 Therapeutic effect of YN-011 on obese rhesus monkeys

The obese rhesus monkeys used in this study were sourced from Sichuan Primate Biotechnology Co., Ltd. Fifteen male obese rhesus monkeys, aged 8-21 years (equivalent to 30-60 years in humans), with a body weight of 9.25-15.70 kg, fasting plasma glucose (FPG) levels between 5.50-8.58 mmol/L, and HbA1c levels between 4.5-5.0%, were selected. These monkeys were within the first year of disease onset and had not received any medication. Their liver and kidney functions were normal. The animals were stratified based on FPG levels and randomly assigned to different groups. The selected animals underwent an intravenous glucose tolerance test (IVGTT) to measure blood glucose and insulin levels before the administration of the test compounds. The experimental groups included a placebo group and two YN-011 treatment groups: YN-011 50 $\mu$g/kg and YN-011 25 $\mu$g/kg, with 5 animals in each group. The treatments were administered via subcutaneous injections (SC) once a week for 4 consecutive weeks, specifically on Day 0 (D0), Day 7 (D7), Day 14 (D14), and Day 21 (D21).

The effects of YN-011 on body weight (BW) in obese rhesus monkeys are shown in Table 7 and Figure 8. In the placebo group, there were no significant changes in body weight observed throughout the entire study period, indicating the stability of the model. In the YN-011 25 $\mu$g/kg group, compared to the baseline, body weight consistently decreased (2.60%-6.71%, $P < 0.05$ or $P < 0.01$) from D7 to D28 with a 6.17% decrease at D28. Compared to the placebo group, the BW in the YN-011 25 $\mu$g/kg group significantly decreased on D14 and D21 ($P < 0.05$). In the YN-011 50 $\mu$g/kg group,

compared to the baseline, body weight consistently decreased (4.06%-7.32%, $P < 0.05$) from D7 to D28 with a significant 7.32% decrease at D28 ($P < 0.05$). Compared to the placebo group, the BW in the YN-011 50 μg/kg group significantly or extremely significantly decreased ($P < 0.05$ or $P < 0.01$) from D7 to D28.

**Table 7. Effects of repeated subcutaneous injections of YN-011 on body weight (BW) in obese rhesus monkeys.**

| Group and Time points | BW (kg) | | | | | | |
|---|---|---|---|---|---|---|---|
| | D1 SC1[a] Baseline value | D7 SC2 | D14 SC3 | D21 SC4 | D28 | D35 | D42 |
| Placebo Group N = 5 | 10.75 ± 1.08 | 10.66 ± 1.10 | 10.64 ± 1.11 | 10.64 ± 1.12 | 10.47 ± 1.13 | 10.52 ± 1.11 | 10.44 ± 1.05 |
| YN-011 Group 25 μg/kg N = 5 | 12.72 ± 1.82 | 12.39 ± 1.76# | 12.16 ± 1.61## | 12.06 ± 1.59## | 11.84 ± 1.46## | 12.05 ± 1.56## | 12.03 ± 1.51# |
| YN-011 Group 50 μg/kg N = 5 | 10.29 ± 1.01 | 9.85 ± 0.80# | 9.64 ± 0.77# | 9.63 ± 0.69# | 9.52 ± 0.76# | 9.79 ± 0.83# | 9.81 ± 0.91# |
| *Note: "a" represents the measurement before administration; # compared to baseline, P < 0.05; ## compared to baseline, P < 0.01. SC refers to subcutaneous injection.* | | | | | | | |

**Example 7: Preventive and Therapeutic Effects of YN-011 in Non-Alcoholic Steatohepatitis (NASH) Model of Rhesus Monkeys**

7.1 Experimental Methods

*7.1.1 Liver Biopsy under GE Ultrasound Guidance*

Number of animals: 15

Amount of specimens collected: ≤ 2 needles, each needle with a specimen length ≤ 1.5 cm, and the total cumulative specimen length ≤ 3 cm.

Approximately 2 cm of the collected tissue was immediately placed in 4% buffered formalin at room temperature and fixed for at least 24 hours. After appropriate modifications, dehydration, embedding, sectioning, HE staining, and Masson staining were performed.

Equipment: GE Vivid S5 ultrasound system was used for ultrasound, LEICA RM2135 microtome was used for sectioning, OLYMPUS BX43 microscope was used for slide examination, and OLYMPUS DP22-CU camera was used for photomicroscopy.

Tissue sections were stained with H&E and Masson stains. Pathological slides were evaluated by a pathologist according to the diagnostic and treatment guidelines for non-alcoholic fatty liver disease (NAFLD) jointly drafted by the American Association for the Study of Liver Diseases (AASLD), the American College of Gastroenterology (ACG), and the American Gastroenterological Association (AGA). The diagnostic criteria can be found in Table 2 and Table 3.

*7.1.2 Tissue Processing, Embedding, and Sectioning*

After adequate fixation of the liver biopsy specimens, they were dehydrated, infiltrated with paraffin, embedded, and sectioned (5 μm) directly.

*7.1.3 H&E Staining*

Routine deparaffinization of the sections was performed. The sections were sequentially immersed in 100% ethanol I, 100% ethanol II, 95%, 85%, and 75% ethanol for 3 minutes each. They were then rinsed with tap water for 3 minutes, stained with hematoxylin-eosin (HE) staining solution for 12 minutes, rinsed with tap water for 5 minutes, and stained with eosin Y solution (aqueous) for 3 minutes. Dehydration was carried out rapidly with 95% ethanol, followed by two rounds of dehydration with absolute ethanol for 2-5 minutes each. The sections were then cleared with xylene twice for 10 minutes each. Neutral mounting medium was applied, and microscopic examination was performed.

*7.1.4 Masson Staining*

Routine deparaffinization of the sections was performed. The sections were stained with Weigert's iron hematoxylin for 5-7 minutes, and simultaneously, weak acid working solution was prepared by mixing distilled water and weak acid solution in a ratio of 2:1. The sections were rinsed with weak acid working solution for 1 minute. After rinsing with phosphomolybdic acid solution for 1-2 minutes, the sections were directly placed in aniline blue staining solution for 1-2 minutes, followed by rinsing with the prepared weak acid working solution for 1 minute. Dehydration was carried out rapidly with 95% ethanol, followed by two rounds of dehydration with absolute ethanol for 2-5 minutes each. The sections were then cleared with xylene twice for 10 minutes each. Neutral mounting medium was applied, and microscopic examination was performed.

*7.1.5 Histopathological Examination of Tissues*

The sections were observed under a microscope (with a digital imaging system), and pathological diagnosis and photography were performed. Based on the histopathological diagnosis, the HE and Masson results were scored according to the scoring criteria for non-alcoholic fatty liver disease activity score (NAS, Table 8) and liver fibrosis staging (Table 9).

**Table 8 NAS Scoring Criteria**

| Scoring / Feature | 0 | 1 | 2 | 3 |
|---|---|---|---|---|
| ~~Lipoatrophy~~Steatosis | < 5% | 5%~33% | 34%~66% | > 66% |
| Interlobular inflammation (200x field of view) | No foci of inflammation were seen | Few to 2 foci of inflammation | 2-4 foci of inflammation | More than 4 foci of inflammation |
| Balloon-like degeneration | No balloon-like degeneration was seen | Few cells balloon-like degeneration | Massive balloon-like degeneration of cells | / |

Note: NAS score = steatosis score + interlobular inflammation score + balloon-like degeneration score

**Table 9 Liver fibrosis grading scale**

| Level | Grade Description |
|---|---|
| 0 | No fibrosis observed |
| 1a | Mild perisinus fibrosis in zone 3 |
| 1b | Moderate sinus fibrosis in zone 3 |
| 1c | Periportal fibrosis |
| 2 | Perisinus and periportal fibrosis |
| 3 | Bridging fibrosis |
| 4 | Cirrhosis |

*7.1.6 GE 3.0T MRI Quantitative Analysis of Hepatic Steatosis*

The GE 3.0T MRI scanner (750W3T MRI, GE Healthcare) was used for liver imaging in this study. The IDEAL-IQ sequence was employed, which combines the IDEAL (Iterative Decomposition of water and fat with Echo Asymmetry and

Least-squares estimation) technique with fast three-dimensional multi-echo imaging. This technique includes multi-echo water-fat separation, region-growing algorithm, as well as various imaging methods for tissue fat quantification and R2* relaxation rate. Six contrast images, including in-phase, opposed-phase, water-only, fat-only, fat fraction maps, and R2* maps, were acquired through a single breath-hold scan by collecting signals from six different echo time (TE) values. The images were reconstructed using computer algorithms to obtain the aforementioned contrast images. The IDEAL-IQ hybrid water-fat separation algorithm consisted of two steps. The first step involved complex domain reconstruction to obtain water and fat images, as well as the T2* map. The second step generated an additional set of estimated water and fat images. These two sets of images were then combined using a hybrid algorithm to generate the final water and fat images.

Prior to conducting the experiment, a Phantom test was performed. Five standard fat solutions with known fat content were prepared by modifying the body phantom preparation method used by Clare P. et al.[11]. Pure water was used as 0% fat. Sodium dodecyl sulfate (60 mmol) was dissolved in 1 L of deionized water, heated to 50°C, and then 40 g of gelatin was added and mixed thoroughly. Soybean oil (96, 108, 114, 117, and 120 ml, obtained from China National Pharmaceutical Group Chemical Reagent Co., Ltd.) was mixed with 0, 24, 12, 6, and 3 ml of the aforementioned solution, respectively, to prepare solutions with fat contents of 0%, 2.5%, 5%, 10%, 20%, and 100%. These solutions were filled into six 100 ml EP tubes for subsequent scanning procedures.

Phantom validation experiments were performed before each scan. All animals underwent a baseline scan and one additional scan after the completion of the drug administration, totaling two scans. The scans were conducted in the 3.0T MRI room, and the scan parameters are provided in Table 10. The anesthesia procedure was as follows: intramuscular injection of 10 mg/kg ketamine hydrochloride, endotracheal intubation, and maintenance of anesthesia with isoflurane and oxygen using a ventilator. During the scanning process, veterinary monitoring of electrocardiography, blood oxygenation, respiratory rate, etc., was performed, and veterinary supervision was provided until the animals fully recovered consciousness and resumed spontaneous respiration.

**Table 10: MRI scan parameters of the liver**

| Features | No. | Sequence Name | Required level |
|---|---|---|---|
| Positioning like | 1 | 3-PI Loc SSFSE | NA |
| Water-fat separation | 2 | BH LAVA-Flex | Liver cross-section |
| Fat content | 3 | BH IDEAL IQ | Liver cross-section |

Image Post-processing and Analysis: The collected and stored Hepatic steatosis MRI images of Rhesus monkeys were analyzed using the 750W 3T MRI workstation. The liver MRI images were selected by choosing the planes with the maximum exposure of the right hepatic lobe area (layers 1-3), avoiding large blood vessels, bile ducts, and gallbladder to prevent volume effects. Regions of interest (ROI) were selected, with each ROI having an area of 90-110 $mm^2$. Hepatic steatosis was defined as MRI-PDFF% (Magnetic Resonance Imaging Proton Density Fat Fraction) > 6% (consistent with clinical standards).

<u>7.2 Experimental Results</u>

The in vivo pharmacological study of YN-011 was conducted in NASH Rhesus monkeys. The evaluated clinical parameters included liver lipid content assessed by MRI-PDFF%, NAFLD Activity Score (NAS), liver fibrosis stage assessed by liver histology, body weight, body mass index (BMI), blood lipid profile, fructose metabolism, other biochemical parameters, and food consumption.

The selected Rhesus monkeys were 11-23 years old, which corresponds to 30-70 years in humans. All Rhesus monkeys were males, with weights ranging from 13.63 to 22.85 kg. They had abnormal lipid metabolism for more than 2 years, MRI-PDFF% ranging from 7.8% to 11.9%, NAS scores of 3 or higher, and fibrosis scores of 0-1c within 6 months. These animals clinically met the definition of NASH.

Fifteen Rhesus monkeys were divided into 3 groups, with 5 monkeys in each group. They received subcutaneous injections of 0 (solvent control, placebo control group), 0.050, or 0.150 mg/kg of YN-011 once a week for 13 weeks (QW). The 0.150 mg/kg YN-011 group received adaptive dosing (0.1 mg/kg for the first dose, followed by 0.150 mg/kg for the remaining 12 doses).

During the study, all Rhesus monkeys received designated treatments, and none of them missed the scheduled biopsy or discontinued the treatment. No significant adverse reactions were observed in any of the treatment groups during the study.

As shown in Table 11, the liver lipid content decreased by approximately 40% after 13 weeks of weekly YN-011 treatment compared to the placebo control group.

To investigate whether YN-011 treatment led to histological improvements in the liver, liver biopsy specimens were collected from all Rhesus monkeys before the first injection and on day 89 after the first injection. Liver slices were stained with HE and Masson stains for histological analysis. The degrees of steatosis, inflammation, hepatocyte ballooning, and fibrosis were scored according to the standards for NAFLD activity and NASH progression. Liver biopsy results showed a reduction in both NAS and liver fibrosis scores in Rhesus monkeys treated with YN-011, with no significant progression of NASH (Table 12).
MRI-PDFF detected liver fat decreased from 9.0% ± 0.9% to 5.0% ± 0.2% in the 50 μg/kg YN-011 group and from 9.4% ± 1.5% to 5.6% ± 1.5% in the 150 μg/kg YN-011 group. Compared to baseline, the percentage reductions were 43.8% and 39.7%, respectively, at the end of the treatment. There was no significant difference in MRI-PDFF changes between the 50 μg/kg and 150 μg/kg YN-011 groups.

Histological analysis showed that in the 50 μg/kg YN-011 group, the average NAS decreased from 3.6 ± 0.5 at baseline to 1.6 ± 0.5 (P = 0.003). In the 150 μg/kg YN-011 group, the average NAS decreased from 3.6 ± 0.5 at baseline to 1.4 ± 0.5 (P < 0.001). There was no significant difference in NAS between the 50 μg/kg and 150 μg/kg YN-011 groups.

Regarding metabolic measurements, including liver injury biomarkers, lipid profiles, and body weight, YN-011 treatment demonstrated excellent improvements in both the 50 μg/kg and 150 μg/kg groups.

**Table 11: Effects of repeated subcutaneous administration of YN-011 on liver lipid content percentage change in NASH Rhesus monkeys after 13 weeks.**

| Group | Average ROI Baseline (%) | Mean ROI at day 80 of dosing (%) | Change rate (%) |
|---|---|---|---|
| Placebo | 8.9 ± 1.0 | 9.0 ± 1.1 | 1.9 ± 7.9 |
| YN-011 (0.05 mg/kg) | 9.0 ± 0.9 | 5.0 ± 0.2** | -43.8 ± 7.4## |
| YN-011 (0.15 mg/kg) | 9.4 ± 1.5 | 5.6 ± 1.5** | -39.7 ± 14.2## |
| *Note: ROI stands for Region of Interest; ** Compared to baseline, P < 0.01; ## Compared to placebo group, P < 0.01; Change rate = (Current value - Baseline value) / Baseline value × 100%.* | | | |

**Table 12: Effects of repeated subcutaneous administration of YN-011 on liver NAS and fibrosis scores in NASH Rhesus monkeys.**

| Group | Animal number | Baseline | | | | | Day 89 post-injection | | | | |
|---|---|---|---|---|---|---|---|---|---|---|---|
| | | Steatosis | Inflammation | Balloon-like degeneration | NAS Score | Fibrosis score | Steatosis | Inflammation | Balloon-like degeneration | NAS Score | Fibrosis score |
| YN-011 Group 150 μg/kg (n = 5) | 4801 | 2 | 1 | 0 | 3 | 0~1a | 0 | 1 | 0 | 1 | 0~1 a |
| | 1261 | 3 | 1 | 0 | 4 | 1b | 0 | 1 | 0 | 1 | 0~1 a |
| | 4283 | 2 | 1 | 0 | 3 | 1a | 1 | 0 | 0 | 1 | 1a |
| | 4985 | 2 | 1 | 1 | 4 | 1a | 1 | 1 | 0 | 2 | 1a |
| | 2065 | 2 | 1 | 1 | 4 | 1b | 1 | 1 | 0 | 2 | 1b |
| | Mean ± SD | / | / | / | 3.6 ± 0.5 | / | / | / | / | 1.4 ± 0.5** | / |

(continued)

| Group | Animal number | Baseline | | | | | Day 89 post-injection | | | | |
|---|---|---|---|---|---|---|---|---|---|---|---|
| | | Steatosis | Inflammation | Balloon-like degeneration | NAS Score | Fibrosis score | Steatosis | Inflammation | Balloon-like degeneration | NAS Score | Fibrosis score |
| YN-011 Group 50 μg/kg (n = 5) | 4821 | 1 | 1 | 1 | 3 | 1a | 0 | 1 | 1 | 2 | 1a |
| | 5091 | 1 | 1 | 1 | 3 | 1a | 0 | 1 | 0 | 1 | 0~1 a |
| | 1545 | 1 | 2 | 1 | 4 | 1c | 0 | 1 | 1 | 2 | 0~1 a |
| | 1919 | 2 | 2 | 0 | 4 | 0-1a | 1 | 1 | 0 | 2 | 0~1 a |
| | 157 | 1 | 3 | 0 | 4 | 1c | 0 | 1 | 0 | 1 | 1a |
| | Mean ± SD | / | / | / | 3.6 ± 0.5 | / | / | / | / | 1.6 ± 0.5 | / |
| Placebo group (n = 5) | 1477 | 1 | 2 | 0 | 4 | 1a | 2 | 1 | 0 | 4 | 1a |
| | 1493 | 1 | 2 | 0 | 3 | 1a | 1 | 2 | 0 | 3 | 1c |
| | 4779 | 3 | 1 | 0 | 3 | 1a | 3 | 1 | 0 | 3 | 1a |
| | 2043 | 1 | 2 | 0 | 3 | 1b | 1 | 2 | 0 | 3 | 1c |
| | 3581 | 2 | 1 | 0 | 3 | 1c | 2 | 1 | 0 | 3 | 1c |
| | Mean ± SD | / | / | / | 3.2 ± 0.4 | / | / | / | / | 3.2 ± 0.4 | / |

Furthermore, compared to baseline, the YN-011 treatment group (Day 7- Day 84) showed a significant decrease in body weight and BMI in NASH Rhesus monkeys. YN-011 treatment also significantly reduced serum low-density lipoprotein cholesterol (LDL-c), total cholesterol (TC), and total triglycerides (TG) at various measurement time points during the treatment period, with 0.15 mg/kg YN-011 displaying more consistent lipid-lowering effects. The levels of high-density lipoprotein cholesterol (HDL-c) in the YN-011 treatment group showed a decreasing trend compared to baseline. These changes in serum lipid profile were attributed to the reduced food consumption in YN-011 treated animals. Throughout the study, plasma fasting plasma glucose (FPG) levels fluctuated within the normal range, and no significant differences were detected between the YN-011 and placebo groups after YN-011 and placebo administration. Plasma fructosamine (FRA), a validated biomarker reflecting average glycemic control over the past 2-3 weeks, showed a decreasing trend in the YN-011 group one week after the first injection and a significant reduction of 4.3% after 12 weeks of treatment with 150 μg/kg YN-011. These results indicate that YN-011 has glucose control efficacy in NASH Rhesus monkeys. The dose-dependent reduction in food consumption observed in animals treated with YN-011 is a normal pharmacological effect of YN-011. In conclusion, these data demonstrate that multiple subcutaneous injections of YN-011 provide significant therapeutic benefits for NASH Rhesus monkeys.

**Example 8: In vitro experiments on neurodegenerative diseases**

8.1 TNF-α concentration-dependent decrease in the viability of SH-SY5Y neuronal cells

SH-SY5Y neuronal cells were digested using 0.25% trypsin to prepare a single-cell suspension. The single-cell suspension was seeded in a 96-well plate at a density of 10,000 cells per well in 100 μl of medium. The plate was placed in a CO2 incubator overnight (37°C, 5% CO2) to allow cell adhesion. The medium was replaced with medium containing 10% FBS or 5% FBS, and cells were stimulated with 20, 40, 60, 80, or 100 ng/ml TNF-α for 48 hours. Each drug treatment was performed in six replicate wells. After 48 hours of drug treatment, 10 μl of CCK-8 solution was added to each well, and the plate was gently shaken to mix the reagents. The plate was then incubated in a CO2 incubator for 1-4 hours. The absorbance at 450 nm was measured using a microplate reader, and cell viability was calculated using the following

formula:

$$\text{Cell viability (\%)} = [\text{A (treated)} - \text{A (blank)}] / [\text{A (control)} - \text{A (blank)}] \times 100$$

A (treated): OD value of wells containing cells, CCK-8 solution, and drug solution
A (control): OD value of wells containing cells and CCK-8 solution without drug solution
A (blank): OD value of wells without cells

The experimental results (as shown in Figure 9) demonstrated a significant decrease in the viability of SH-SY5Y neurons with increasing concentrations of TNF-α.

8.2 Inhibitory effects of YN-011 and γ-Aminobutyric acid (GABA) on TNF-α-induced decrease in SH-SY5Y cell viability

Please refer to section 8.1 for the experimental procedure, but the drug treatment in this experiment is different from section 8.1. Specifically, the drug treatment in this experiment is as follows: using medium containing 5% FBS, cells were treated with 60 ng/ml TNF-α alone or co-treated with 10, 100, or 500 nM YN-011, or co-treated with 1, 10, or 100 μM GABA for 48 hours. Each drug treatment was performed in six replicate wells.

The experimental results (as shown in Figure 10) revealed a significant decrease in SH-SY5Y cell viability in the presence of 60 ng/ml TNF-α, while treatment with 10, 100, and 500 nM YN-011 or 100 μM GABA significantly enhanced cell viability.

8.3 Significant increase in SH-SY5Y cell viability with the combined use of YN-011 and GABA

Please refer to section 8.1 for the experimental procedure, but the drug treatment in this experiment is different from section 8.1. Specifically, the drug treatment in this experiment is as follows: using medium containing 5% FBS, cells were treated with 60 ng/ml TNF-α alone or co-treated with 100 nM YN-011 and/or 100 μM GABA for 48 hours. Each drug treatment was performed in six replicate wells.

The experimental results (as shown in Figure 11) demonstrated a significant decrease in the viability of SH-SY5Y neurons in the presence of 60 ng/ml TNF-α, while co-treatment with 100 nM YN-011 and 100 μM GABA significantly enhanced cell viability. The combined use of YN-011 and GABA exhibited stronger potency in enhancing cell viability compared to individual use.

8.4 YN-011 reduces TNF-α-induced apoptosis in SH-SY5Y neuronal cells

The experimental procedure is as follows:

1) Digest SH-SY5Y neuronal cells in a 10 cm culture dish using 0.25% trypsin and seed them into a 12-well plate.
2) Place the culture plate in a CO2 incubator for overnight pre-culture (37°C, 5% CO2) to allow cell adhesion.
3) Using medium containing 5% FBS, treat the cells in each well with 60 ng/ml TNF-α alone or co-treated with 10, 100, or 500 nM YN-011. Incubate the cells at 37°C for 48 hours. Each drug treatment is performed in three replicate wells.
4) After 48 hours, discard the supernatant, gently wash the cells once with PBS, and place the cell culture plate on ice. Add 120 μl of RIPA cell lysis buffer (Beyotime P0013B) to each well, including protease and phosphatase inhibitors, and lyse the cells for 15 minutes.
5) Transfer the cell lysate to a 1.5 ml EP tube and centrifuge at 14,000 rpm for 30 minutes.
6) Carefully aspirate the supernatant into a new EP tube, add 5x loading buffer containing β-mercaptoethanol, and boil the samples at 100°C for 10 minutes to denature the proteins.
7) Perform SDS-PAGE gel electrophoresis with 10 μg of protein loaded per lane. Position the gel vertically against the power rack in the electrophoresis tank, ensuring that the concave side of the gel faces the power rack. Two gels can share one power rack. Fix the gel and power rack in the electrophoresis tank as required. Add the electrophoresis buffer to create separate chambers between the two gels and the buffer in the electrophoresis tank. Gently remove the comb from the gel.
8) Electrophoresis: Connect the electrophoresis tank to the power supply using two electrodes, ensuring that the red and black electrode plugs match the corresponding ports. During electrophoresis, use low voltage constant voltage electrophoresis for the upper gel. Turn on the power and adjust the voltage to 80 V (usually takes about 15 minutes). When the bromophenol blue dye enters the lower gel, switch to high voltage constant voltage electrophoresis and adjust the voltage to 120 V until the bromophenol blue dye reaches the bottom of the gel.
9) Perform wet transfer. The wet transfer sandwich arrangement is as follows: sponge/filter paper/gel/membrane/filter

paper/sponge, tightly arranged. There should be no air bubbles between the gel and membrane, and the orientation of the sandwich should be correct, with the negative electrode toward the protein side of the gel, migrating toward the positive electrode (membrane). After SDS-PAGE is complete, gently separate the two glass plates of the gel using a razor blade, allowing the gel to rest on one of the glass plates. Use a blade to cut the gel at the boundary between the separating gel and the stacking gel, and cut off a small corner in the upper left corner of the separating gel to mark the sample order. Then carefully transfer the gel into transfer buffer. Cut a PVDF membrane of the same size as the gel, soak it in methanol for 5 seconds. Cut 6 pieces of filter paper of the same size and equilibrate them with the transfer buffer at the same time as the PVDF membrane and the gel for 15 minutes. Place a sponge pad, filter paper, gel, membrane, filter paper, and sponge pad (from bottom to top) on the transfer apparatus, ensuring the exclusion of air bubbles, especially between the membrane and filter paper, gel and membrane, and filter paper and gel. Set the transfer current to a constant current of 200 mA, and the transfer time is approximately 90 minutes.

10) Remove the transferred PVDF membrane, rinse it briefly with TBST, discard TBST, and add 5% milk blocking solution to cover the PVDF membrane. Incubate at room temperature on a rocking shaker for 1 hour (30 rpm).

11) Incubate with primary antibodies. Remove the blocking solution, wash the membrane with TBST three times for 5 minutes each time, and cut the membrane according to the molecular weight. Add the appropriate dilution of primary antibodies (Bcl-2, CST #3498S, 1:1000; Cleaved-caspase3, CST #9661S, 1:1000; Caspase3, CST #9662S, 1:1000; HSP90, Proteintech #13171-1-AP, 1:10000) and incubate overnight at 4°C on a rocking shaker.

12) Wash the membrane. Take out the PVDF membrane incubated overnight and store the antibodies at -20°C. Place the PVDF membrane in TBST solution and quickly rinse it three times, each time for 15 minutes.

13) Incubate with secondary antibodies. Add the corresponding species-specific secondary antibodies (Jackson Lab, 1:10000) prepared in blocking solution to the membrane and incubate at room temperature for 1 hour with slow shaking.

14) Wash the membrane. Remove the secondary antibodies and place the PVDF membrane in TBST solution. Quickly rinse it three times, each time for 15 minutes.

15) Perform ECL chemiluminescence. Under dark conditions, prepare fresh developing solution (A solution:B solution = 1:1) according to the instructions of the ECL chemiluminescence kit (Millipore #WBKLS0500). Add the developing solution evenly to the membrane and place it in an imaging instrument for image acquisition.

16) Protein expression analysis. Use Image J software to analyze the grayscale values of western blot bands, and normalize the values to the grayscale value of the reference protein HSP90 to calculate the relative expression level of the target protein.

The experimental results (shown in Figure 12) demonstrate that YN-011 significantly reduces TNF-α-induced apoptosis in SH-SY5Y neuronal cells.

8.5 GABA reduces TNF-α-induced apoptosis in SH-SY5Y neuronal cells

Please refer to section 8.4 for the experimental procedure, but the drug treatment in step 3) of this experiment is different from that in section 8.4. In this experiment, the drug treatment in step 3) is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.

The experimental results (shown in Figure 13) demonstrate that GABA reduces TNF-α-induced apoptosis in SH-SY5Y neuronal cells.

8.6 Reducing TNF-α-induced apoptosis in SH-SY5Y neuronal cells by combined use of YN-011 and GABA

Please refer to section 8.4 for the experimental procedure, but the drug treatment in step 3) of this experiment is different from that in section 8.4. In this experiment, the drug treatment in step 3) is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells.

The experimental results (shown in Figure 14) demonstrate that the combined use of YN-011 and GABA significantly reduces TNF-α-induced apoptosis in SH-SY5Y neuronal cells.

8.7 TNF-α promotes damage in SH-SY5Y neuronal cells

Digest the SH-SY5Y neuronal cells in a 10 cm culture dish using 0.25% trypsin to prepare a single-cell suspension. Seed the single-cell suspension into a 24-well plate. Place the culture plate in a CO2 incubator and pre-culture overnight at 37°C with 5% CO2 to allow the cells to adhere. Treat the cells in each well with 20, 40, 60, or 80 ng/ml TNF-α using medium containing 5% FBS at 37°C for 48 hours. Each drug treatment is performed in three replicate wells.

Add the fluorescent dye Hoechst 33342 (Beyotime) at a dilution of 1:1000 to the wells, and incubate the plate at 37°C for 10 minutes. Remove the culture medium, gently wash twice with PBS, and immediately take the plate to a fluorescence microscope for imaging (20x magnification). Capture 10 fields of view for each cell well and count the number of blue-stained positive cells in each field of view.

The experimental results (shown in Figure 15) demonstrate that TNF-α promotes damage in SH-SY5Y neuronal cells.

8.8 Protective effect of YN-011 on TNF-α-induced damage in neuronal cells

Please refer to section 8.7 for the experimental procedure, but the drug treatment in this experiment is different from that in section 8.7. In this experiment, the drug treatment is as follows: using medium containing 5% FBS, treat the cells in each well with 60 ng/ml TNF-α alone or co-treated with 10, 100, or 500 nM YN-011 at 37°C for 48 hours. Each drug treatment is performed in three replicate wells.

The experimental results (shown in Figure 16) demonstrate that YN-011 has a protective effect on TNF-α-induced damage in neuronal cells.

8.9 Protective effect of GABA on TNF-α-induced damage in neuronal cells

Please refer to section 8.7 for the experimental procedure, but the drug treatment in this experiment is different from that in section 8.7. In this experiment, the drug treatment is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.

The experimental results (shown in Figure 17) demonstrate that GABA has a protective effect on TNF-α-induced damage in neuronal cells.

8.10 Protective effect of combined use of YN-011 and GABA on TNF-α-induced damage in neuronal cells

Please refer to section 8.7 for the experimental procedure, but the drug treatment in this experiment is different from that in section 8.7. In this experiment, the drug treatment is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells.

The experimental results (shown in Figure 18) demonstrate that the combined use of YN-011 and GABA has a protective effect on TNF-α-induced damage in neuronal cells.

8.11 YN-011 reduces apoptosis in TNF-α-induced neuronal cells

The experimental procedure is as follows:

1) Digest the SH-SY5Y neuronal cells in a 10 cm culture dish using 0.25% trypsin to prepare a single-cell suspension. Seed the single-cell suspension into a 24-well plate, placing a sterile coverslip in each well.
2) Place the culture plate in a CO2 incubator and pre-culture overnight at 37°C with 5% CO2 to allow the cells to adhere.
3) Using medium containing 5% FBS, treat the cells in each well with 60 ng/ml TNF-α alone or co-treated with 10, 100, or 500 nM YN-011 at 37°C for 48 hours. Each drug treatment is performed in three replicate wells.
4) After 48 hours, remove the culture medium and add 400 μl of 4% paraformaldehyde (PFA) to each well for fixation at room temperature for 15 minutes.
5) Wash twice with PBS for 5 minutes each.
6) Add 0.1% Triton X-100 for membrane permeabilization at room temperature for 10 minutes.
7) Wash twice with PBS for 5 minutes each.
8) Add 10% goat serum (GS) and incubate at room temperature for 30 minutes for blocking.
9) Remove the 10% GS and add the primary antibody Cleaved-caspase 3 (CST 9661S) diluted in 1% GS at a dilution ratio of 1:400, with 30 μl of antibody solution per coverslip.
10) Place in a refrigerator at 4°C overnight.
11) On the following day, remove the primary antibody, wash three times with PBS for 5 minutes each.
12) Add the fluorescent secondary antibody (Alexa Fluor® 488 Conjugate) and incubate at room temperature in the dark for 1 hour.
13) Wash three times with PBS for 5 minutes each.
14) Stain with DAPI for 5 minutes, then wash three times with PBS for 5 minutes each.

15) Apply mounting medium onto a clean glass slide, gently place the coverslip with the cells upside down on the mounting medium, and let it dry at room temperature in a cool, ventilated area overnight.
16) Capture images under a fluorescence microscope (40x magnification).
17) Capture 10 fields of view for each treatment group and count the number of green-stained positive cells in each field of view.

The experimental results (shown in Figure 19) demonstrate that YN-011 significantly reduces apoptosis in TNF-α-induced neuronal cells.

8.12 GABA reduces apoptosis in TNF-α-induced neuronal cells

Please refer to section 8.11 for the experimental procedure, but the drug treatment in this experiment is different from that in section 8.11. In this experiment, the drug treatment in step 3) is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.
The experimental results (shown in Figure 20) demonstrate that GABA reduces apoptosis in TNF-α-induced neuronal cells.

8.13 Combined use of YN-011 and GABA reduces apoptosis in TNF-α-induced neuronal cells

Please refer to section 8.11 for the experimental procedure, but the drug treatment in this experiment is different from that in section 8.11. In this experiment, the drug treatment in step 3) is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells.
The experimental results (shown in Figure 21) demonstrate that the combined use of YN-011 and GABA significantly reduces apoptosis in TNF-α-induced neuronal cells.

8.14 YN-011 reduces apoptosis in TNF-α-induced neuronal cells

Digest the SH-SY5Y neuronal cells in a 10 cm culture dish using 0.25% trypsin to prepare a single-cell suspension. Seed the single-cell suspension into a 12-well plate. Place the culture plate in a CO2 incubator and pre-culture overnight at 37°C with 5% CO2 to allow the cells to adhere. Using medium containing 5% FBS, treat the cells in each well with 60 ng/ml TNF-α alone or co-treated with 10, 100, or 500 nM YN-011 at 37°C for 48 hours. Each drug treatment is performed in three replicate wells. Collect the cells and digest the cells with 0.25% trypsin without EDTA for 1 minute. Transfer the cell suspension to a 1.5 ml EP tube, centrifuge at 1000 rpm for 5 minutes, and pellet the cells at the bottom of the tube. Resuspend 105 cells in 100 μl Binding Buffer, add 5 μl of PI and 5 μl of Annexin V-FITC solution, and incubate at room temperature in the dark for 15 minutes. Add 400 μl of Binding Buffer to each tube, and detect using a flow cytometer (Annexin V-FITC excitation wavelength: 488 nm, emission wavelength: 520 nm; PI excitation wavelength: 535 nm, emission wavelength: 617 nm). Count the number of Annexin V-FITC positive cells and PI negative cells in each sample.
The experimental results (shown in Figure 22) demonstrate that YN-011 significantly reduces apoptosis in TNF-α-induced neuronal cells.

8.15 GABA reduces apoptosis in TNF-α-induced neuronal cells

Please refer to section 8.14 for the experimental procedure, but the drug treatment in this experiment is different from that in section 8.14. In this experiment, the drug treatment is as follows: using medium containing 5% FBS, treat the cells in each well with 60 ng/ml TNF-α alone or co-treated with 1, 10, or 100 μM GABA at 37°C for 48 hours. Each drug treatment is performed in three replicate wells.
The experimental results (shown in Figure 23) demonstrate that GABA reduces apoptosis in TNF-α-induced neuronal cells.

8.16 Combined use of YN-011 and GABA reduces apoptosis in TNF-α-induced neuronal cells

Please refer to section 8.14 for the experimental procedure, but the drug treatment in this experiment is different from that in section 8.14. In this experiment, the drug treatment is as follows: using medium containing 5% FBS, treat the cells in each well with 60 ng/ml TNF-α alone or co-treated with 100 μM GABA and/or 100 nM YN-011 at 37°C for 48 hours. Each drug treatment is performed in three replicate wells.
The experimental results (shown in Figure 24) demonstrate that the combined use of YN-011 and GABA

significantly reduces apoptosis in TNF-α-induced neuronal cells.

8.17 GABA reduces mRNA expression of inflammatory factors in HMC3 microglial cells induced by Aβ1-42 oligomers

Under sterile conditions, dissolve the synthesized Aβ1-42 peptide (AnaSpec #AS-20276) in hexafluoroisopropanol (Mecklin #H811026) to obtain a 1 mM solution. Divide the solution into 1.5 ml sterile centrifuge tubes and evaporate the hexafluoroisopropanol in a vacuum evaporator. The dried peptide forms a film in the tubes and is stored at -20°C for later use. Freshly prepare Aβ1-42 oligomers from the dried peptide film as follows: dissolve the dried peptide film in anhydrous DMSO to obtain a 5 mM solution, then dilute with cold cell culture basal medium to obtain a 100 μM stock solution. After incubating at 4°C for 24 hours, use the stock solution for cell treatment.

Seed HMC3 human microglial cells into a 24-well plate. After overnight incubation at 37°C with 5% CO2, treat the cells in each well with 10 μM Aβ1-42 oligomers alone or co-treated with 1, 10, or 100 μM GABA for 48 hours. Each drug treatment is performed in three replicate wells. After 48 hours of treatment, isolate total RNA from the cells in each well using 1 ml of Trizol (Invitrogen #15596026) following the manufacturer's instructions. Dissolve the RNA pellet in 20 μl of DEPC-treated water. Measure the RNA concentration and purity using a NanoDrop 2000. Convert RNA into cDNA using a reverse transcription kit (Yeasen #11141ES60). Prepare the PCR reaction as follows: 5 μl of PCR mix (Yeasen #10108ES03), 0.2 μl of forward primer, 0.2 μl of reverse primer, 1 μl of cDNA, and 3.6 μl of water. Perform qPCR on an Applied Biosystems 7500 real-time PCR system using the following thermal program: initial denaturation at 95°C for 3 minutes, followed by 40 cycles of denaturation at 95°C for 30 seconds, annealing at 60°C for 30 seconds, and extension at 72°C for 1 minute. Compare the relative mRNA expression of TNF-α and IL-6 between treated and untreated cells using the ΔΔCt method, normalized to the expression of the housekeeping gene RPLP0.

The experimental results (shown in Figure 25) demonstrate that in the presence of 10 μM Aβ1-42 oligomers, the mRNA expression of TNF-α and IL-6 in HMC3 cells is significantly increased, while 100 μM GABA significantly reduces the mRNA expression of TNF-α, and both 10 μM and 100 μM GABA significantly reduce the mRNA expression of IL-6.

8.18 YN-011 reduces mRNA expression of inflammatory factors in HMC3 microglial cells induced by Aβ1-42 oligomers.

Please refer to section 8.17 for the experimental procedure, but the drug treatment in this experiment is different from that in section 8.17. In this experiment, the drug treatment is as follows: treat the cells in each well with 10 μM Aβ1-42 oligomers alone or co-treated with 10, 100, or 500 nM YN-011 for 48 hours. Each drug treatment is performed in three replicate wells.

The experimental results (shown in Figure 26) demonstrate that in the presence of 10 μM Aβ1-42 oligomers, the mRNA expression of TNF-α and IL-6 in HMC3 cells is significantly increased, while 100 nM and 500 nM YN-011 significantly reduce the mRNA expression of TNF-α and IL-6.

8.19 Combined use of YN-011 and GABA reduces mRNA expression of inflammatory factors in HMC3 microglial cells induced by Aβ1-42 oligomers.

Please refer to section 8.17 for the experimental procedure, but the drug treatment in this experiment is different from that in section 8.17. In this experiment, the drug treatment is as follows: treat the cells in each well with 10 μM Aβ1-42 oligomers alone or co-treated with 100 μM GABA and/or 100 nM YN-011 for 48 hours. Each drug treatment is performed in three replicate wells.

The experimental results (shown in Figure 27) demonstrate that compared to the individual treatment of GABA or YN-011, the combined use of 100 μM GABA and 100 nM YN-011 exhibits significantly stronger inhibitory effects on the TNF-α mRNA expression induced by 10 μM Aβ1-42 oligomers.

8.20 GABA reduces inflammatory factor expression in HMC3 microglial cells induced by Aβ1-42 oligomers.

Seed HMC3 human microglial cells into a 12-well plate. After overnight incubation at 37°C with 5% CO2, treat the cells in each well with 10 μM Aβ1-42 oligomers alone or co-treated with 1, 10, or 100 μM GABA for 48 hours. Each drug treatment is performed in three replicate wells. After 48 hours of treatment, wash the cells with PBS and lyse the cells in RIPA buffer supplemented with protease inhibitor and phosphatase inhibitor on ice for 15 minutes. Centrifuge the cell lysate at 14,000 rpm for 30 minutes. Collect the supernatant, mix with 5x loading buffer supplemented with β-mercaptoethanol, and boil at 100°C for 10 minutes. Separate denatured proteins by SDS-PAGE. Briefly, load 10 μg of protein onto precast mini polyacrylamide gels (SurePAGE, GenScript #M00657) and run at 120 V voltage. Subsequently, transfer the gel onto a PVDF membrane (0.22 μm pore size) at 200 mA for 90 minutes. After unblocking, incubate the membrane at room temperature with primary antibodies diluted in 5% milk solution (TNF-α, Proteintech #60291-Ig; IL-6, Proteintech

#21865-1-AP; HSP90, Proteintech #13171-1-AP) overnight at 4°C. After washing, incubate the membrane with corresponding secondary antibodies (Jackson Lab, 1:10000) at room temperature for 1 hour. After washing, visualize the antibody signals on the membrane using an ECL kit (Millipore #WBKLS0500) following the manufacturer's instructions. Calculate the relative protein levels normalized to the endogenous protein HSP90 using ImageJ.

The results (shown in Figure 28) demonstrate that in the presence of 10 μM Aβ1-42 oligomers, the expression of TNF-α and IL-6 in HMC3 cells is significantly increased, while 100 μM GABA significantly reduces IL-6 expression, and both 10 μM and 100 μM GABA significantly reduce TNF-α expression.

8.21 YN-011 reduces inflammatory factor expression in HMC3 microglial cells induced by Aβ1-42 oligomers.

Please refer to section 8.20 for the experimental procedure, but the drug treatment in this experiment is different from that in section 8.20. In this experiment, the drug treatment is as follows: treat the cells in each well with 10 μM Aβ1-42 oligomers alone or co-treated with 10, 100, or 500 nM YN-011 for 48 hours. Each drug treatment is performed in three replicate wells.

The experimental results (shown in Figure 29) demonstrate that in the presence of 10 μM Aβ1-42 oligomers, the expression of TNF-α and IL-6 in HMC3 cells is significantly increased, while 100 nM and 500 nM YN-011 significantly reduce the expression of TNF-α and IL-6.

8.22 Combined use of YN-011 and GABA reduces inflammatory factor expression in HMC3 microglial cells induced by Aβ1-42 oligomers.

Please refer to section 8.20 for the experimental procedure, but the drug treatment in this experiment is different from that in section 8.20. In this experiment, the drug treatment is as follows: treat the cells in each well with 10 μM Aβ1-42 oligomers alone or co-treated with 100 μM GABA and/or 100 nM YN-011 for 48 hours. Each drug treatment is performed in three replicate wells.

The experimental results (shown in Figure 30) demonstrate that compared to the individual treatment of GABA or YN-011, the combined use of 100 μM GABA and 100 nM YN-011 exhibits significantly stronger inhibitory effects on the expression of TNF-α and IL-6 induced by 10 μM Aβ1-42 oligomers.

**Example 9: Neurodegenerative In Vivo Disease**

Neurodegenerative diseases such as Alzheimer's disease (AD), Parkinson's disease (PD), Huntington's disease (HD), etc., are central nervous system disorders characterized by progressive loss of function in central neurons or their myelin sheaths, resulting in gradual loss of neuronal cell structure and function, leading to symptoms such as dementia and movement disorders.

Among them, Alzheimer's disease (AD), also known as senile dementia, is characterized by progressive cognitive impairment and memory loss, with no effective treatment currently available. Studies have shown that AD patients' brains accumulate neurotoxic plaques formed by abnormal aggregation of beta-amyloid (Aβ). Aβ can cause the formation of senile plaques in the brain and apoptosis of nerve cells, making it a crucial factor in the development of AD. Aβ is a peptide consisting of 39-43 amino acids, and the most common subtypes of Aβ in the human body are Aβ40 and Aβ42, which are prone to aggregation, leading to the formation of Aβ deposits and subsequent neurotoxic effects.

Research has shown that GLP-1 has neuroprotective biological effects and can improve AD symptoms [10, 25, 26]. In animal models, GLP-1 improves cognitive function in AD mice, reduces Aβ deposition, alleviates glial cell overactivation induced by Aβ, and mitigates oxidative stress and inflammation in the brain, demonstrating the neuroprotective role of GLP-1 in AD. The present invention discloses a long-acting GLP-1 receptor agonist (GLP-1RA) that exerts the biological effect of protecting neurons and improving central nervous system function, thereby helping to improve AD symptoms.

The experiment uses model mice with Alzheimer's disease and wild-type control adult mice of the same genetic background. The animals are individually housed in cages, maintaining a 12/12-hour light/dark cycle (lights on at 08:00, lights off at 20:00), and the temperature is kept at 21.5 degrees Celsius. Food and water are available ad libitum. Prior to the behavioral experiments, the mice are intraperitoneally (i.p.) injected with YN-011 or a placebo for 16 weeks.

Morris Water Maze Setup

The maze is made of white opaque plastic with a diameter of 120 cm and wall height of 40 cm, and it is filled with water at 25°C to avoid excessive cooling of body temperature. A small escape platform (10 × 6.5 × 21.5 cm) is placed in a fixed position within a quadrant, 25 cm away from the surrounding walls, and hidden 1 cm below the water surface. There are several fixed visual cues on the walls of the room.

Spatial Memory

Four equidistant points (north, south, east, west) along the circumference of the pool serve as starting positions. The starting positions are randomly assigned for training in four trials per day. During training, the mice are trained to find a fixed safety platform submerged in the pool. After 24 hours following the end of the training period, the safety platform is removed from the pool, and the mice's swimming path length and time are recorded, starting from a random starting position (n = 12 per group). Data collected are analyzed using one-way ANOVA and two-way ANOVA to evaluate spatial memory, represented by the time and path length required to reach the original position of the safety platform, and spatial acuity, represented by the time spent searching in the area where the safety platform was located.

Detection of Aβ40 and Aβ42 Levels

Aβ40 and Aβ42 levels are measured using an Aβ detection kit. In brief, the hemispheres of the brains from AD model mice treated with YN-011 and control mice are homogenized in Tris-buffered saline (25 mM Tris HCl, pH 7.4; 150 mM NaCl) containing a proteinase inhibitor (Sigma, 250 ml/5 ml buffer). The brain homogenates are then centrifuged at 100,000 g and 4°C for 1 hour. The supernatant is then diluted 1:10, and ELISA is performed. The ELISA measures only soluble beta-amyloid oligomer levels and not monomers. Protein quantification is performed using the Bradford protein assay. The final Aβ values are determined after normalization to total protein levels (n = 6 per group).

**Example 10: Phase IIb and III Clinical Trials of YN-011 (Monotherapy)**

10.1 Trial Design

The Phase IIb and III clinical trials are multicenter, randomized, double-blind, placebo-controlled studies conducted to evaluate the efficacy and safety of YN-011 in patients with poorly controlled type 2 diabetes mellitus (T2DM) despite dietary and exercise interventions. The Phase IIb stage is an exploratory clinical trial to investigate dose response and safety assessment (divided into four groups: YN-011 1 mg group, YN-011 2 mg group, YN-011 3 mg group, and placebo control group), aiming to obtain the Recommended Phase 3 Dose (RP3D) of YN-011 for the Phase III stage. The Phase III stage is a confirmatory clinical trial of efficacy.. Key Inclusion Criteria for Phase IIb and III Studies:

- Age between 18 and 75 years at screening
- Diagnosed with type 2 diabetes for at least 8 weeks (according to WHO 2000 criteria) and not receiving any antihyperglycemic drug treatment in the 8 weeks before screening
- Glycated hemoglobin HbA1c: 7.5% ≤ HbA1c ≤ 11% at screening
- Glycated hemoglobin HbA1c: 7.5% ≤ HbA1c ≤ 10.5% before randomization
- Fasting plasma glucose (FPG) < 13.9 mmol/L at screening and before randomization
- BMI ≥ 18.5 kg/m2 and ≤ 40 kg/m2

Key Exclusion Criteria for Phase IIb and III Studies:

- Type 1 diabetes
- Use of any DPP-4 inhibitors and/or GLP-1 analogs in the 3 months before screening
- Continuous insulin treatment for more than 14 days in the year before screening (excluding gestational diabetes requiring insulin treatment)
- Fasting C-peptide < 0.3 nmol/mL
- Diabetic ketoacidosis, diabetic lactic acidosis, or hyperosmolar non-ketotic diabetic coma in the 6 months before screening
- Unstable or requiring treatment for proliferative retinopathy or maculopathy, severe diabetic neuropathy, intermittent claudication, or diabetic foot in the 6 months before screening
- Severe hypoglycemic event without apparent cause (Grade 3 hypoglycemia) in the 6 months before screening, or 3 or more hypoglycemic events (blood glucose < 3.9 mmol/L) in the month before screening, or recurrent symptoms related to hypoglycemia
- Serious trauma, severe infection, or surgery within the month before screening that may affect glycemic control
- Blood donation or significant blood loss (>400 mL) or blood transfusion in the past 3 months
- Uncontrolled hypertension
- Patients with a history of acute or chronic pancreatitis, symptomatic gallbladder disease, pancreatic injury, or other

high-risk factors for pancreatitis, or patients with amylase and/or lipase ≥ 1.5 times the upper limit of normal (ULN) at screening
- History of medullary thyroid carcinoma, multiple endocrine neoplasia (MEN) 2A or 2B syndrome, or relevant family history; or history of other malignant tumors
- Clinically significant gastric emptying abnormalities, severe chronic gastrointestinal disease, long-term use of medications directly affecting gastrointestinal motility, or gastrointestinal surgery within the 6 months before screening, as determined by the investigator
- Blood disorders or any condition causing hemolysis or red blood cell instability
- Uncontrolled hyperthyroidism or hypothyroidism
- Positive for hepatitis B surface antigen (HBsAg) with hepatitis B virus load (HBV-DNA) higher than the local laboratory detection limit, positive for hepatitis C antibody (HCV-Ab), human immunodeficiency virus antibody (HIV-Ab), treponema pallidum antibody (TP-Ab), or positive for novel coronavirus pneumonia (COVID-19) nucleic acid test
- Acute or chronic hepatitis or laboratory findings meeting any of the following criteria: alanine aminotransferase (ALT) level ≥ 2.5 × ULN and/or aspartate aminotransferase (AST) ≥ 2.5 × ULN, fasting triglycerides > 5.7 mmol/L, estimated glomerular filtration rate (eGFR) calculated by the CKD-EPI (EPI-(Scr)) equation < 60 mL/min/1.73 m2
- Any other condition that the investigator or primary physician deems unsuitable for participation in the study

The treatment regimen for the Phase IIb stage dose confirmation is as follows for enrolled subjects:

Subjects Enrolled in the Phase IIb Efficacy Confirmation Stage:

| Group | Treatment (subcutaneous abdominal injection, 1 time/week, 52 weeks of continuous dosing) | | |
|---|---|---|---|
| | Double-blind treatment period (first 12 weeks) | Double-blind treatment period (post 12 weeks) | Open treatment period (28 weeks) |
| | 24 weeks in total | | |
| Dose Group 1 | YN-011 1mg | YN-011 1mg | Subjects entering open treatment prior to RP3D determination: YN-011 (1mg)<br>Subjects entering the open treatment period after RP3D determination:<br>· YN-011 dose group selected by IDMC (RP3D low dose group or high dose group): maintain the original dose treatment<br>· Other subjects were randomized twice in a 1:1 ratio to receive either low or high dose of YN-011 RP3D |
| Dose Group 2 | YN-011 2mg | YN-011 2mg | |
| Dose Group 3 | YN-011 3mg | YN-011 3mg | |
| Control Group | Placebo | Placebo | Subjects entering open treatment prior to RP3D determination: YN-011 (1mg)<br>Subjects entering the open treatment period after RP3D determination: secondary randomization in a 1:1 ratio to receive either low or high dose of YN-011 RP3D |

Subjects Enrolled in the Phase III Efficacy Confirmation Stage:

| Group | Treatment (subcutaneous abdominal injection, 1 time/week, 52 weeks of continuous dosing) | |
|---|---|---|
| | Double-blind treatment period (24 weeks) | Open treatment period (28 weeks) |
| Dose Group 1 | YN-011 RP3D low dose, open treatment period to maintain the original dose treatment | |

(continued)

| Group | Treatment (subcutaneous abdominal injection, 1 time/week, 52 weeks of continuous dosing) | |
|---|---|---|
| | Double-blind treatment period (24 weeks) | Open treatment period (28 weeks) |
| Dose Group 2 | YN-011 RP3D high dose, open treatment period to maintain the original dose treatment | |
| Control Group | Placebo | Secondary randomization in a 1:1 ratio to receive either low or high dose of YN-011 RP3D |

Primary efficacy endpoints included the comparison of YN-011 with placebo in terms of the change in HbA1c levels from baseline after 12 weeks (Phase IIb) or 24 weeks (Phase III) of double-blind administration in patients with T2DM. Secondary efficacy endpoints mainly included changes from baseline (at 12 weeks for Phase IIb and 24 weeks for Phase III) in FPG, fasting insulin, fasting C-peptide, fasting glucagon, fasting lipid profile, and fasting body weight. Other secondary endpoints included HbA1c target achievement rate (proportion of subjects with HbA1c <7.0% and <6.5%), glucose area under the curve during mixed meal tolerance test (MMTT), and insulin or C-peptide area under the curve during MMTT. Safety assessments included adverse events, laboratory tests, vital signs, and 12-lead electrocardiogram evaluation.

10.2 Treatment Effect of YN-011 in Type 2 Diabetes

Clinical trial results showed a reduction of 1.73% in HbA1c levels after 24 weeks of treatment with 1 mg YN-011. It has been reported that semaglutide at a dose of 1 mg reduced HbA1c levels by 1.55% after 30 weeks of treatment (Sorli et al., Lancet Diabetes Endocrinol 2017; 5: 251-60), and dulaglutide at a dose of 1.5 mg reduced HbA1c levels by 1.46% after 26 weeks of treatment (Shi et al., J Diabetes Investig 2020; 11: 142-150).
urthermore, clinical trial results also showed that the incidence of hypoglycemia (<3.9 mmol/L) after 24 weeks of treatment with 1 mg and 3 mg YN-011 was 0.8% and 1.7%, respectively. It has been reported that the incidence of hypoglycemia after 26 weeks of treatment with dulaglutide at doses of 0.75 mg and 1.5 mg was 4.1% and 6.3%, respectively (Shi et al., J Diabetes Investig 2020; 11: 142-150).
Moreover, the incidence of nausea after 24 weeks of treatment with 1 mg and 3 mg YN-011 was 3.4% and 6.0%, respectively. It has been reported that the incidence of nausea after 30 weeks of treatment with semaglutide at doses of 0.5 mg and 1 mg was 20% and 24%, respectively (Sorli et al., Lancet Diabetes Endocrinol 2017; 5: 251-60), and the incidence of nausea after 26 weeks of treatment with dulaglutide at a dose of 1.5 mg was 9.5% (Shi et al., J Diabetes Investig 2020; 11: 142-150). For liraglutide, the incidence of nausea after 24 weeks of treatment with 1 mg and 3 mg doses was 5.6% and 10%, respectively (Shuai et al., Diabetes Obes Metab. 2021; 23(1):116-124).

**Example 11: Phase IIb and III Clinical Trials of YN-011 in Combination with Metformin**

11.1 Trial Design

The Phase IIb and III clinical trials were multicenter, randomized, double-blind, placebo-controlled studies evaluating the efficacy and safety of YN-011 in patients with T2DM who had inadequate glycemic control on metformin therapy. The Phase IIb trial aimed to explore dose response and evaluate safety (divided into three groups: YN-011 1 mg + metformin, YN-011 3 mg + metformin, and placebo + metformin), to determine the Recommended Phase 3 Dose (RP3D) for the Phase III trial, which aimed at efficacy confirmation..
The inclusion criteria for subjects in the Phase IIb and III studies of YN-011 in combination with metformin were primarily the same as those in Implementation Example 10, except for the following point:

- Diagnosed with type 2 diabetes for at least 8 weeks (WHO 2000), and met one of the following conditions:

  a) Received monotherapy with metformin for ≥8 weeks with a dose of ≥1500 mg/day or maximum tolerated dose (<1500 mg/day but ≥1000 mg/day) (eligible subjects could directly enter the run-in period).
  b) Received monotherapy with metformin for <8 weeks with a dose of ≥1500 mg/day or maximum tolerated dose (<1500 mg/day but ≥1000 mg/day) (eligible subjects needed to enter the metformin dose stabilization period).
  c) Received monotherapy with metformin at a dose <1500 mg/day and had not reached the maximum tolerated dose (eligible subjects needed to enter the metformin dose titration period and dose stabilization period).

Please refer to the exclusion criteria in Implementation Example 10 for the primary exclusion criteria for subjects in the Phase IIb and III studies of YN-011 in combination with metformin.

The treatment regimen for subjects enrolled in the Phase IIb dose confirmation stage is as follows:

Subjects enrolled in the Phase IIb dose confirmation stage:

| Group | Treatment (subcutaneous abdominal injection, 1 time/week, 52 weeks of continuous dosing) | | |
|---|---|---|---|
| | Double-blind treatment period (first 12 weeks) | Double-blind treatment period (post 12 weeks) | Open treatment period (28 weeks) |
| | 24 weeks in total | | |
| Dose Group 1 | YN-011 1mg | YN-011 1mg | Before the completion of the interim analysis: YN-011 1mg<br>After completion of interim analysis: treated with YN-011 RP3D dose |
| Dose Group 2 | YN-011 3mg | YN-011 3mg | |
| Control Group | Placebo | Placebo | |

Subjects enrolled in the Phase III dose confirmation stage:

| Group | Treatment (subcutaneous abdominal injection, 1 time/week, 52 weeks of continuous dosing) | |
|---|---|---|
| | Double-blind treatment period (24 weeks) | Open treatment period (28 weeks) |
| YN-011 Group | YN-011 RP3D dose, open treatment period to maintain the original dose treatment | |
| Control Group | Placebo | YN-011 RP3Dose treatment |

Primary efficacy endpoints included the comparison of YN-011 in combination with metformin versus placebo in combination with metformin in terms of the change in HbA1c levels from baseline after 12 weeks (Phase IIb) or 24 weeks (Phase III) of double-blind administration in patients with T2DM who had inadequate glycemic control on metformin therapy. Please refer to Implementation Example 10 for the secondary efficacy endpoints and safety assessments.

11.2 Treatment Effect of YN-011 in Combination with Metformin in Type 2 Diabetes

Clinical trial results showed a reduction of 1.8% in HbA1c levels and 2.42% in FPG levels after 24 weeks of treatment with 3 mg YN-011 in combination with metformin. It has been reported that dulaglutide at a dose of 1.5 mg in combination with metformin reduced HbA1c levels by 1.42% and FPG levels by 1.93% after 40 weeks of treatment (Dungan et al., Lancet 2014; 384: 1349-57).

Furthermore, clinical trial results also showed that the incidence of hypoglycemia (<3.9 mmol/L) after 24 weeks of treatment with 3 mg YN-011 in combination with metformin was 1.8%, which is comparable to the incidence of hypoglycemia (1.7%) in the placebo group in combination with metformin. It has been reported that the incidence of hypoglycemia after 40 weeks of treatment with dulaglutide at a dose of 1.5 mg in combination with metformin was 9% (Dungan et al., Lancet 2014; 384: 1349-57).

Moreover, the incidence of nausea after 24 weeks of treatment with 3 mg YN-011 in combination with metformin was 7.0%. It has been reported that the incidence of nausea after 40 weeks of treatment with dulaglutide at a dose of 1.5 mg in combination with metformin was 20% (Dungan et al., Lancet 2014; 384: 1349-57), and the incidence of nausea after 30 weeks of treatment with semaglutide at a dose of 1 mg in combination with metformin was 13.4% (Ji et al., Diabetes Obes Metab. 2021; 23:404-414).

The examples provided above represent the preferred embodiments disclosed in the present application. However, it should be understood that the present application is not limited to the disclosed examples. Instead, the present application aims to cover various modifications and equivalent examples within the spirit and scope of the appended claims.

All publications, patents, and patent applications are incorporated by reference in their entirety into the present disclosure. Specifically, the sequences associated with each accession number provided in the present disclosure,

including, for example, protein and/or nucleotide sequences provided in tables or elsewhere, are incorporated by reference.

The scope of the claims should not be limited by the preferred examples and embodiments, but should be understood as the broadest interpretation consistent with the specification.

## References

1. Leech, C.A., et al., Expression of cAMP-regulated guanine nucleotide exchange factors in pancreatic beta-cells. Biochem Biophys Res Commun, 2000. 278(1): p. 44-7.
2. Drucker, D.J., Glucagon-like peptides. Diabetes, 1998. 47(2): p. 159-69.
3. Montrose-Rafizadeh, C., et al., Pancreatic glucagon-like peptide-1 receptor couples to multiple G proteins and activates mitogen-activated protein kinase pathways in Chinese hamster ovary cells. Endocrinology, 1999. 140(3): p. 1132-40.
4. Ahren, B. and O. Schmitz, GLP-1 receptor agonists and DPP-4 inhibitors in the treatment of type 2 diabetes. Horm Metab Res, 2004. 36(11-12): p. 867-76.
5. Green, B.D., et al., Structurally modified analogues of glucagon-like peptide-1 (GLP-1) and glucose-dependent insulinotropic polypeptide (GIP) as future antidiabetic agents. Curr Pharm Des, 2004. 10(29): p. 3651-62.
6. Chang, A.M., et al., The GLP-1 derivative NN2211 restores beta-cell sensitivity to glucose in type 2 diabetic patients after a single dose. Diabetes, 2003. 52(7): p. 1786-91.
7. Liu, H.K., et al., N-acetyl-GLP-1: a DPP IV-resistant analogue of glucagon-like peptide-1 (GLP-1) with improved effects on pancreatic beta-cell-associated gene expression. Cell Biol Int, 2004. 28(1): p. 69-73.
8. Kim, J.G., et al., Development and characterization of a glucagon-like peptide 1-albumin conjugate: the ability to activate the glucagon-like peptide 1 receptor in vivo. Diabetes, 2003. 52(3): p. 751-9.
9. Nielsen, R., et al., Effect of liraglutide on myocardial glucose uptake and blood flow in stable chronic heart failure patients: A double-blind, randomized, placebo-controlled LIVE sub-study. J Nucl Cardiol, 2019. 26(2): p. 585-597.
10. Duarte, A.I., et al., Liraglutide Protects Against Brain Amyloid-beta1-42 Accumulation in Female Mice with Early Alzheimer's Disease-Like Pathology by Partially Rescuing Oxidative/Nitrosative Stress and Inflammation. Int J Mol Sci, 2020. 21(5).
11. Elbassuoni, E.A. and R.F. Ahmed, Mechanism of the neuroprotective effect of GLP-1 in a rat model of Parkinson's with pre-existing diabetes. Neurochem Int, 2019. 131: p. 104583.
12. Muskiet, M.H.A., et al., GLP-1 and the kidney: from physiology to pharmacology and outcomes in diabetes. Nat Rev Nephrol, 2017. 13(10): p. 605-628.
13. Hou, Y., et al., Nutrient Optimization Reduces Phosphorylation and Hydroxylation Level on an Fc-Fusion Protein in a CHO Fed-Batch Process. Biotechnol J, 2019. 14(3): p. e1700706.
14. Karlin, S. and S.F. Altschul, Methods for assessing the statistical significance of molecular sequence features by using general scoring schemes. Proc Natl Acad Sci U S A, 1990. 87(6): p. 2264-8.
15. Karlin, S. and S.F. Altschul, Applications and statistics for multiple high-scoring segments in molecular sequences. Proc Natl Acad Sci U S A, 1993. 90(12): p. 5873-7.
16. Altschul, S.F., et al., Basic local alignment search tool. J Mol Biol, 1990. 215(3): p. 403-10.
17. Altschul, S.F., et al., Gapped BLAST and PSI-BLAST: a new generation of protein database search programs. Nucleic Acids Res, 1997. 25(17): p. 3389-402.
18. Myers, E.W. and W. Miller, Optimal alignments in linear space. Comput Appl Biosci, 1988. 4(1): p. 11-7.
19. Bettinger, J.Q., et al., Quantitative Analysis of in Vivo Methionine Oxidation of the Human Proteome. J Proteome Res, 2020. 19(2): p. 624-633.
20. Allen, L.V., Jr., Remington: The Science and Practice of Pharmacy: from the past into the future. Int J Pharm Compd, 2012. 16(5): p. 358-62.
21. Toft-Nielsen, M.B., S. Madsbad, and J.J. Holst, Continuous subcutaneous infusion of glucagon-like peptide 1 lowers plasma glucose and reduces appetite in type 2 diabetic patients. Diabetes Care, 1999. 22(7): p. 1137-43.
22. Geiser, J.S., et al., Clinical Pharmacokinetics of Dulaglutide in Patients with Type 2 Diabetes: Analyses of Datafrom Clinical Trials. Clin Pharmacokinet, 2016. 55(5): p. 625-34.
23. Bodanszky, M., Principles of peptide synthesis. 2nd rev. ed. Springer laboratory. 1993, Berlin ; New York: Springer-Verlag. xii, 329 p.
24. Pratley, R.E., et al., Semaglutide versus dulaglutide once weekly in patients with type 2 diabetes (SUSTAIN 7): a randomised, open-label, phase 3b trial. Lancet Diabetes Endocrinol, 2018. 6(4): p. 275-286.
25. Zheng, J., et al., GLP-1 improves the supportive ability of astrocytes to neurons by promoting aerobic glycolysis in Alzheimer's disease. Mol Metab, 2021. 47: p. 101180.

26. Park, J.S., et al., Blocking microglial activation of reactive astrocytes is neuroprotective in models of Alzheimer's disease. Acta Neuropathol Commun, 2021. 9(1): p. 78.

## Claims

**1.** A fusion protein comprising a GLP-1 polypeptide and an immunoglobulin Fc domain, wherein the GLP-1 polypeptide is covalently linked to the immunoglobulin Fc domain, and the GLP-1 polypeptide is selected from human GLP-1 (7-37), human GLP-1 (7-36), and DPP-IV resistant human GLP-1, and the GLP-1 polypeptide contains one or more amino acid substitutions selected from the group consisting of A8G, G22E, and R36G relative to native human GLP-1; The immunoglobulin Fc domain comprises or is an IgG2-Fc domain, and the IgG2-Fc domain contains one or more amino acid substitutions selected from the group consisting of C222S, A330S, and P331S.

**2.** The fusion protein according to claim 1, wherein the GLP-1 polypeptide has a certain level of hydroxylation at lysine 34 (K34) relative to native human GLP-1.

**3.** The fusion protein according to claim 1 or 2, wherein the hydroxylation level is between 10% and 100%, for example, greater than or equal to 10%, or greater than or equal to 15%, or greater than or equal to 20%, or greater than or equal to 26%, or greater than or equal to 30%, or greater than or equal to 40%, or greater than or equal to 50%, or greater than or equal to 60%, or greater than or equal to 70%, or greater than or equal to 80%, or greater than or equal to 90%.

**4.** The fusion protein according to any one of claims 1-3, wherein the GLP-1 polypeptide is substantially unoxidized at tryptophan 31 (W31) relative to native human GLP-1.

**5.** The fusion protein according to claim 4, wherein oxidation level of the GLP-1 polypeptide at W31 relative to native human GLP-1 is less than 0.5% or undetectable.

**6.** The fusion protein according to any one of claims 1-5, wherein the GLP-1 polypeptide has at least 90% sequence identity to the amino acid sequences as set forth in SEQ ID NO: 1, SEQ ID NO: 2, or SEQ ID NO: 3, and contains one or more amino acid substitutions selected from the group consisting of A8G, G22E, and R36G relative to native human GLP-1.

**7.** The fusion protein according to any one of claims 1-6, wherein the GLP-1 polypeptide is selected from human GLP-1 (7-37), human GLP-1 (7-36), and DPP-IV resistant human GLP-1, and contains A8G and G22E substitutions relative to native human GLP-1.

**8.** The fusion protein according to claim 7, wherein the GLP-1 polypeptide is selected from human GLP-1 (7-37), human GLP-1 (7-36), and DPP-IV resistant human GLP-1, and contains A8G, G22E, and R36G substitutions relative to native human GLP-1.

**9.** The fusion protein according to claim 8, wherein the GLP-1 polypeptide is human GLP-1 (7-37), and contains A8G, G22E, and R36G substitutions relative to native human GLP-1.

**10.** The fusion protein according to any one of the preceding claims, wherein the amino acid sequence of the GLP-1 polypeptide is as set forth in SEQ ID NO: 3.

**11.** The fusion protein according to any one of claims 1-10, wherein the IgG2-Fc domain is derived from human IgG2.

**12.** The fusion protein according to any one of the preceding claims, wherein the IgG2-Fc domain has at least 90% sequence identity to the amino acid sequences as set forth in SEQ ID NO: 5 or SEQ ID NO: 6, and contains one or more amino acid substitutions selected from the group consisting of C222S, A330S, and P331S.

**13.** The fusion protein according to any one of the preceding claims, wherein the IgG2-Fc domain has at least 90% sequence identity to the amino acid sequences as set forth in SEQ ID NO: 5 or SEQ ID NO: 6, and contains A330S and P331S substitutions.

**14.** The fusion protein according to claim 13, wherein the IgG2-Fc domain further contains C222S substitution.

**15.** The fusion protein according to claim 14, wherein the amino acid sequence of the IgG2-Fc domain is as set forth in SEQ ID NO: 6.

**16.** The fusion protein according to any one of the preceding claims, wherein the amino acid sequence of the GLP-1 polypeptide is as set forth in SEQ ID NO: 3, and the amino acid sequence of the immunoglobulin Fc domain is as set forth in SEQ ID NO: 6.

**17.** The fusion protein according to any one of the preceding claims, wherein the GLP-1 polypeptide is located at N-terminus or C-terminus of the immunoglobulin Fc domain.

**18.** The fusion protein according to any one of the preceding claims, wherein the GLP-1 polypeptide is directly covalently linked to the immunoglobulin Fc domain.

**19.** The fusion protein according to any one of claims 1-17, wherein the GLP-1 polypeptide and the immunoglobulin Fc domain are covalently linked via a linker.

**20.** The fusion protein according to claim 19, wherein the linker is selected from the group consisting of a cleavable linker, a non-cleavable linker, a flexible linker, a rigid linker, a helical linker, and a non-helical linker.

**21.** The fusion protein according to claim 20, wherein the linker includes a connecting peptide.

**22.** The fusion protein according to claim 21, wherein the peptide contains glycine and serine residues.

**23.** The fusion protein according to claim 22, wherein the connecting peptide comprising glycine and serine residues includes one, two, three, four, or more repeats of SEQ ID NO: 39 (GGGS), SEQ ID NO: 40 (GGGGS), SEQ ID NO: 41 (GGGGGS), or SEQ ID NO: 42 (GGGGGGGS).

**24.** The fusion protein according to any one of claims 19-23, wherein the linker comprises an amino acid sequence selected from group consisting of SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, and SEQ ID NO: 19.

**25.** The fusion protein according to claim 24, wherein the linker comprises the amino acid sequence as set forth in SEQ ID NO: 9.

**26.** The fusion protein according to any one of claims 19-25, wherein the amino acid sequence of the GLP-1 polypeptide is as set forth in SEQ ID NO: 3, the amino acid sequence of the immunoglobulin Fc domain is as set forth in SEQ ID NO: 6, and the amino acid sequence of the linker is as set forth in SEQ ID NO: 9.

**27.** The fusion protein according to any one of the preceding claims, wherein the fusion protein has an amino acid sequence as set forth in SEQ ID NO: 7 or an amino acid sequence with at least 80% sequence identity to SEQ ID NO: 7.

**28.** The fusion protein according to any one of the preceding claims, wherein the IgG2-Fc domain has a certain level of oxidation at No. 253 residue of methionine (M253) corresponding to SEQ ID NO: 7.

**29.** The fusion protein according to claim 28, wherein the oxidation level at the M253 residue corresponding to SEQ ID NO: 7 is less than or equal to 5%, whose residue is in the IgG2-Fc domain.

**30.** The fusion protein according to any one of the preceding claims, further comprising a signal peptide.

**31.** The fusion protein according to claim 30, wherein the signal peptide is the human CD33 signal peptide.

**32.** The fusion protein according to claim 30 or 31, wherein the signal peptide has an amino acid sequence as set forth in SEQ ID NO: 4 or an amino acid sequence with at least 90% sequence identity to SEQ ID NO: 4.

**33.** The fusion protein according to any one of claims 30-32, wherein the fusion protein has an amino acid sequence as set forth in SEQ ID NO: 8 or an amino acid sequence with at least 80% sequence identity to SEQ ID NO: 8.

**34.** The fusion protein according to any one of the preceding claims, wherein the fusion protein has a half-life in a subject's

body of at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, or at least 14 days.

**35.** A dimer comprising two identical peptide chains connected by a disulfide bond, wherein each peptide chain comprises the fusion protein according to any one of claims 1-34.

**36.** A nucleic acid molecule comprising a nucleotide sequence encoding the fusion protein according to any one of claims 1-34.

**37.** The nucleic acid molecule according to claim 36, comprising a nucleotide sequence as set forth in SEQ ID NO: 26 or SEQ ID NO: 27, or a nucleotide sequence with at least 70% sequence identity to SEQ ID NO: 26 or SEQ ID NO: 27.

**38.** A vector comprising the nucleic acid molecule according to claim 36 or 37.

**39.** A cell comprising a nucleic acid molecule according to claim 36 or 37, or a vector according to claim 38.

**40.** The cell according to claim 39, further recombinantly expressing or naturally expressing lysine hydroxylase.

**41.** The cell according to claim 40, wherein the expression level or activity of lysine hydroxylase expressed in the said cells are higher than the level or activity of lysine hydroxylase in COS-7 cells.

**42.** The cell according to any one of claims 39-41, wherein the cell is a prokaryotic cell or a eukaryotic cell.

**43.** The cell according to claim 42, wherein the eukaryotic cell is a mammalian cell.

**44.** The cell according to claim 43, wherein the mammalian cell is a human cell or Chinese hamster ovary (CHO) cell.

**45.** The cell according to claim 43, wherein the mammalian cell is human embryonic kidney cells 293 (HEK293), or CHO-K1 cells, or CHO-S cells, or CHO-DG44 cells.

**46.** A composition comprising the fusion protein according to any one of claims 1-34, or the dimer according to claim 35, or the nucleic acid molecule according to claim 36 or 37, or the vector according to claim 38, or the cell according to any one of claims 39-45.

**47.** The composition according to claim 46, wherein in the fusion protein, greater than or equal to 10%, or greater than or equal to 15%, or at least greater than or equal to 20%, or greater than or equal to 26%, or greater than or equal to 30%, or greater than or equal to 40%, or greater than or equal to 50%, or greater than or equal to 60%, or greater than or equal to 70%, or greater than or equal to 80%, or greater than or equal to 90% of the GLP-1 polypeptide in the fusion protein is hydroxylated at position K34 relative to native human GLP-1.

**48.** A method for constructing a cell according to any one of claims 39-45, comprising:

a) introducing a nucleic acid molecule encoding the fusion protein into a vector to construct an expression vector;
b) introducing the expression vector into a cell that recombinantly expresses or naturally expresses lysine hydroxylase to obtain a recombinant cell; preferably, the lysine hydroxylase level or activity expressed by the recombinant cell is higher than the level or activity of lysine hydroxylase expressed by COS-7 cells; more preferably, the cell is a CHO cell, particularly CHO-K1 cells; more preferably, the vector is a pKN012 vector.

**49.** A method for constructing a recombinant cell, comprising:

a) inserting the nucleotide sequence as set forth in SEQ ID NO: 26 into the NcoI and HindIII sites of a pKN012 vector to generate a pKN012-GLP1-IgG2/Fc expression vector;
b) introducing the pKN012-GLP1-IgG2/Fc expression vector into a CHO-K1 cell to obtain the recombinant cell.

**50.** A method for producing a fusion protein, comprising the step of using the cell according to any of claims 39-45 or the cell obtained by the construction method according to claim 48 or 49 to produce the fusion protein.

**51.** The method according to claim 50, further comprising the step of detecting the hydroxylation level of the fusion protein

at position K34 relative to native human GLP-1.

**52.** A method for detecting the quality of a fusion protein, wherein the fusion protein comprises a GLP-1 polypeptide and an immunoglobulin IgG2-Fc domain, the method comprises the following step: detecting the hydroxylation level of the fusion protein at position K34 relative to native human GLP-1.

**53.** The method according to claim 52, wherein the GLP-1 polypeptide is selected from human GLP-1 (7-37), human GLP-1 (7-36), and DPP-IV resistant human GLP-1, and the GLP-1 polypeptide contains one or more amino acid substitutions selected from the following group consisting of A8G, G22E, and R36G relative to native human GLP-1; and/or the IgG2-Fc domain contains one or more amino acid substitutions selected from the group consisting of C222S, A330S, and P331S.

**54.** The method according to claim 52 or 53, wherein when the hydroxylation level of the fusion protein at position K34 relative to native human GLP-1 is between 10% and 100% (e.g., greater than or equal to 10%, or greater than or equal to 15%, or greater than or equal to 20%, or greater than or equal to 26%, or greater than or equal to 30%, or greater than or equal to 40%, or greater than or equal to 50%, or greater than or equal to 60%, or greater than or equal to 70%, or greater than or equal to 80%, or greater than or equal to 90%), the quality of the fusion protein is confirmed as qualified.

**55.** Use of the fusion protein according to any one of claims 1-34, or the dimer according to claim 35, or the nucleic acid molecule according to claim 36 or 37, or the vector according to claim 38, or the cell according to any one of claims 39-45, or the composition according to claim 46 or 47, in the manufacture of a medicament for the treatment or prevention of diseases.

**56.** The use according to claim 55, wherein the disease is selected from the group consisting of metabolic diseases associated with glucose or lipid metabolism disorders, complications of metabolic diseases, and neurological disorders.

**57.** The use according to claim 56, wherein the metabolic diseases associated with glucose or lipid metabolism disorders are selected from the group consisting of diabetes, non-alcoholic steatohepatitis (NASH), non-alcoholic fatty liver disease (NAFLD), obesity, and metabolic syndrome.

**58.** The use according to claim 56 or 57, wherein the metabolic disease associated with glucose or lipid metabolism disorders is diabetes (e.g., type 2 diabetes).

**59.** The use according to claim 56, wherein the complications of metabolic diseases include cardiovascular complications, renal complications, or hepatic complications caused by metabolic diseases.

**60.** The use according to claim 56, wherein the neurological disorder is a neurodegenerative disease.

**61.** The use according to claim 60, wherein the neurodegenerative disease is selected from the group consisting of Alzheimer's disease, motor neuron disease, Huntington's disease, and Parkinson's disease.

# FIG. 1

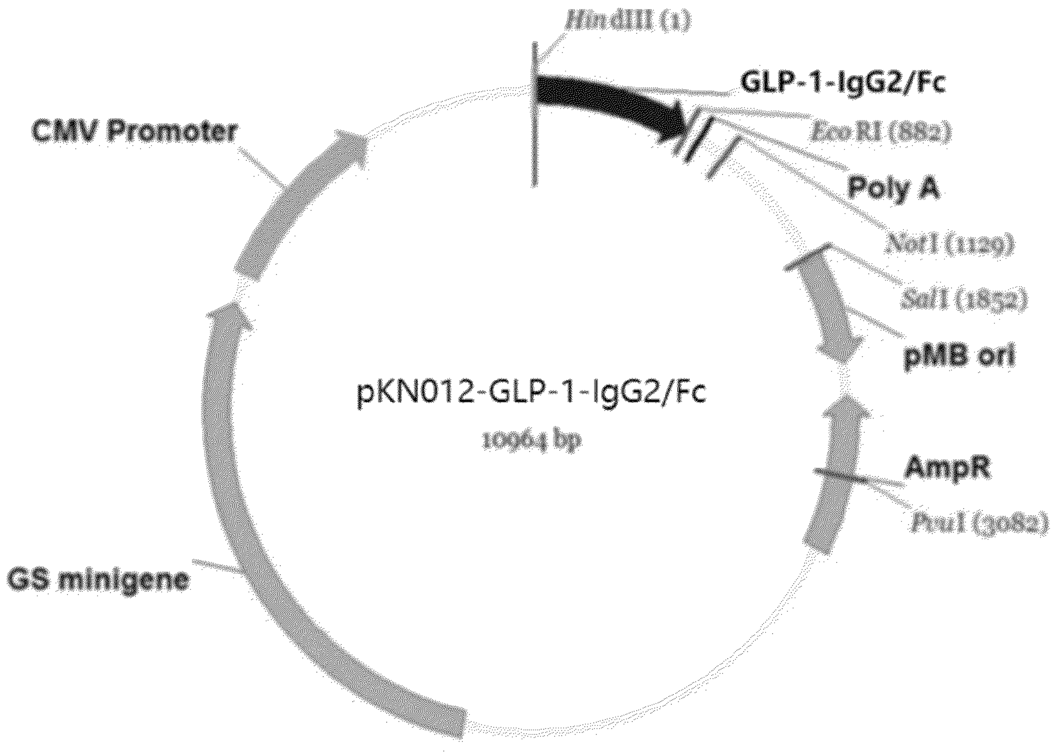
HindIII (1)
GLP-1-IgG2/Fc
EcoRI (882)
Poly A
NotI (1129)
SalI (1852)
pMB ori
AmpR
PvuI (3082)
CMV Promoter
GS minigene
pKN012-GLP-1-IgG2/Fc
10964 bp

FIG. 2

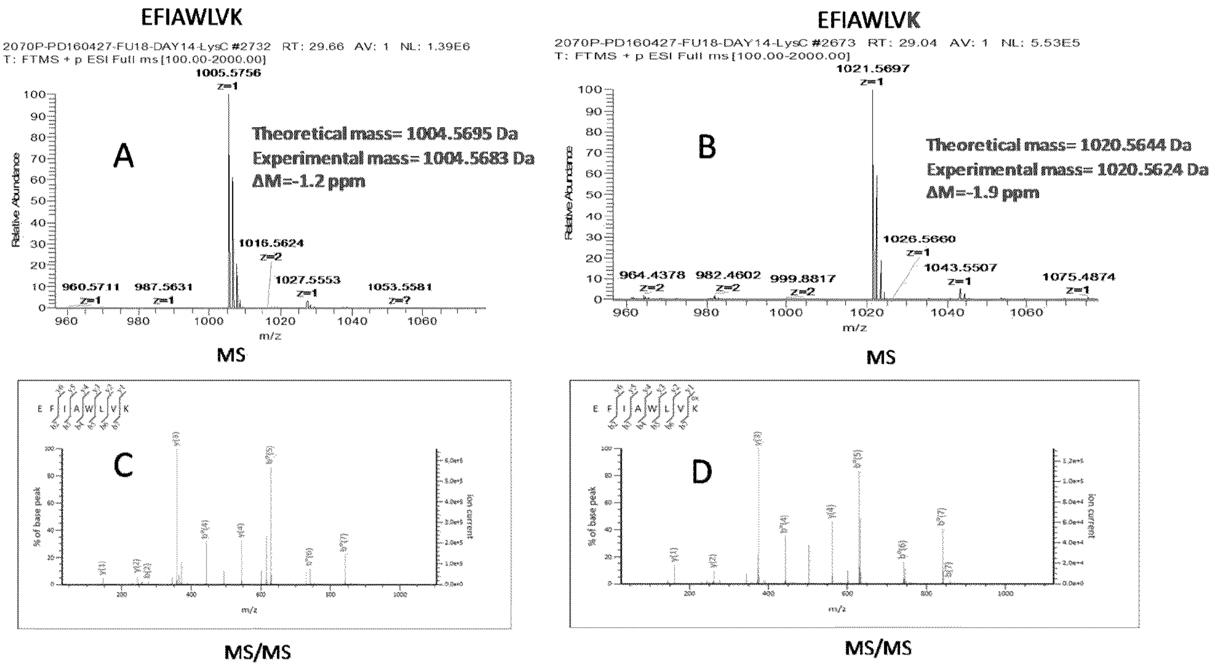
EFIAWLVK
2070P-PD160427-FU18-DAY14-LysC #2732 RT: 29.66 AV: 1 NL: 1.39E6
T: FTMS + p ESI Full ms [100.00-2000.00]
A
Theoretical mass= 1004.5695 Da
Experimental mass= 1004.5683 Da
ΔM=-1.2 ppm
EFIAWLVK
2070P-PD160427-FU18-DAY14-LysC #2673 RT: 29.04 AV: 1 NL: 5.53E5
T: FTMS + p ESI Full ms [100.00-2000.00]
B
Theoretical mass= 1020.5644 Da
Experimental mass= 1020.5624 Da
ΔM=-1.9 ppm
MS
MS
C
D
MS/MS
MS/MS

# FIG. 3A

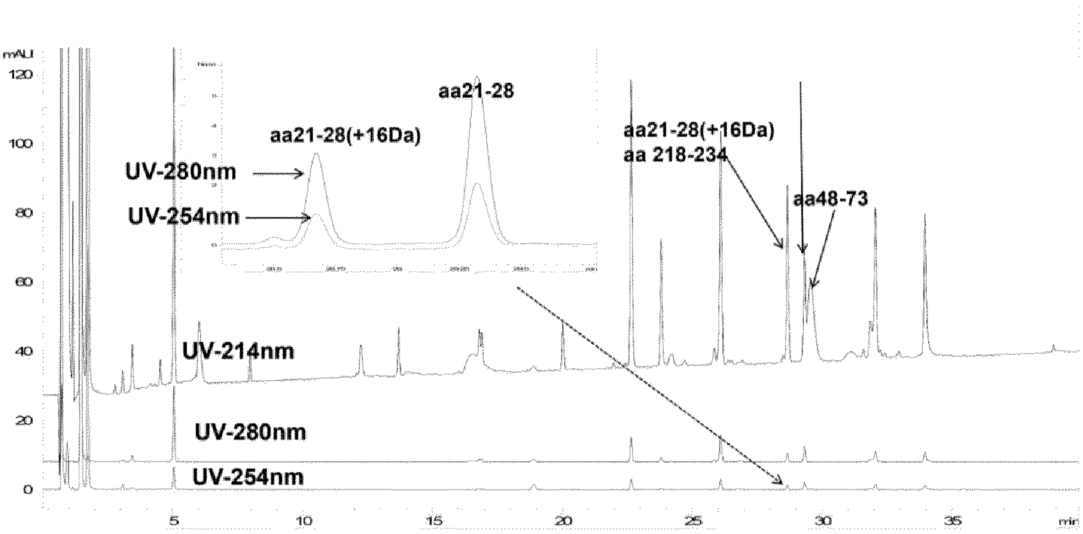


$\varepsilon_W = 5500$

$\varepsilon_Y = 1490$

| | |
|---|---|
| 21-28 | EFIA**W**LVK |
| 218-234 | TTPPMLDSDGSFFL**Y**SK |
| 48-73 | SCVECPPCPAPPVAGPSVFLFPPKPK |

FIG. 3B

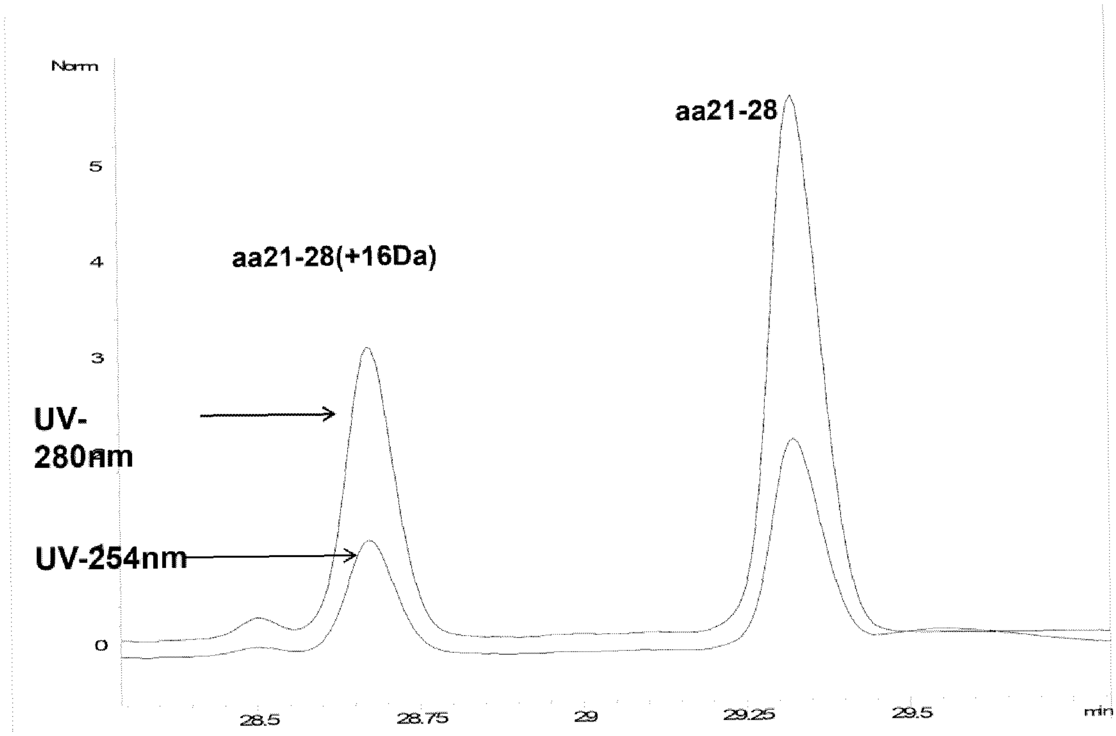
Norm
aa21-28
5
4
aa21-28(+16Da)
3
UV-
280nm
UV-254nm
0
28.5
28.75
29
29.25
29.5
min

FIG. 4

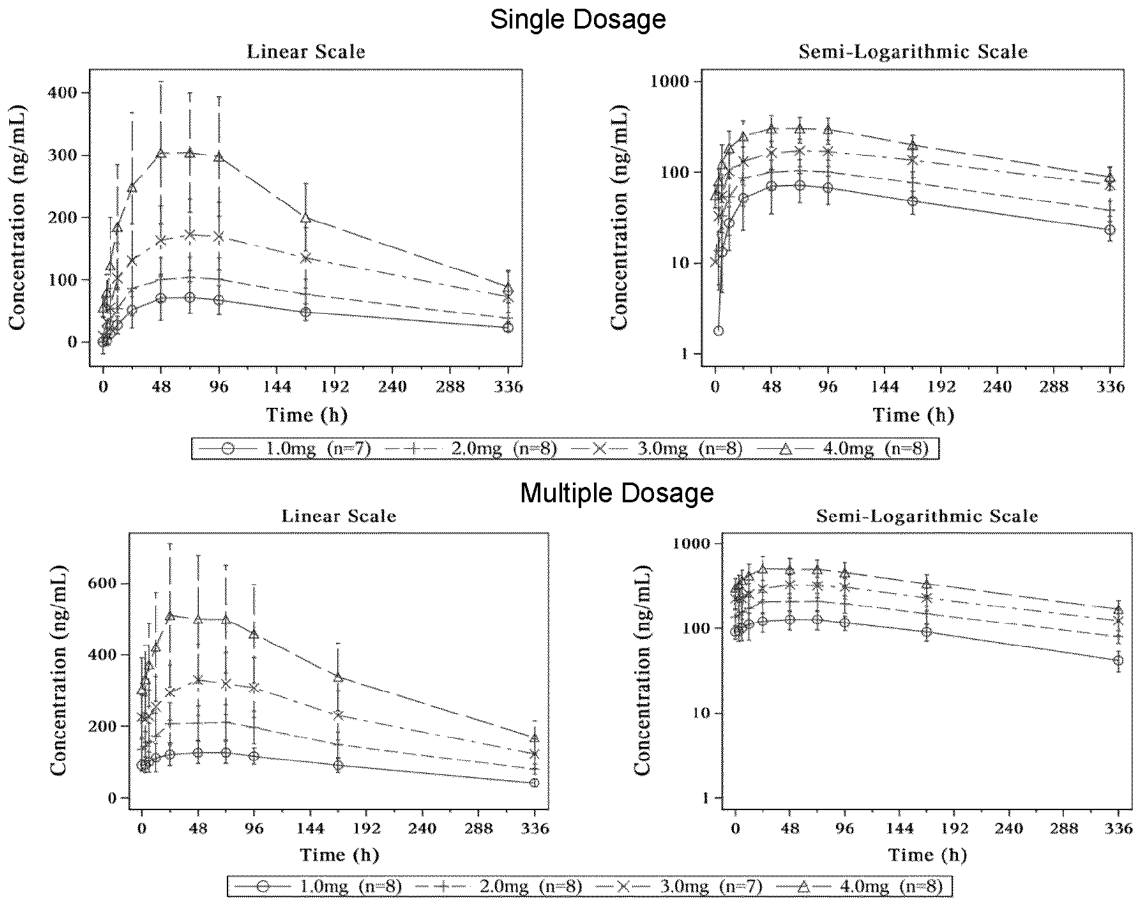
Single Dosage
Linear Scale
Semi-Logarithmic Scale
Concentration (ng/mL)
Time (h)
1.0mg (n=7) 2.0mg (n=8) 3.0mg (n=8) 4.0mg (n=8)
Multiple Dosage
Linear Scale
Semi-Logarithmic Scale
Concentration (ng/mL)
Time (h)
1.0mg (n=8) 2.0mg (n=8) 3.0mg (n=7) 4.0mg (n=8)

# FIG. 5

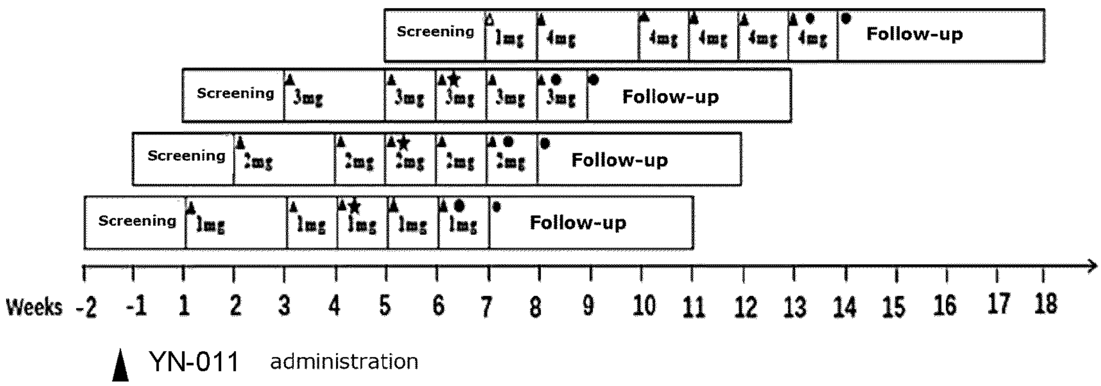
Screening
1mg
4mg
4mg
4mg
4mg
4mg
Follow-up
Screening
3mg
3mg
3mg
3mg
3mg
Follow-up
Screening
2mg
2mg
2mg
2mg
2mg
Follow-up
Screening
1mg
1mg
1mg
1mg
1mg
Follow-up
Weeks -2 -1 1 2 3 4 5 6 7 8 9 10 11 12 13 14 15 16 17 18
YN-011 administration

FIG. 6

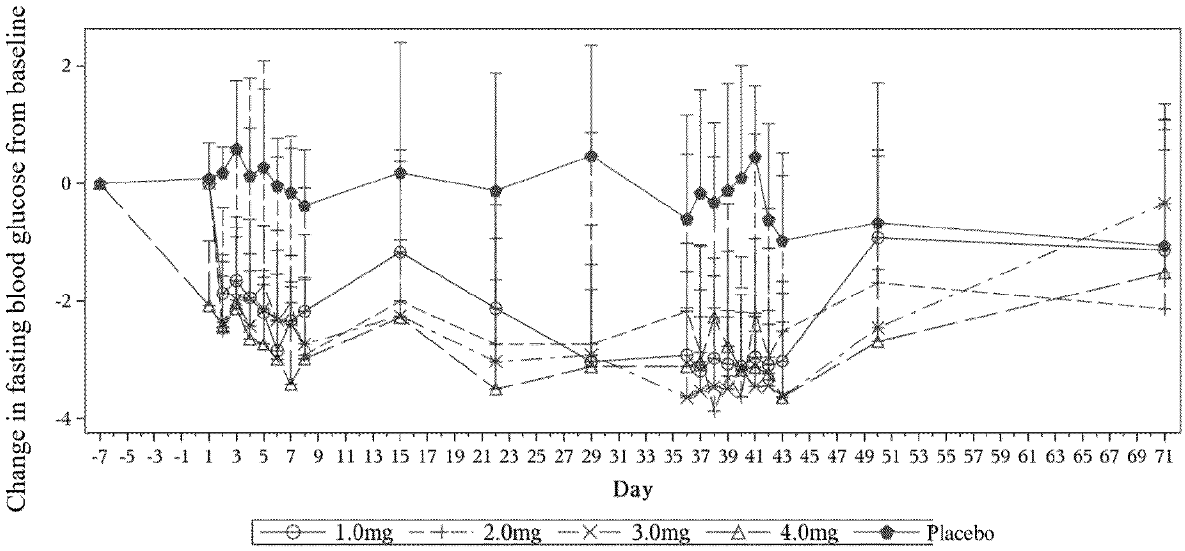
Change in fasting blood glucose from baseline
2
0
-2
-4
-7 -5 -3 -1 1 3 5 7 9 11 13 15 17 19 21 23 25 27 29 31 33 35 37 39 41 43 45 47 49 51 53 55 57 59 61 63 65 67 69 71
Day
1.0mg
2.0mg
3.0mg
4.0mg
Placebo

FIG. 7

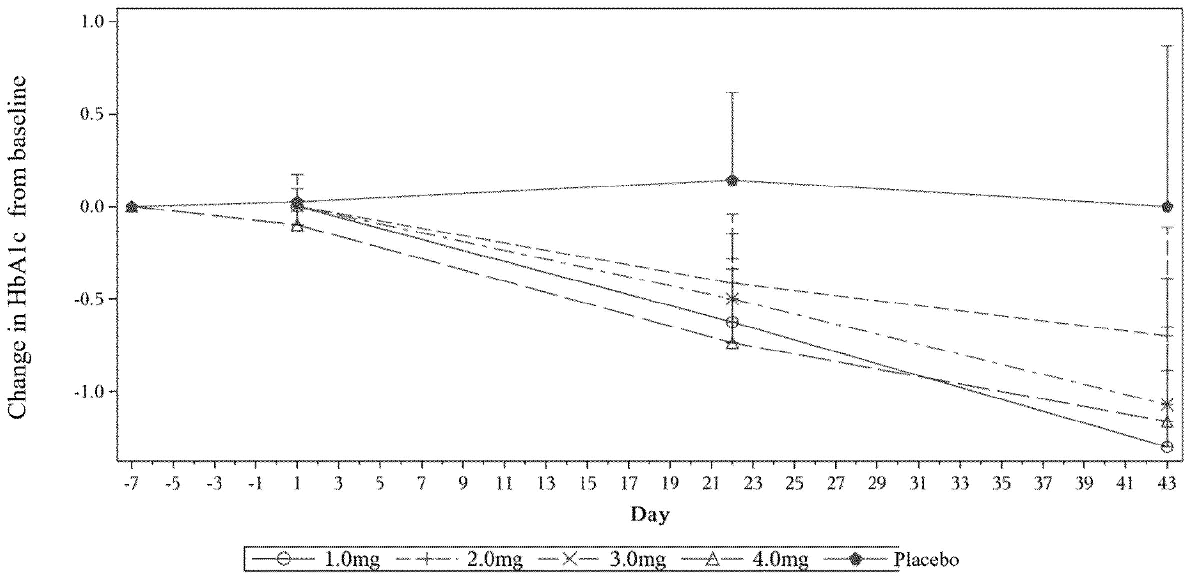
1.0
0.5
0.0
-0.5
-1.0
Change in HbA1c from baseline
-7 -5 -3 -1 1 3 5 7 9 11 13 15 17 19 21 23 25 27 29 31 33 35 37 39 41 43
Day
1.0mg
2.0mg
3.0mg
4.0mg
Placebo

FIG. 8

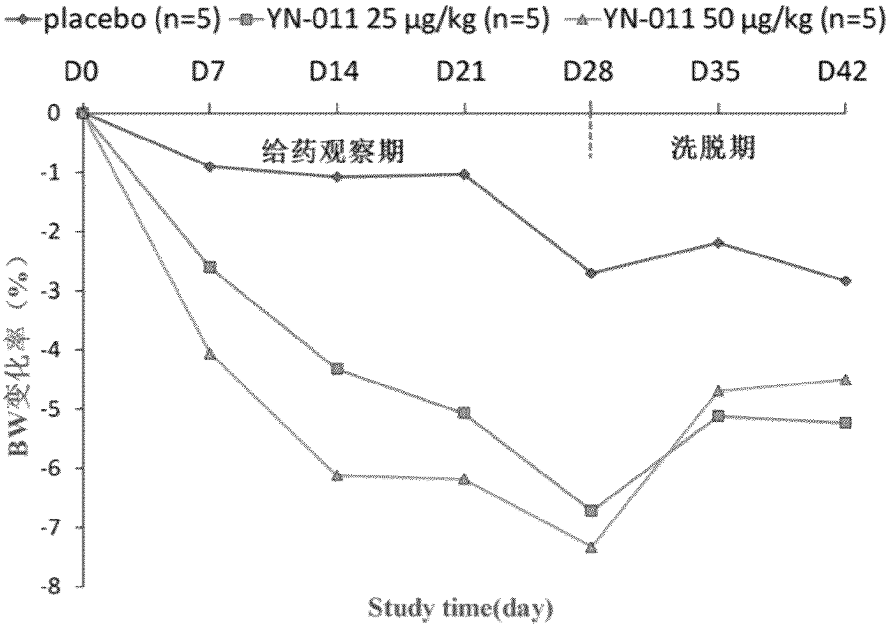
placebo (n=5)
YN-011 25 μg/kg (n=5)
YN-011 50 μg/kg (n=5)
D0
D7
D14
D21
D28
D35
D42
0
-1
-2
-3
-4
-5
-6
-7
-8
给药观察期
洗脱期
BW变化率（%）
Study time(day)

# FIG. 9

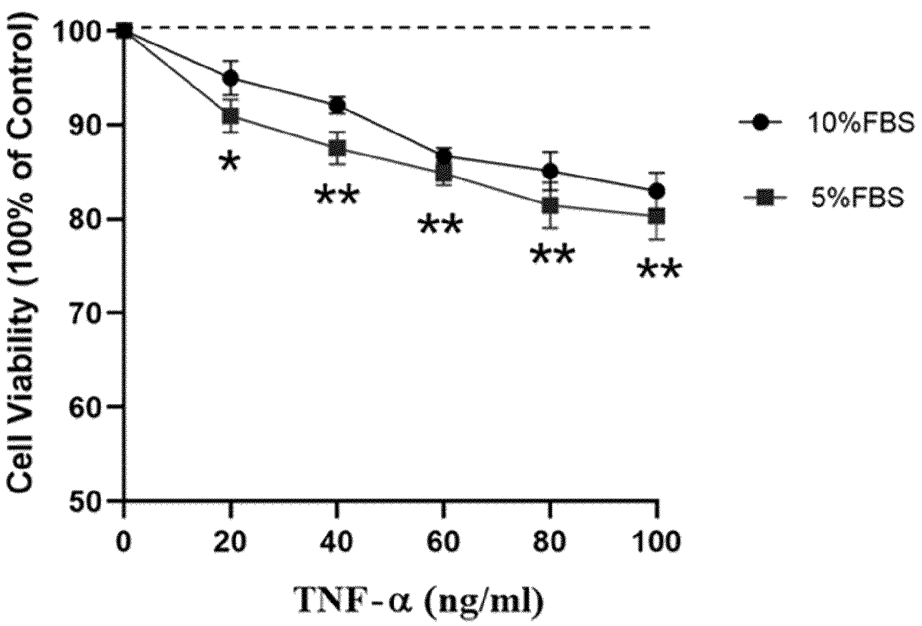
Cell Viability (100% of Control)
100
90
80
70
60
50
0
20
40
60
80
100
TNF-α (ng/ml)
*
**
**
**
**
10%FBS
5%FBS

# FIG. 10

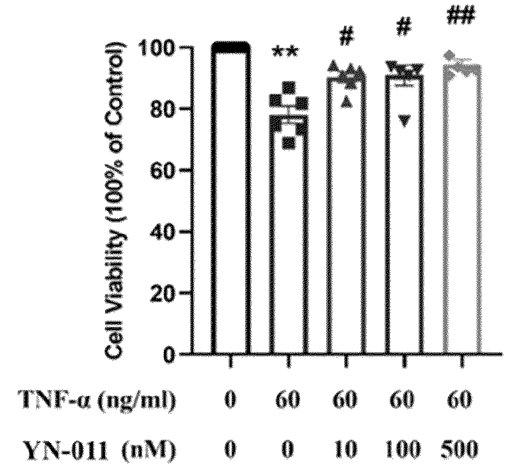
Cell Viability (100% of Control)
100
80
60
40
20
0
**
#
#
##
TNF-α (ng/ml) 0 60 60 60 60
YN-011 (nM) 0 0 10 100 500

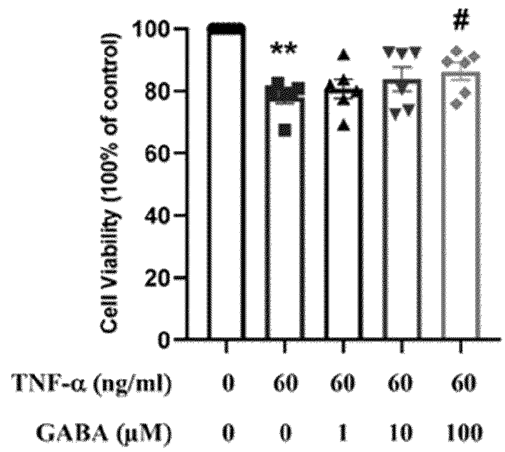
Cell Viability (100% of control)
100
80
60
40
20
0
**
#
TNF-α (ng/ml) 0 60 60 60 60
GABA (μM) 0 0 1 10 100

# FIG. 11

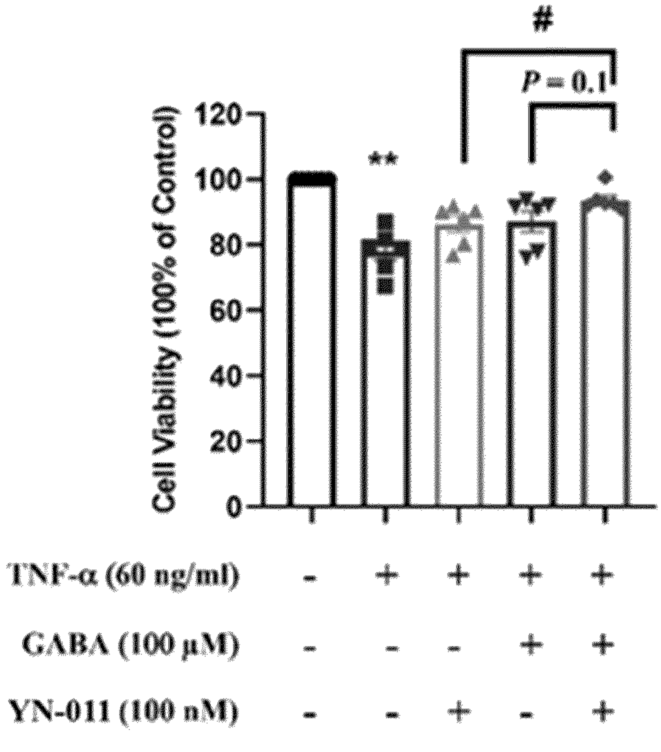
#
P = 0.1
**
Cell Viability (100% of Control)
120
100
80
60
40
20
0
TNF-α (60 ng/ml) - + + + +
GABA (100 μM) - - - + +
YN-011 (100 nM) - - + - +

# FIG. 12

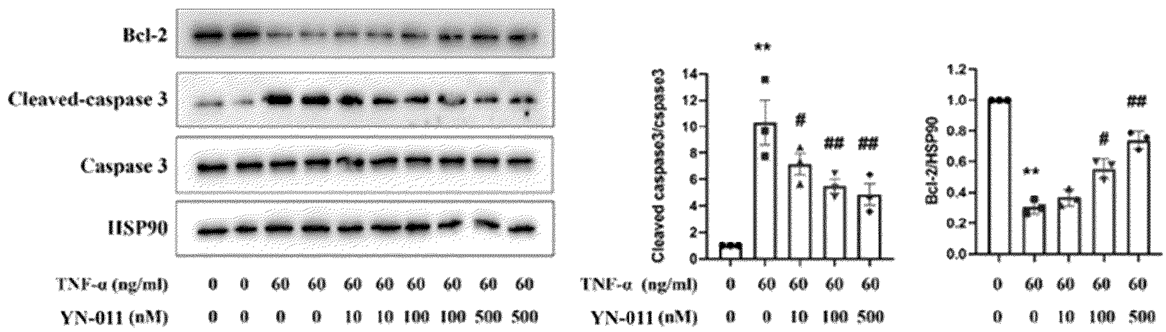
Bcl-2
Cleaved-caspase 3
Caspase 3
HSP90
TNF-α (ng/ml) 0 0 60 60 60 60 60 60 60 60
YN-011 (nM) 0 0 0 0 10 10 100 100 500 500
Cleaved caspase3/cspase3
TNF-α (ng/ml) 0 60 60 60 60
YN-011 (nM) 0 0 10 100 500
Bcl-2/HSP90
0 60 60 60 60
0 0 10 100 500

# FIG. 13

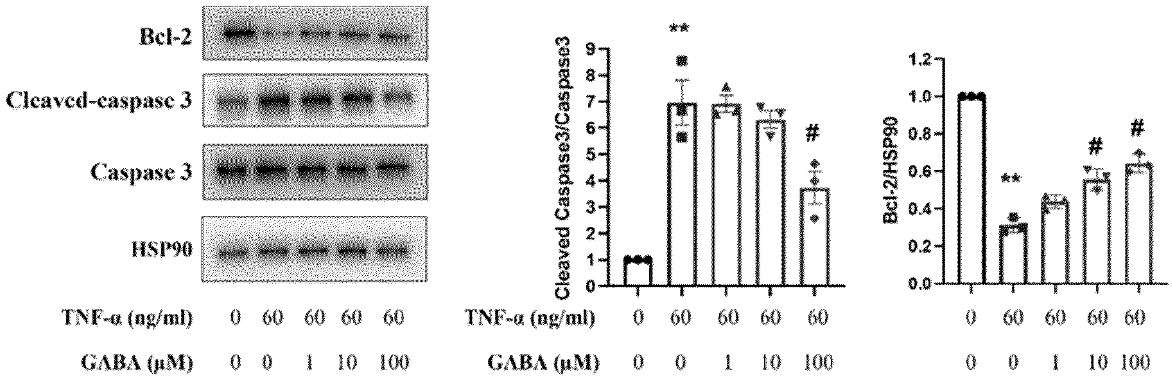
Bcl-2
Cleaved-caspase 3
Caspase 3
HSP90
TNF-α (ng/ml) 0 60 60 60 60
GABA (μM) 0 0 1 10 100
Cleaved Caspase3/Caspase3
**
#
TNF-α (ng/ml) 0 60 60 60 60
GABA (μM) 0 0 1 10 100
Bcl-2/HSP90
**
#
#
0 60 60 60 60
0 0 1 10 100

FIG. 14

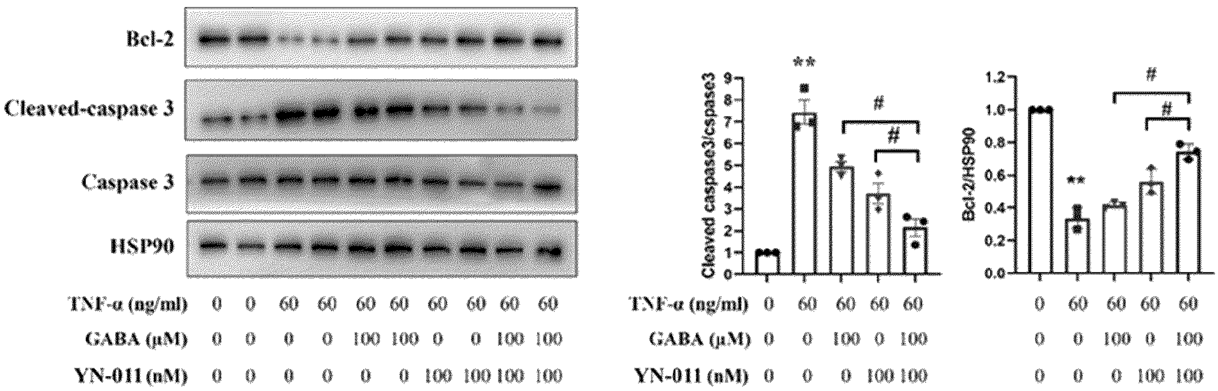
Bcl-2
Cleaved-caspase 3
Caspase 3
HSP90
TNF-α (ng/ml) 0 0 60 60 60 60 60 60 60 60
GABA (μM) 0 0 0 0 100 100 0 0 100 100
YN-011 (nM) 0 0 0 0 0 0 100 100 100 100
Cleaved caspase3/cspase3
**
#
#
TNF-α (ng/ml) 0 60 60 60 60
GABA (μM) 0 0 100 0 100
YN-011 (nM) 0 0 0 100 100
Bcl-2/HSP90
**
#
#
0 60 60 60 60
0 0 100 0 100
0 0 0 100 100

# FIG. 15

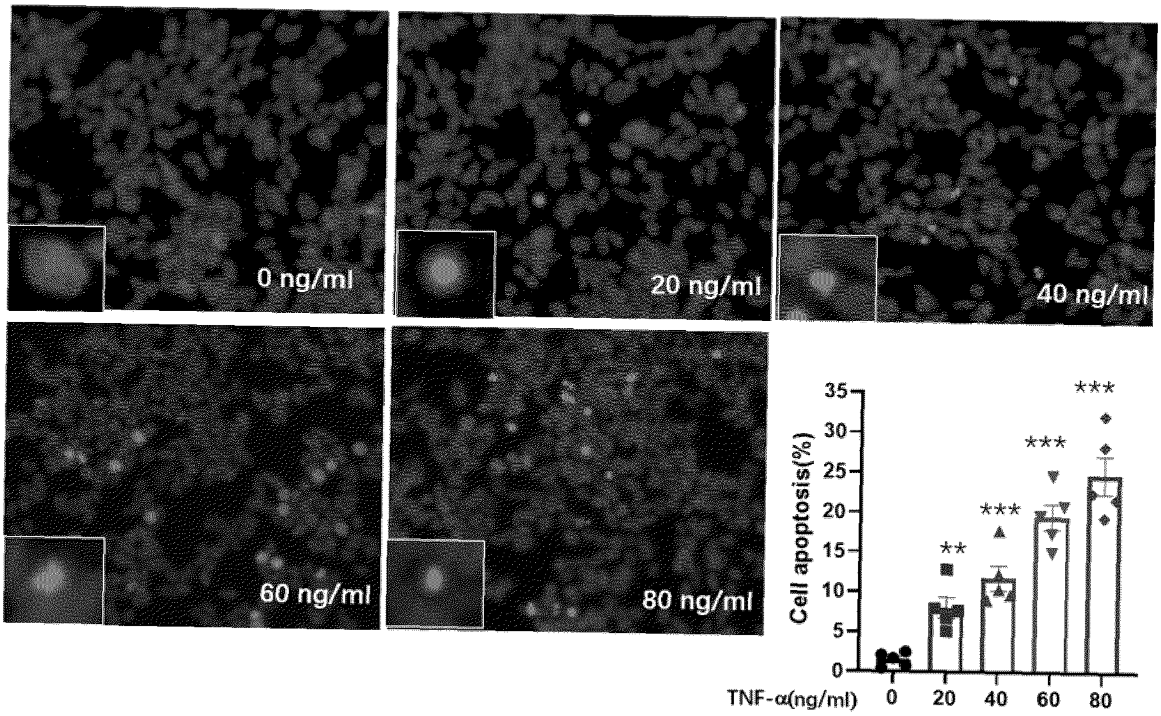
0 ng/ml
20 ng/ml
40 ng/ml
60 ng/ml
80 ng/ml
Cell apoptosis(%)
35
30
25
20
15
10
5
0
**
***
***
***
TNF-α(ng/ml) 0 20 40 60 80

FIG. 16

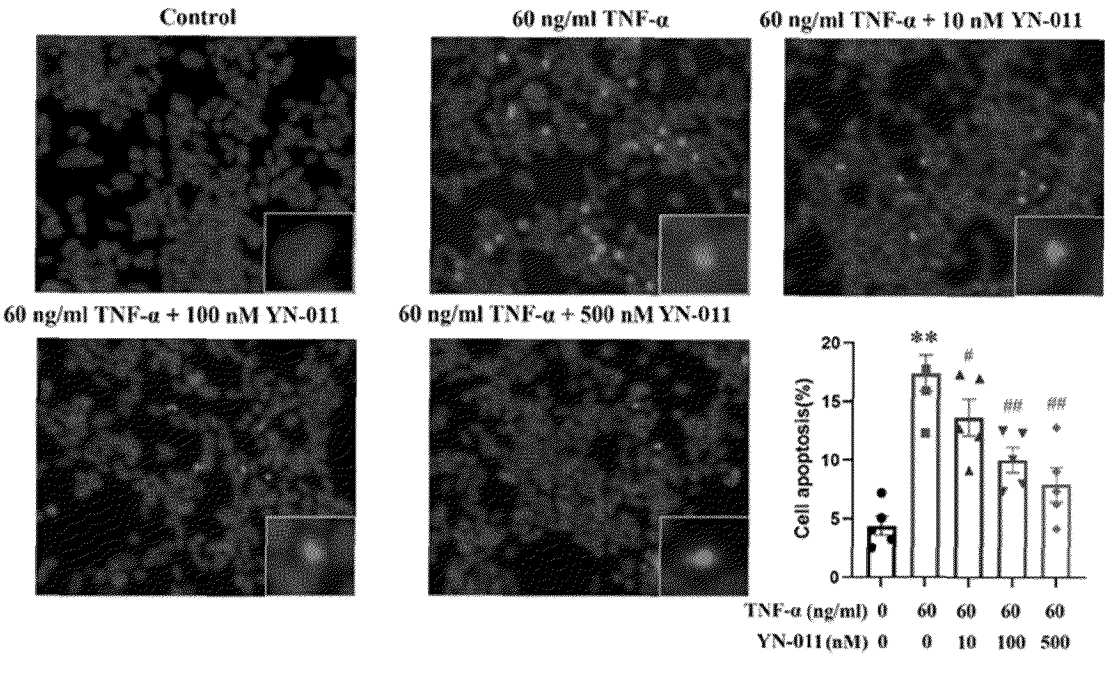
Control
60 ng/ml TNF-α
60 ng/ml TNF-α + 10 nM YN-011
60 ng/ml TNF-α + 100 nM YN-011
60 ng/ml TNF-α + 500 nM YN-011
Cell apoptosis(%)
20
15
10
5
0
**
#
##
##
TNF-α (ng/ml) 0 60 60 60 60
YN-011 (nM) 0 0 10 100 500

# FIG. 17

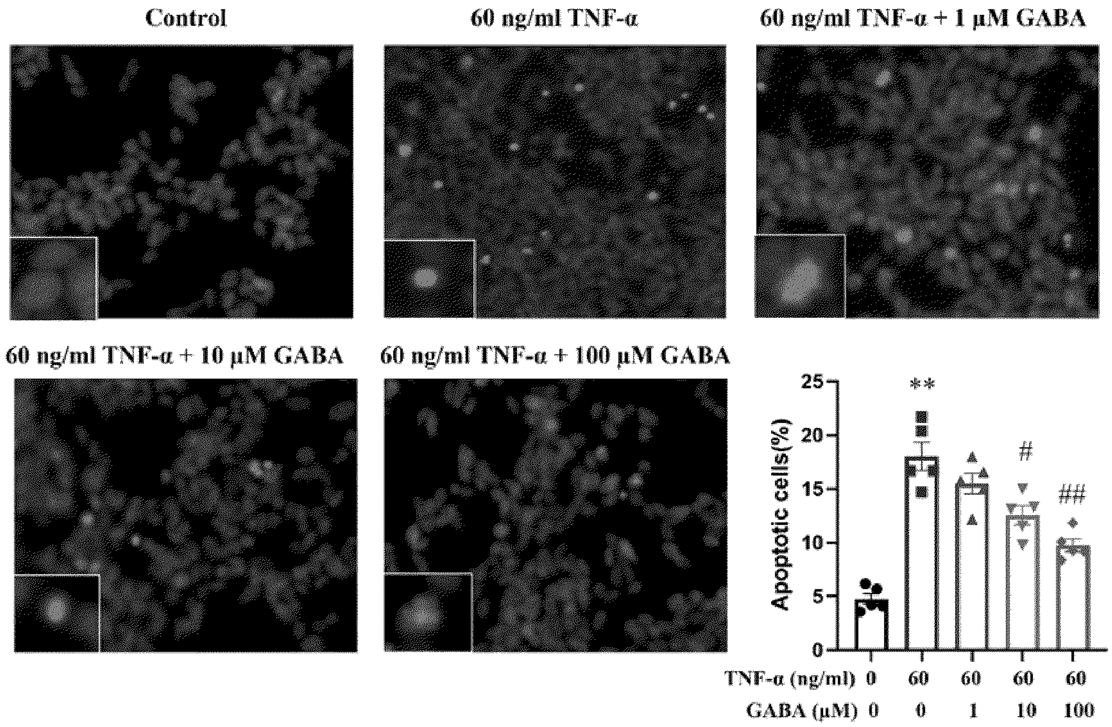
Control
60 ng/ml TNF-α
60 ng/ml TNF-α + 1 µM GABA
60 ng/ml TNF-α + 10 µM GABA
60 ng/ml TNF-α + 100 µM GABA
Apoptotic cells(%)
25
20
15
10
5
0
**
#
##
TNF-α (ng/ml) 0 60 60 60 60
GABA (µM) 0 0 1 10 100

# FIG. 18

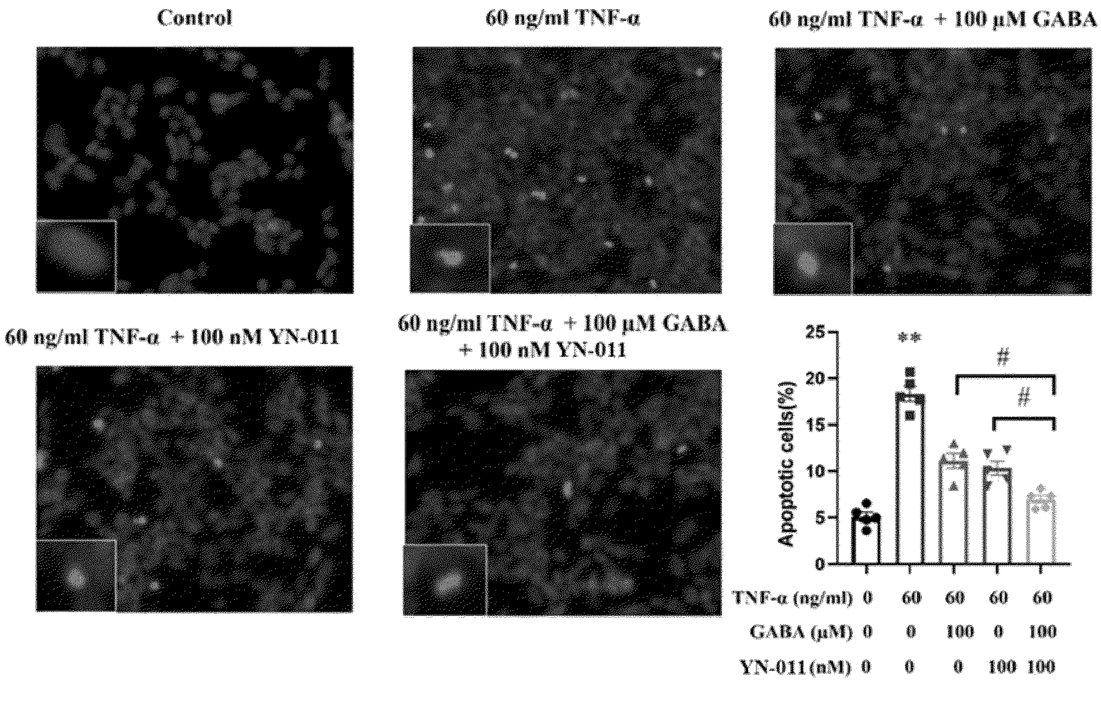
Control
60 ng/ml TNF-α
60 ng/ml TNF-α + 100 μM GABA
60 ng/ml TNF-α + 100 nM YN-011
60 ng/ml TNF-α + 100 μM GABA + 100 nM YN-011
Apoptotic cells(%)
**
#
#
TNF-α (ng/ml) 0 60 60 60 60
GABA (μM) 0 0 100 0 100
YN-011 (nM) 0 0 0 100 100

# FIG. 19

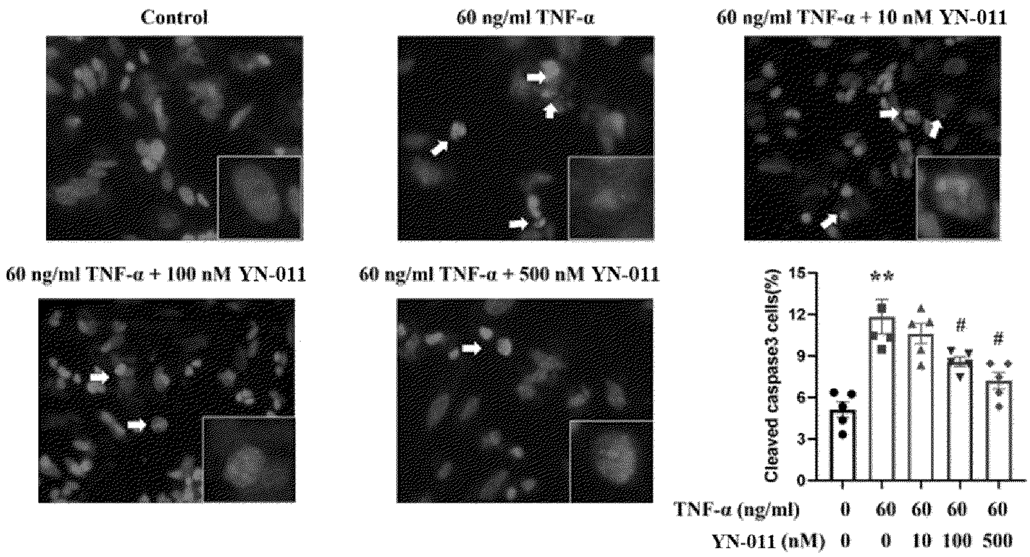
Control
60 ng/ml TNF-α
60 ng/ml TNF-α + 10 nM YN-011
60 ng/ml TNF-α + 100 nM YN-011
60 ng/ml TNF-α + 500 nM YN-011
Cleaved caspase3 cells(%)
15
12
9
6
3
0
**
#
#
TNF-α (ng/ml) 0 60 60 60 60
YN-011 (nM) 0 0 10 100 500

# FIG. 20

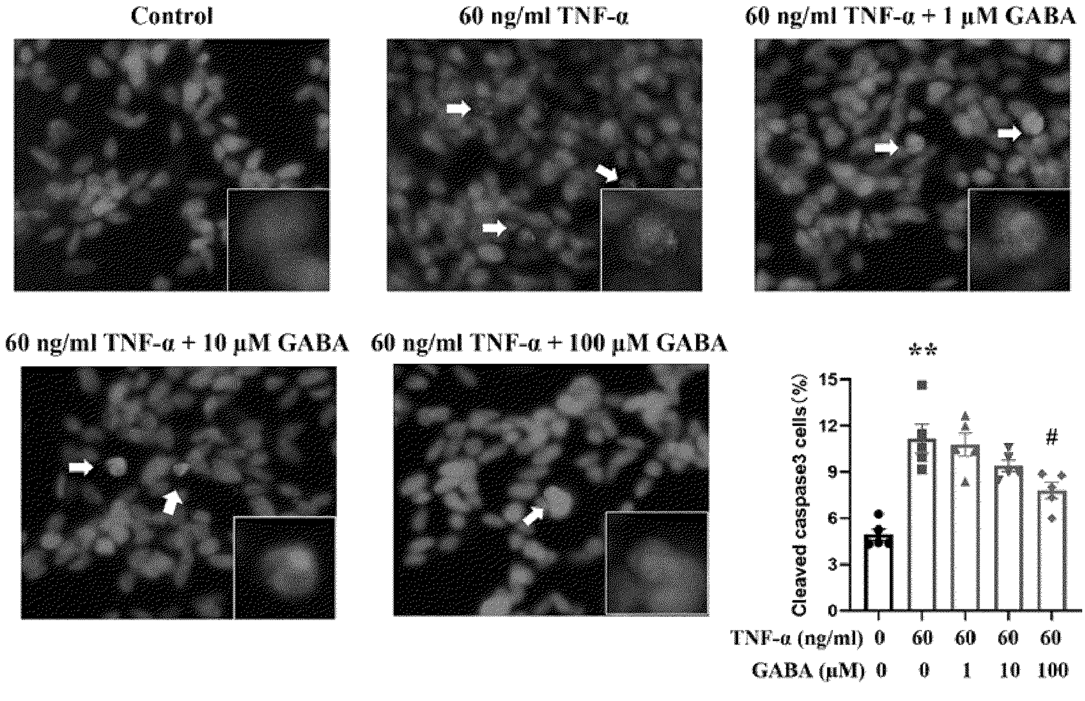
Control
60 ng/ml TNF-α
60 ng/ml TNF-α + 1 μM GABA
60 ng/ml TNF-α + 10 μM GABA
60 ng/ml TNF-α + 100 μM GABA
**
#
Cleaved caspase3 cells (%)
15
12
9
6
3
0
TNF-α (ng/ml) 0 60 60 60 60
GABA (μM) 0 0 1 10 100

# FIG. 21

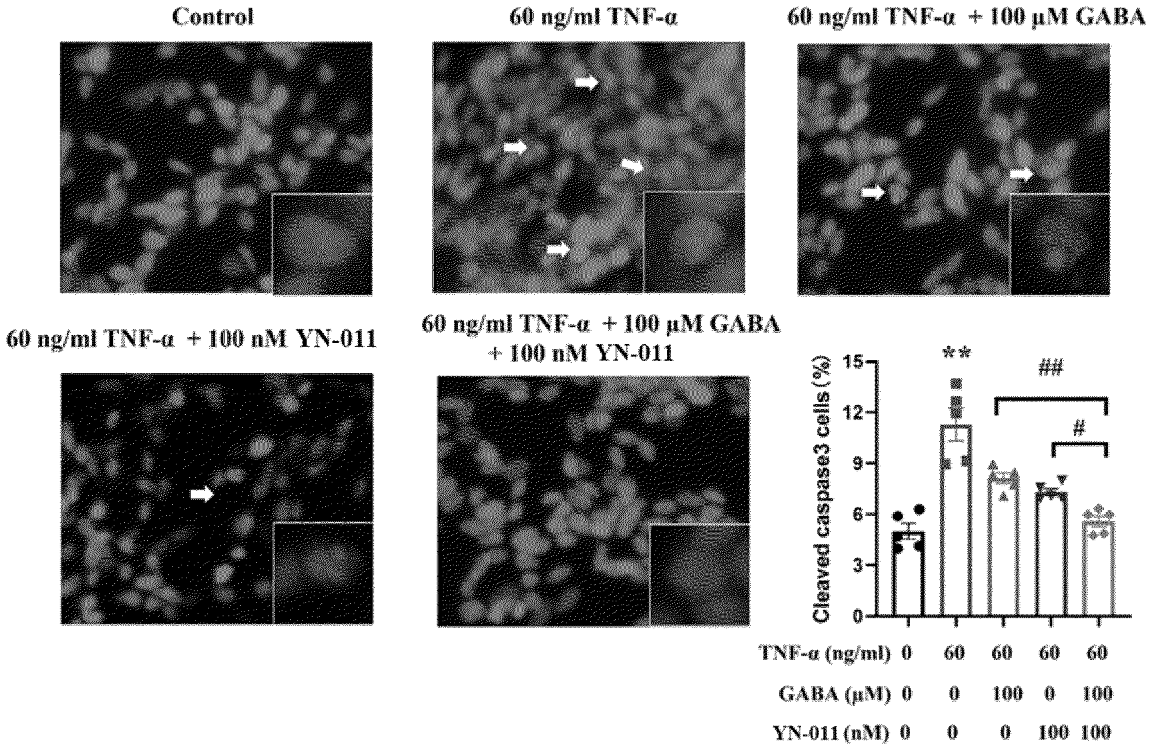
Control
60 ng/ml TNF-α
60 ng/ml TNF-α + 100 μM GABA
60 ng/ml TNF-α + 100 nM YN-011
60 ng/ml TNF-α + 100 μM GABA + 100 nM YN-011
Cleaved caspase3 cells (%)
15
12
9
6
3
0
**
##
#
TNF-α (ng/ml) 0 60 60 60 60
GABA (μM) 0 0 100 0 100
YN-011 (nM) 0 0 0 100 100

# FIG. 22

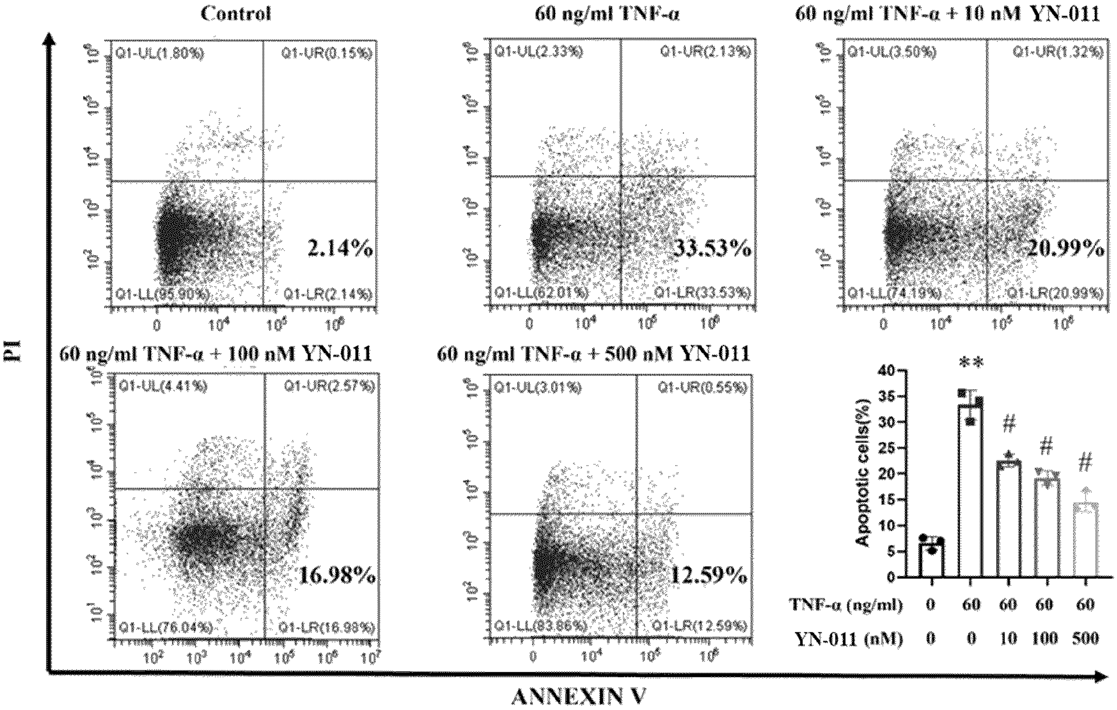
Control
60 ng/ml TNF-α
60 ng/ml TNF-α + 10 nM YN-011
60 ng/ml TNF-α + 100 nM YN-011
60 ng/ml TNF-α + 500 nM YN-011
Q1-UL(1.80%)
Q1-UR(0.15%)
Q1-LL(95.90%)
Q1-LR(2.14%)
2.14%
Q1-UL(2.33%)
Q1-UR(2.13%)
Q1-LL(62.01%)
Q1-LR(33.53%)
33.53%
Q1-UL(3.50%)
Q1-UR(1.32%)
Q1-LL(74.19%)
Q1-LR(20.99%)
20.99%
Q1-UL(4.41%)
Q1-UR(2.57%)
Q1-LL(76.04%)
Q1-LR(16.98%)
16.98%
Q1-UL(3.01%)
Q1-UR(0.55%)
Q1-LL(83.86%)
Q1-LR(12.59%)
12.59%
PI
ANNEXIN V
Apoptotic cells(%)
**
#
TNF-α (ng/ml) 0 60 60 60 60
YN-011 (nM) 0 0 10 100 500

# FIG. 23

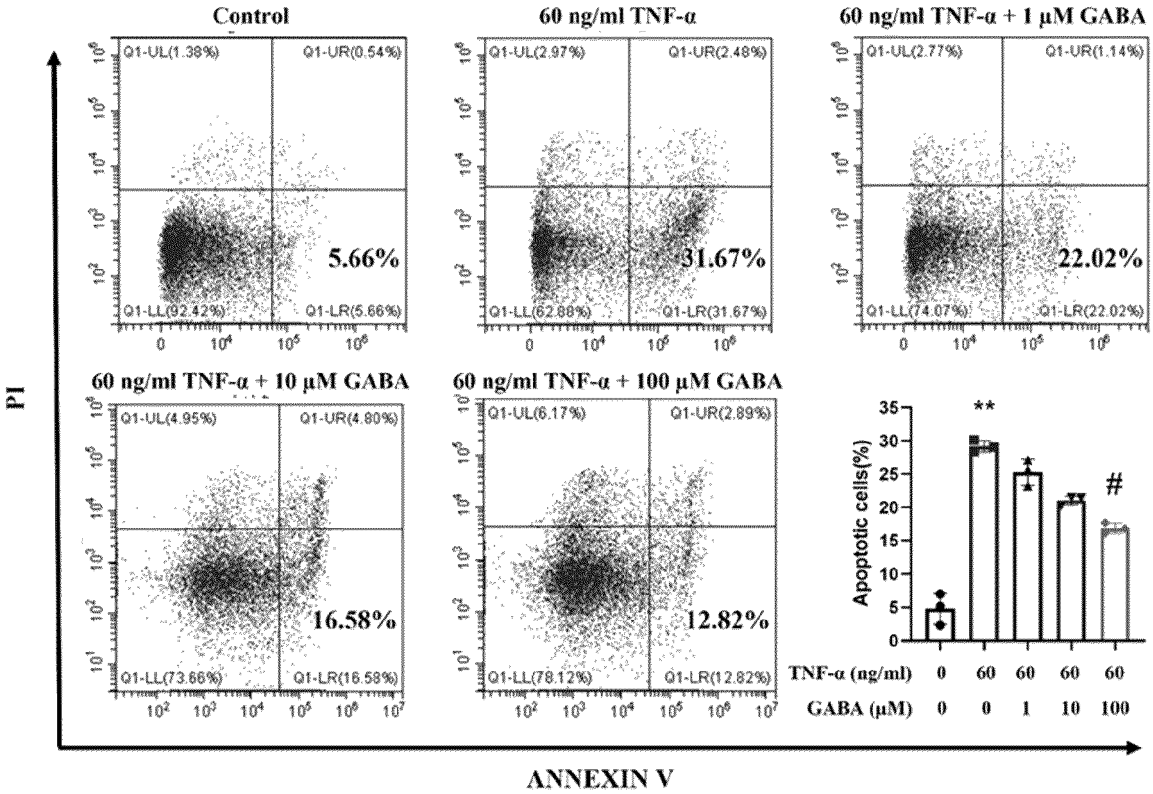
Control
60 ng/ml TNF-α
60 ng/ml TNF-α + 1 μM GABA
60 ng/ml TNF-α + 10 μM GABA
60 ng/ml TNF-α + 100 μM GABA
Q1-UL(1.38%)
Q1-UR(0.54%)
Q1-LL(92.42%)
Q1-LR(5.66%)
5.66%
Q1-UL(2.97%)
Q1-UR(2.48%)
Q1-LL(62.88%)
Q1-LR(31.67%)
31.67%
Q1-UL(2.77%)
Q1-UR(1.14%)
Q1-LL(74.07%)
Q1-LR(22.02%)
22.02%
Q1-UL(4.95%)
Q1-UR(4.80%)
Q1-LL(73.66%)
Q1-LR(16.58%)
16.58%
Q1-UL(6.17%)
Q1-UR(2.89%)
Q1-LL(78.12%)
Q1-LR(12.82%)
12.82%
PI
ANNEXIN V
Apoptotic cells(%)
**
#
TNF-α (ng/ml) 0 60 60 60 60
GABA (μM) 0 0 1 10 100

# FIG. 24

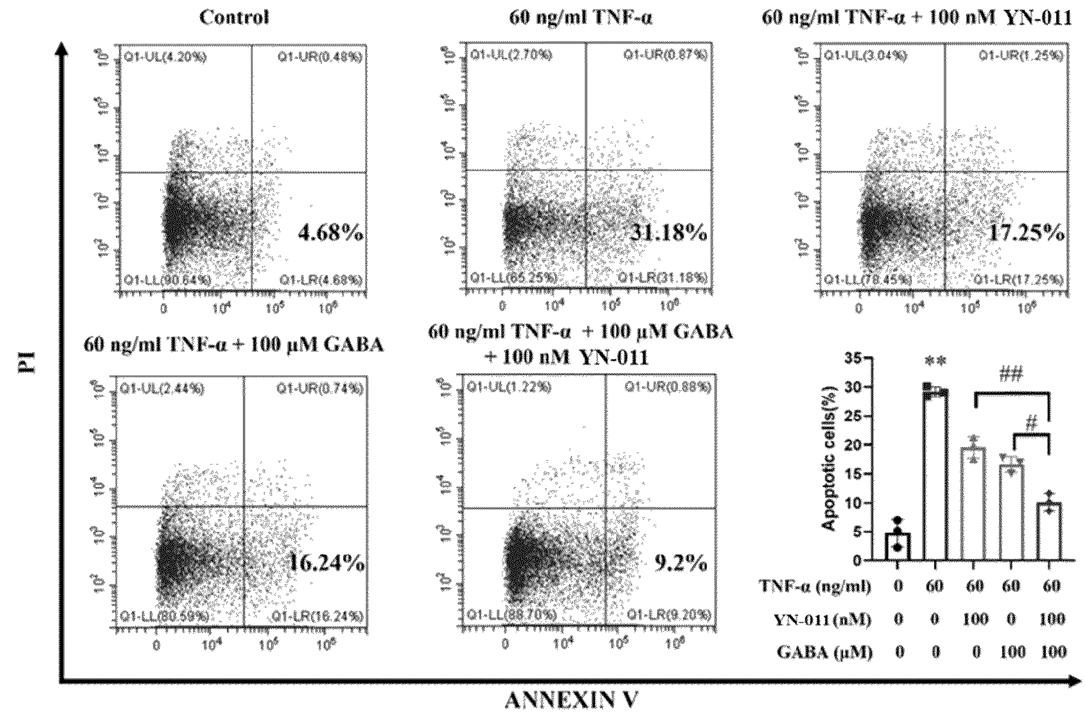
Control
60 ng/ml TNF-α
60 ng/ml TNF-α + 100 nM YN-011
60 ng/ml TNF-α + 100 μM GABA
60 ng/ml TNF-α + 100 μM GABA + 100 nM YN-011
PI
ANNEXIN V
Q1-UL(4.20%)
Q1-UR(0.48%)
Q1-LL(90.64%)
Q1-LR(4.68%)
4.68%
Q1-UL(2.70%)
Q1-UR(0.87%)
Q1-LL(65.25%)
Q1-LR(31.18%)
31.18%
Q1-UL(3.04%)
Q1-UR(1.25%)
Q1-LL(78.45%)
Q1-LR(17.25%)
17.25%
Q1-UL(2.44%)
Q1-UR(0.74%)
Q1-LL(80.59%)
Q1-LR(16.24%)
16.24%
Q1-UL(1.22%)
Q1-UR(0.88%)
Q1-LL(88.70%)
Q1-LR(9.20%)
9.2%
Apoptotic cells(%)
**
##
#
TNF-α (ng/ml) 0 60 60 60 60
YN-011 (nM) 0 0 100 0 100
GABA (μM) 0 0 0 100 100

# FIG. 25

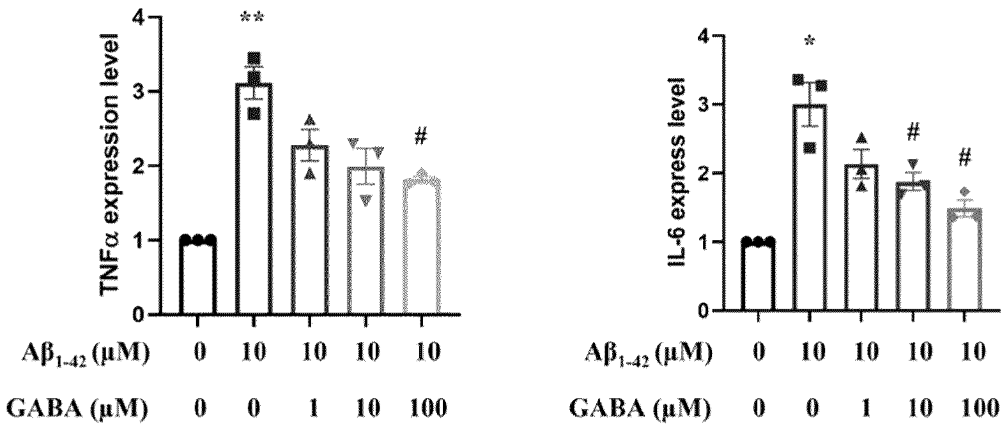

TNFα expression level
IL-6 express level
0
1
2
3
4
**
*
#
#
#
Aβ1-42 (μM) 0 10 10 10 10
GABA (μM) 0 0 1 10 100
Aβ1-42 (μM) 0 10 10 10 10
GABA (μM) 0 0 1 10 100

# FIG. 26

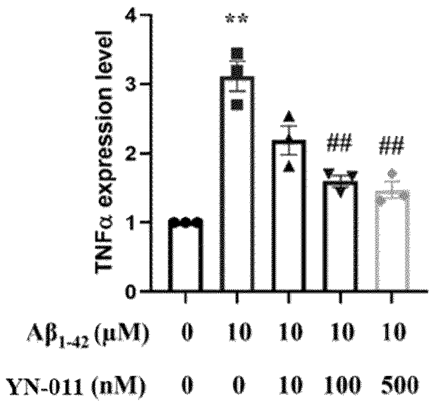
TNFα expression level
4
3
2
1
0
**
##
##
Aβ1-42 (μM) 0 10 10 10 10
YN-011 (nM) 0 0 10 100 500

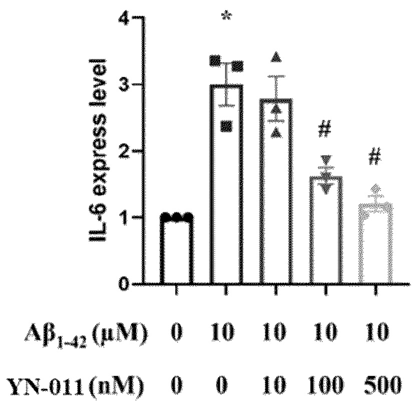
IL-6 express level
4
3
2
1
0
*
#
#
Aβ1-42 (μM) 0 10 10 10 10
YN-011 (nM) 0 0 10 100 500

# FIG. 27

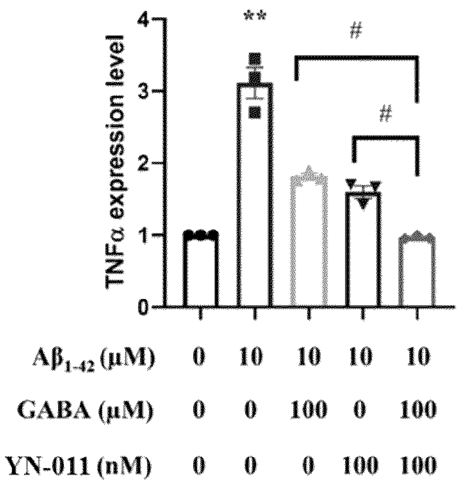
TNFα expression level
4
3
2
1
0
**
#
#
Aβ1-42 (μM) 0 10 10 10 10
GABA (μM) 0 0 100 0 100
YN-011 (nM) 0 0 0 100 100

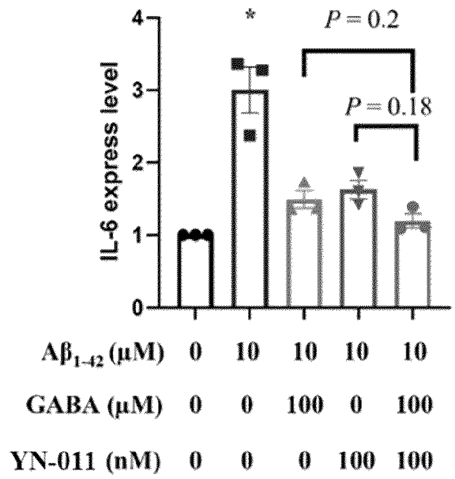
IL-6 express level
4
3
2
1
0
*
P = 0.2
P = 0.18
Aβ1-42 (μM) 0 10 10 10 10
GABA (μM) 0 0 100 0 100
YN-011 (nM) 0 0 0 100 100

# FIG. 28

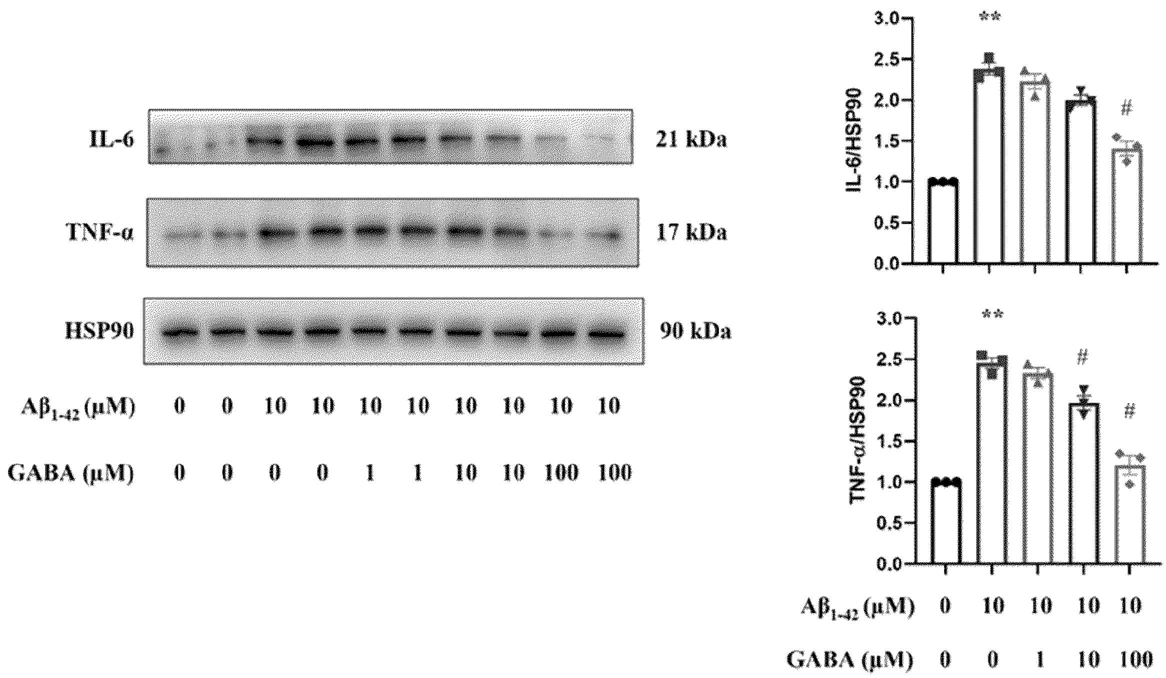
IL-6
21 kDa
TNF-α
17 kDa
HSP90
90 kDa
Aβ1-42 (μM) 0 0 10 10 10 10 10 10 10 10
GABA (μM) 0 0 0 0 1 1 10 10 100 100
IL-6/HSP90
TNF-α/HSP90
3.0
2.5
2.0
1.5
1.0
0.5
0.0
**
#
Aβ1-42 (μM) 0 10 10 10 10
GABA (μM) 0 0 1 10 100

# FIG. 29

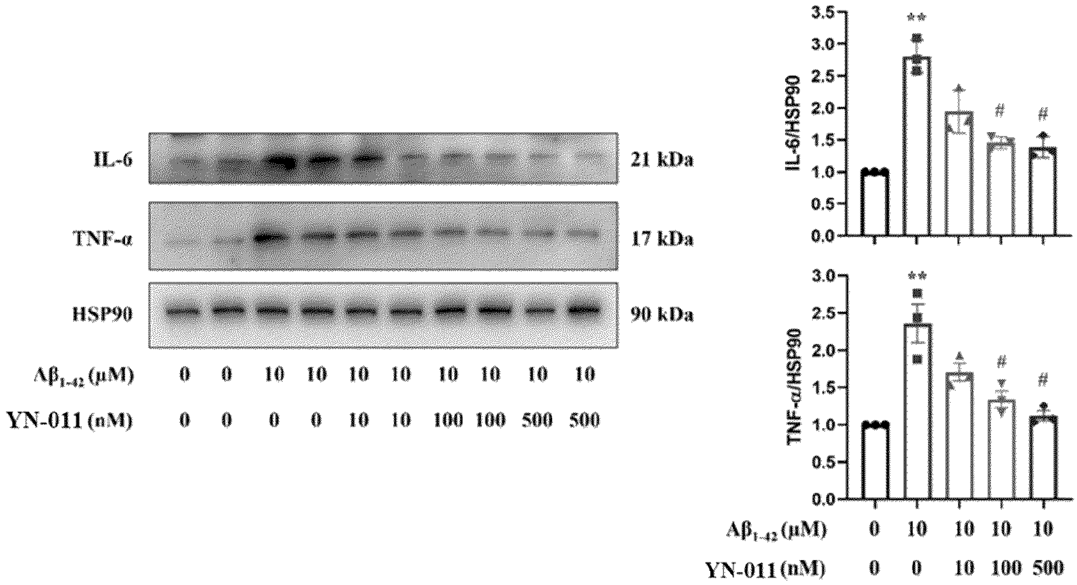
IL-6
21 kDa
TNF-α
17 kDa
HSP90
90 kDa
Aβ1-42 (μM) 0 0 10 10 10 10 10 10 10 10
YN-011 (nM) 0 0 0 0 10 10 100 100 500 500
IL-6/HSP90
TNF-α/HSP90
**
#
Aβ1-42 (μM) 0 10 10 10 10
YN-011 (nM) 0 0 10 100 500

# FIG. 30

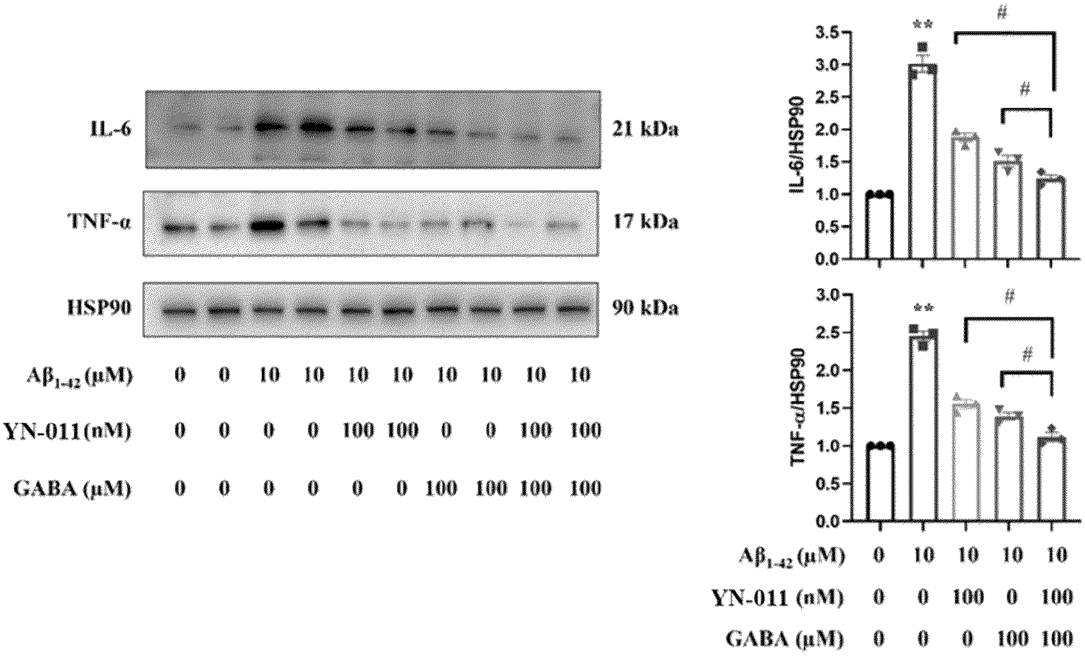
IL-6
21 kDa
TNF-α
17 kDa
HSP90
90 kDa
Aβ1-42 (μM) 0 0 10 10 10 10 10 10 10 10
YN-011(nM) 0 0 0 0 100 100 0 0 100 100
GABA (μM) 0 0 0 0 0 0 100 100 100 100
IL-6/HSP90
TNF-α/HSP90
**
#
Aβ1-42 (μM) 0 10 10 10 10
YN-011 (nM) 0 0 100 0 100
GABA (μM) 0 0 0 100 100

## INTERNATIONAL SEARCH REPORT

International application No.
**PCT/CN2023/101978**

**A. CLASSIFICATION OF SUBJECT MATTER**

C07K19/00(2006.01)i; C07K14/605(2006.01)i; A61K45/06(2006.01)i; C07K14/00(2006.01)i; A61K38/16(2006.01)i

According to International Patent Classification (IPC) or to both national classification and IPC

**B. FIELDS SEARCHED**

Minimum documentation searched (classification system followed by classification symbols)

IPC:C07K19,C07K14,A61K45,A61K38

Documentation searched other than minimum documentation to the extent that such documents are included in the fields searched

Electronic data base consulted during the international search (name of data base and, where practicable, search terms used)

CNTXT, ENTXT, ENTXTC, OETXT, VEN, CJFD, PubMed; ISI web of knowledge; 中国专利生物序列检索系统, China Patent Biological Sequence Search System; Genbank; EMBL-EBI; STN: GLP-1, 活性, 赖氨酸, 羟基, 羟基, 色氨酸, 氧化, A8G, IgG2, GLP?1, SEQ ID NOs: 1-19, 26, 27, A8G, G22E, R36G, C222S, A330S, P331S

**C. DOCUMENTS CONSIDERED TO BE RELEVANT**

| Category* | Citation of document, with indication, where appropriate, of the relevant passages | Relevant to claim No. |
|---|---|---|
| X | YANG, Yi et al. "Expression and Characterization of a Potent Long-Acting GLP-1 Receptor Agonist, GLP-1-IgG2σ-Fc" *PLoS One,* 27 May 2016 (2016-05-27), pp. 1-14 ISSN: 1932-6203, abstract, and description, page 2, paragraph 1 to page 12, paragraph 2, and figures 1A-1D | 1-2, 6-27, 30-46, 48-50, 55-61 |
| X | US 2016310575 A1 (INDIANA UNIVERSITY RESERCH AND TECHNOLOGY CORPORATION) 27 October 2016 (2016-10-27) claims 1-30, and description, paragraphs 11-266 | 1-2, 6-27, 30-46, 48-50, 55-61 |
| Y | YANG, Yi et al. "Expression and Characterization of a Potent Long-Acting GLP-1 Receptor Agonist, GLP-1-IgG2σ-Fc" *PLoS One,* 27 May 2016 (2016-05-27), pp. 1-14 ISSN: 1932-6203, abstract, and description, page 2, paragraph 1 to page 12, paragraph 2, and figures 1A-1D | 3-48, 50-61 |
| Y | US 2016310575 A1 (INDIANA UNIVERSITY RESERCH AND TECHNOLOGY CORPORATION) 27 October 2016 (2016-10-27) claims 1-30, and description, paragraphs 11-266 | 3-48, 50-61 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 September 2023** | **28 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/101978**

**C. DOCUMENTS CONSIDERED TO BE RELEVANT**

| Category* | Citation of document, with indication, where appropriate, of the relevant passages | Relevant to claim No. |
|---|---|---|
| Y | CN 114395597 A (GUANGZHOU CANTON BIOLOGICS CO., LTD. et al.) 26 April 2022 (2022-04-26) description, paragraphs 2-11 and 38-129 | 3-48, 50-61 |
| Y | CN 1322137 A (ELI LILLY AND CO.) 14 November 2001 (2001-11-14) description, page 5, line 25 to page 10, line 25 | 4-48, 50, 51, 55-61 |
| Y | CN 106659770 A (GLAXOSMITHKLINE LLC) 10 May 2017 (2017-05-10) description, paragraphs 338-443 | 4-48, 50, 51, 55-61 |

Form PCT/ISA/210 (second sheet) (July 2022)

**INTERNATIONAL SEARCH REPORT**

International application No.
**PCT/CN2023/101978**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/101978**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **55-61 (in part)**
because they relate to subject matter not required to be searched by this Authority, namely:

Claim 55 (in part) relates to the use of the fusion protein according to any one of claims 1-34, or the dimer of claim 35, or the polynucleotide of claim 36 or 37, or the carrier of claim 38, or the cell of claims 39-45, or the composition of claim 46 or 47 in a drug, wherein "the use as a drug" is comprised therein; and claims 56-61 further define the type of disease. Claims 55-61 (in part) relate to a method for diagnosing and treating diseases, which falls within methods for treatment and diagnosis of diseases (PCT Rule 39.1(iv)). The present report is provided on the basis of a reasonable expectation that claims 55-61 (in part) are amended to claims for preparing a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/101978**

| Patent document cited in search report | Publication date (day/month/year) | Patent family member(s) | Publication date (day/month/year) |
|---|---|---|---|
| US 2016310575 A1 | 27 October 2016 | WO 2015095684 A1 | 25 June 2015 |
| | | JP 2017504598 A | 09 February 2017 |
| | | US 10137170 B2 | 27 November 2018 |
| | | EP 3082847 A1 | 26 October 2016 |
| | | CN 106029087 A | 12 October 2016 |
| CN 114395597 A | 26 April 2022 | None | |
| CN 1322137 A | 14 November 2001 | JP 2002526454 A | 20 August 2002 |
| | | AU 6398599 A | 10 April 2000 |
| | | AU 766375 B2 | 16 October 2003 |
| | | TWI 260986 B | 01 September 2006 |
| | | BR 9913932 A | 16 October 2001 |
| | | WO 0016797 A2 | 30 March 2000 |
| | | WO 0016797 A3 | 25 May 2000 |
| | | MY 155270 A | 30 September 2015 |
| | | EP 1115421 A2 | 18 July 2001 |
| | | EP 1115421 B1 | 10 June 2009 |
| | | MX 2001003008 A1 | 01 September 2001 |
| | | AU 2003270960 A1 | 22 January 2004 |
| | | CN 1163267 C | 25 August 2004 |
| | | EP 1652531 A1 | 03 May 2006 |
| | | AU 2003270960 B2 | 21 December 2006 |
| | | IN 225241 B | 07 November 2008 |
| | | PH 1199902368 B1 | 05 November 2009 |
| CN 106659770 A | 10 May 2017 | CA 2952969 A1 | 30 December 2015 |
| | | JP 2017524680 A | 31 August 2017 |
| | | WO 2015200324 A1 | 30 December 2015 |
| | | US 2018021409 A1 | 25 January 2018 |
| | | US 2018369341 A1 | 27 December 2018 |
| | | EP 3160491 A1 | 03 May 2017 |
| | | EP 3160491 A4 | 17 January 2018 |
| | | US 2019175701 A1 | 13 June 2019 |
| | | KR 20170021313 A | 27 February 2017 |
| | | AR 100942 A1 | 09 November 2016 |
| | | BR 112016030588 A2 | 31 October 2017 |
| | | RU 2017101667 A | 26 July 2018 |
| | | AU 2015280110 A1 | 12 January 2017 |
| | | TW 201613630 A | 16 April 2016 |
| | | IN 201617044116 A | 07 April 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8658174 B
- WO 2021163972 A1
- CN 111217915 A
- WO 2011056713 A2
- WO 2000034332 A1
- WO 2002046227 A2

### Non-patent literature cited in the description


- **HOLLIGER et al.** *Proc. Natl. Acad. Sci. USA*, 1993, vol. 90, 6444-6448
- **POLJAK et al.** *Structure*, 1994, vol. 2, 1121-1123
- **BATZER et al.** *Nucleic Acid Res.*, 1991, vol. 19, 5081
- **OHTSUKA et al.** *J. Biol. Chem.*, 1985, vol. 260, 2605-2608
- **ROSSOLINI et al.** *Mol. Cell. Probes*, 1994, vol. 8, 91-98
- **SORLI et al.** *Lancet Diabetes Endocrinol*, 2017, vol. 5, 251-60
- **SHI et al.** *J Diabetes Investig*, 2020, vol. 11, 142-150
- **SHUAI et al.** *Diabetes Obes Metab.*, 2021, vol. 23 (1), 116-124
- **DUNGAN et al.** *Lancet*, 2014, vol. 384, 1349-57
- **JI et al.** *Diabetes Obes Metab.*, 2021, vol. 23, 404-414
- **LEECH, C.A. et al.** Expression of cAMP-regulated guanine nucleotide exchange factors in pancreatic beta-cells. *Biochem Biophys Res Commun*, 2000, vol. 278 (1), 44-7
- **DRUCKER, D.J.** Glucagon-like peptides. *Diabetes*, 1998, vol. 47 (2), 159-69
- **MONTROSE-RAFIZADEH, C. et al.** Pancreatic glucagon-like peptide-1 receptor couples to multiple G proteins and activates mitogen-activated protein kinase pathways in Chinese hamster ovary cells. *Endocrinology*, 1999, vol. 140 (3), 1132-40
- **AHREN, B.** ; **O. SCHMITZ**. GLP-1 receptor agonists and DPP-4 inhibitors in the treatment of type 2 diabetes. *Horm Metab Res*, 2004, vol. 36 (11-12), 867-76
- **GREEN, B.D. et al.** Structurally modified analogues of glucagon-like peptide-1 (GLP-1) and glucose-dependent insulinotropic polypeptide (GIP) as future antidiabetic agents. *Curr Pharm Des*, 2004, vol. 10 (29), 3651-62
- **CHANG, A.M. et al.** The GLP-1 derivative NN2211 restores beta-cell sensitivity to glucose in type 2 diabetic patients after a single dose. *Diabetes*, 2003, vol. 52 (7), 1786-91
- **LIU, H.K. et al.** N-acetyl-GLP-1: a DPP IV-resistant analogue of glucagon-like peptide-1 (GLP-1) with improved effects on pancreatic beta-cell-associated gene expression. *Cell Biol Int*, 2004, vol. 28 (1), 69-73
- **KIM, J.G. et al.** Development and characterization of a glucagon-like peptide 1-albumin conjugate: the ability to activate the glucagon-like peptide 1 receptor in vivo. *Diabetes*, 2003, vol. 52 (3), 751-9
- **NIELSEN, R. et al.** Effect of liraglutide on myocardial glucose uptake and blood flow in stable chronic heart failure patients: A double-blind, randomized, placebo-controlled LIVE sub-study. *J Nucl Cardiol*, 2019, vol. 26 (2), 585-597
- **DUARTE, A.I. et al.** Liraglutide Protects Against Brain Amyloid-beta1-42 Accumulation in Female Mice with Early Alzheimer's Disease-Like Pathology by Partially Rescuing Oxidative/Nitrosative Stress and Inflammation. *Int J Mol Sci*, 2020, vol. 21 (5)
- **ELBASSUONI, E.A.** ; **R.F. AHMED**. Mechanism of the neuroprotective effect of GLP-1 in a rat model of Parkinson's with pre-existing diabetes. *Neurochem Int*, 2019, vol. 131, 104583
- **MUSKIET, M.H.A. et al.** GLP-1 and the kidney: from physiology to pharmacology and outcomes in diabetes. *Nat Rev Nephrol*, 2017, vol. 13 (10), 605-628
- **HOU, Y. et al.** Nutrient Optimization Reduces Phosphorylation and Hydroxylation Level on an Fc-Fusion Protein in a CHO Fed-Batch Process. *Biotechnol J*, 2019, vol. 14 (3), e1700706
- **KARLIN, S.** ; **S.F. ALTSCHUL**. Methods for assessing the statistical significance of molecular sequence features by using general scoring schemes. *Proc Natl Acad Sci U S A*, 1990, vol. 87 (6), 2264-8

- **KARLIN, S.** ; **S.F. ALTSCHUL**. Applications and statistics for multiple high-scoring segments in molecular sequences. *Proc Natl Acad Sci U S A*, 1993, vol. 90 (12), 5873-7
- **ALTSCHUL, S.F. et al.** Basic local alignment search tool. *J Mol Biol*, 1990, vol. 215 (3), 403-10
- **ALTSCHUL, S.F. et al.** Gapped BLAST and PSI-BLAST: a new generation of protein database search programs. *Nucleic Acids Res*, 1997, vol. 25 (17), 3389-402
- **MYERS, E.W.** ; **W. MILLER**. Optimal alignments in linear space. *Comput Appl Biosci*, 1988, vol. 4 (1), 11-7
- **BETTINGER, J.Q. et al.** Quantitative Analysis of in Vivo Methionine Oxidation of the Human Proteome. *J Proteome Res*, 2020, vol. 19 (2), 624-633
- **ALLEN, L.V., JR.** Remington: The Science and Practice of Pharmacy: from the past into the future. *Int J Pharm Compd*, 2012, vol. 16 (5), 358-62
- **TOFT-NIELSEN, M.B.** ; **S. MADSBAD** ; **J.J. HOLST**. Continuous subcutaneous infusion of glucagon-like peptide 1 lowers plasma glucose and reduces appetite in type 2 diabetic patients. *Diabetes Care*, 1999, vol. 22 (7), 1137-43
- **GEISER, J.S. et al.** Clinical Pharmacokinetics of Dulaglutide in Patients with Type 2 Diabetes: Analyses of Datafrom Clinical Trials. *Clin Pharmacokinet*, 2016, vol. 55 (5), 625-34
- **BODANSZKY, M.** Principles of peptide synthesis. Springer-Verlag, 1993, vol. xii, 329
- **PRATLEY, R.E. et al.** Semaglutide versus dulaglutide once weekly in patients with type 2 diabetes (SUSTAIN 7): a randomised, open-label, phase 3b trial. *Lancet Diabetes Endocrinol*, 2018, vol. 6 (4), 275-286
- **ZHENG, J. et al.** GLP-1 improves the supportive ability of astrocytes to neurons by promoting aerobic glycolysis in Alzheimer's disease. *Mol Metab*, 2021, vol. 47, 101180
- **PARK, J.S. et al.** Blocking microglial activation of reactive astrocytes is neuroprotective in models of Alzheimer's disease. *Acta Neuropathol Commun*, 2021, vol. 9 (1), 78